# EUROPEAN PATENT APPLICATION

(11) **EP 1 939 183 A1**
(43) Date of publication of application: **02.07.2008**
(21) Application number: 06781525.8
(22) Date of filing: 25.07.2006
(51) Int. Cl.: C07D 233/61, A61K 31/4164, A61P 1/00, A61P 1/08, A61P 1/12, A61P 1/16, A61P 11/00, A61P 11/02, A61P 11/06, A61P 13/00, A61P 13/10, A61P 25/00, A61P 43/00, C07D 249/08, C07D 405/06, C07D 409/06

(54) **NOVEL NITROGENATED HETEROCYCLIC COMPOUND**

(30) Priority: 25.07.2005 JP 2005215146
(71) Applicant: Mitsubishi Tanabe Pharma Corporation, Chuo-ku Osaka-shi Osaka 541-8505 (JP)
(72) Inventor: AIZAWA, Hideyuki, Chuo-ku, Osaka-shi Osaka 541-8505 (JP); SEKI, Makoto, Chuo-ku, Osaka-shi Osaka 541-8505 (JP); ENDOH, Jun-ichi, Chuo-ku, Osaka-shi Osaka 541-8505 (JP); TANAKA, Minoru, Chuo-ku, Osaka-shi Osaka 541-8505 (JP); FUJIE, Naoto, Chuo-ku, Osaka-shi Osaka 541-8505 (JP); SAKUMA, Osamu, Chuo-ku, Osaka-shi Osaka 541-8505 (JP); KAMAHORI, Takao, Chuo-ku, Osaka-shi Osaka 541-8505 (JP)
(74) Representative: Merkle, Gebhard
(86) International application number: PCT/JP2006/314613
(87) International publication number: WO 2007/013421

(57) **Abstract**

A nitrogen-containing heterocyclic derivative represented by the following general formula (I), or a hydrate or solvate thereof, which has a selective antagonistic action on the muscarinic M₃ receptor and causes reduced cardiac side effect and thus is safe, and has superior pharmacological efficacy and prolonged action even by inhalation administration, and a pharmaceutical composition comprising the compound and a pharmaceutically acceptable carrier.

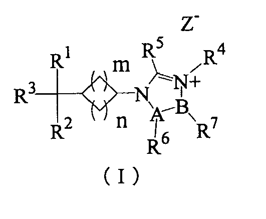

## Description

### Technical Field

The present invention relates to novel nitrogen-containing heterocyclic compounds.

### Background Art

It is known that at least three kinds of subtypes of muscarinic receptor exist, i.e., M₁ receptor exists in the brain, M₂ receptor exists in the heart, and M₃ receptor exists in smooth muscles and the glandular system.

Since medicaments having a muscarinic receptor antagonistic action have a spasmolytic action and an inhibitory action on secretion, they are used as therapeutic agents for functional disorders of respiratory organs, urinary organs, digestive organs, and the like, and compounds including atropine, butylscopolamine, ipratropium, tiotropium, oxybutynin, propantheline and the like have been clinically used. However, these compounds have affinity to each of M₁, M₂ and M₃ as the muscarinic receptors at almost the same level, and therefore, it is known that side effects due to the non-selective antagonism to acetylcholine are unavoidable. Accordingly, medicaments have been desired which have highly selective muscarinic receptor antagonistic action, especially cause no adverse reaction on the heart where the M₂ receptor is involved. Further, since the efficacies of these medicaments based on the muscarinic M₃ receptor antagonistic action thereof are maintained only for a short period of time, which requires frequent administrations clinically, medicaments having superior sustained efficacy have been desired.

The inventors of the present invention previously filed patent applications for aminocycloalkane compounds having a selective muscarinic M₃ receptor antagonistic action (Patent document 1: International Publication WO99/05095) and for imidazole compounds having a selective muscarinic M₃ receptor antagonistic action (Patent document 2: International Publication WO00/56718). Among these compounds, the compounds represented by the following general formula:

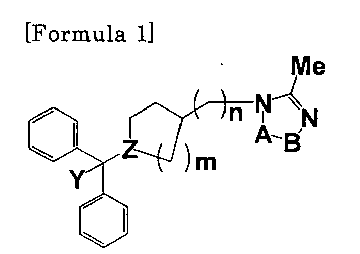

[wherein, in the formula, Y is hydrogen atom, cyano, or carbamoyl, preferably carbamoyl, Z is CH group or nitrogen atom, preferably CH group, -A-B- is -CH₂-CH₂-group or -CH=CH- group, preferably -CH₂-CH₂- group, m is 1 or 2, preferably 1, and n is 0 or 1, preferably 0] described in Patent document 2 are disclosed to have higher selectivity for the muscarinic receptor of smooth muscles than the muscarinic receptor of the heart, and to have potent antagonistic action thereon. However, the actions of these compounds when administered by inhalation are not specifically disclosed or suggested. Further, the compounds of the present invention represented by the following general formula (I) are not specifically disclosed nor suggested. Patent document 1: International Patent Publication WO99/05095
Patent document 2: International Patent Publication WO00/56718

### Disclosure of the Invention

### Object to be Achieved by the Invention

The inventors of the present invention focused their attention to nitrogen-containing heterocyclic compounds, and conducted various researches. As a result, it was found that nitrogen-containing heterocyclic derivatives represented by the general formula (I):

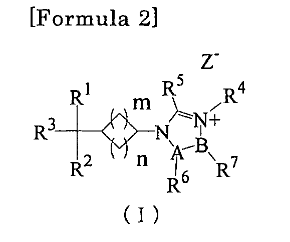

[wherein, in the formula, R¹ represents aryl of which ring may be substituted, R² represents aryl of which ring may be substituted, lower alkyl which may be substituted, or cycloalkyl which may be substituted, R³ represents hydrogen atom, cyano, a CONHR⁸ group (R⁸ represents hydrogen atom or lower alkyl), a CO₂R⁹ group (R⁹ represents hydrogen atom, aryl, lower alkyl, lower alkenyl, lower alkynyl, or aralkyl), hydroxyl group, a OCOR¹⁰ group (R¹⁰ represents aryl, lower alkyl, or aralkyl), or SONH₂ group, R⁴ represents alkyl which may be substituted, or a CO₂R¹¹ group (R¹¹ represents aryl, lower alkyl, or aralkyl), R⁵ represents hydrogen atom, aryl, lower alkyl, cycloalkyl, or aralkyl, R⁶ and R⁷ independently do not exist, or represent hydrogen atom or lower alkyl, -A-B- represents -CH-CH-, -C=C-, -C=N-, or -N=C-, the sum of m and n is an integer of 1 to 6, and Z⁻ represents an anion] had selective antagonistic action on the muscarinic M₃ receptor, and therefore they caused reduced cardiac side effects and thus were safe compounds. Moreover, it was also found that these compounds had superior pharmacological efficacy and sustained action even by inhalation administration, and therefore they were extremely useful for treatments of various kinds of diseases with air flow obstruction in which the muscarinic M₃ receptor is involved, for example, respiratory diseases such as chronic obstructive pulmonary disease, chronic bronchitis, asthma, chronic airway obstruction, pulmonary fibrosis, emphysema, diffuse panbronchiolitis, bronchiectasis, idiopathic interstitial pneumonia and rhinitis, and the like. The present invention was achieved on the basis of these findings.

### Means for Achieving the Object

The gists of the present invention are as follows.
(1) A nitrogen-containing heterocyclic derivative represented by the general formula (I):

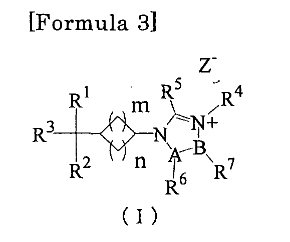

[wherein, in the formula, R¹ represents aryl of which ring may be substituted, R² represents aryl of which ring may be substituted, lower alkyl which may be substituted, or cycloalkyl which may be substituted, R³ represents hydrogen atom, cyano, a CONHR⁸ group (R⁸ represents hydrogen atom or lower alkyl), a CO₂R⁹ group (R⁹ represents hydrogen atom, aryl, lower alkyl, lower alkenyl, lower alkynyl, or aralkyl), hydroxyl group, a OCOR¹⁰ group (R¹⁰ represents aryl, lower alkyl, or aralkyl), or SONH₂ group, R⁴ represents alkyl which may be substituted or a CO₂R¹¹ group (R¹¹ represents aryl, lower alkyl, or aralkyl), R⁵ represents hydrogen atom, aryl, lower alkyl, cycloalkyl, or aralkyl, R⁶ and R⁷ independently do not exist, or represent hydrogen atom or lower alkyl, -A-B- represents -CH-CH-, -C=C-, -C=N-, or -N=C-, the sum of m and n is an integer of 1 to 6, and Z⁻ represents an anion], or a hydrate or solvate thereof.
(2) The nitrogen-containing heterocyclic derivative, or a hydrate or solvate thereof according to (1), wherein R¹ and R² are phenyls which may be independently substituted.
(3) The nitrogen-containing heterocyclic derivative, or a hydrate or solvate thereof according to (1) or (2), wherein R³ is cyano, a CONHR⁸ group (R⁸ represents hydrogen atom, methyl, or ethyl), carboxy, methoxycarbonyl, ethoxycarbonyl, hydroxyl group, methylcarbonyloxy, ethylcarbonyloxy, or SONH₂ group.
(4) The nitrogen-containing heterocyclic derivative, or a hydrate or solvate thereof according to any one of (1) to (3), wherein R⁴ is alkyl having 1 to 8 carbon atoms which may be substituted.
(5) The nitrogen-containing heterocyclic derivative, or a hydrate or solvate thereof according to any one of (1) to (4), wherein R¹ and R² are phenyls, R³ is cyano or CONH₂ group, and R⁴ is alkyl having 1 to 8 carbon atoms which may be substituted.
(6) The nitrogen-containing heterocyclic derivative, or a hydrate or solvate thereof according to any one of (1) to (5), wherein R⁴ is methyl, methoxyethyl, benzyl, acetylmethyl, phenylpropyl, or hydroxyethyl.
(7) The nitrogen-containing heterocyclic derivative, or a hydrate or solvate thereof according to any one of (1) to (6), wherein R⁵ is hydrogen atom or lower alkyl.
(8) The nitrogen-containing heterocyclic derivative, or a hydrate or solvate thereof according to any one of (1) to (7), wherein R⁶ and R⁷ independently do not exist, or represent hydrogen atom, methyl, or ethyl.
(9) The nitrogen-containing heterocyclic derivative, or a hydrate or solvate thereof according to any one of (1) to (8), wherein R⁵ is hydrogen atom or alkyl having 1 to 3 carbon atoms, and R⁶ and R⁷ independently do not exist, or represent hydrogen atom or methyl.
(10) The nitrogen-containing heterocyclic derivative, or a hydrate or solvate thereof according to any one of (1) to (9), wherein -A-B- is -CH-CH- or -C=C-.
(11) The nitrogen-containing heterocyclic derivative, or a hydrate or solvate thereof according to any one of (1) to (10), wherein the sum of m and n is 3 or 4.
(12) The nitrogen-containing heterocyclic derivative, or a hydrate or solvate thereof according to any one of (1) to (11), wherein the sum of m and n is 3.
(13) The nitrogen-containing heterocyclic derivative, or a hydrate or solvate thereof according to any one of (1) to (12), wherein Z⁻ is an anion formed from a halogen atom or an organic sulfonic acid.
(14) The nitrogen-containing heterocyclic derivative, or a hydrate or solvate thereof according to any one of (1) to (13), wherein Z⁻ is chloride ion, bromide ion, iodide ion, tosylate ion, or mesylate ion.
(15) 3-((1R,3S)-3-(Carbamoyl(diphenyl)methyl)cyclopentyl)-1,2-dimethyl-4,5-dihydro-3H-imidazol-1-ium para-toluenesulfonate.
(16) 3-((1R,3S)-3-(Carbamoyldiphenylmethyl)cyclopentan-1-yl)-1-(2-methoxyethyl)-2-methyl-3H-imidazol-1-ium mesylate.
(17) 3-((1S,3R)-3-(Carbamoyldiphenylmethyl)cyclopentan-1-yl)-1-benzyl-2-methyl-3H-imidazol-1-ium bromide.
(18) 3-((1R,3S)-3-(Carbamoyldiphenylmethyl)cyclopentan-1-yl)-2-methyl-1-(2-oxopropyl)-3H-imidazol-1-ium bromide.
(19) 3-((1R,3S)-3-(Carbamoyl(diphenyl)methyl)cyclopentyl)-1-(3-phenylpropyl)-2-methyl-3H-imidazol-1-ium bromide.
(20) 3-((1R,3S)-3-(Carbamoyldiphenylmethyl)cyclopentan-1-yl)-1-hydroxyethyl-2-methyl-3H-imidazol-1-ium bromide.
(21) A pharmaceutical composition comprising a nitrogen-containing heterocyclic derivative represented by the general formula (I):

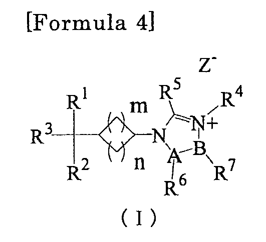

[wherein, in the formula, R¹ represents aryl of which ring may be substituted, R² represents aryl of which ring may be substituted, lower alkyl which may be substituted, or cycloalkyl which may be substituted, R³ represents hydrogen atom, cyano, a CONHR⁸ group (R⁸ represents hydrogen atom or lower alkyl), a CO₂R⁹ group (R⁹ represents hydrogen atom, aryl, lower alkyl, lower alkenyl, lower alkynyl, or aralkyl), hydroxyl group, a OCOR¹⁰ group (R¹⁰ represents aryl, lower alkyl, or aralkyl), or SONH₂ group, R⁴ represents alkyl which may be substituted or a CO₂R¹¹ group (R¹¹ represents aryl, lower alkyl, or aralkyl), R⁵ represents hydrogen atom, aryl, lower alkyl, cycloalkyl, or aralkyl, R⁶ and R⁷ independently do not exist, or represent hydrogen atom or lower alkyl, -A-B- represents -CH-CH-, -C=C-, -C=N-, or -N=C-, the sum of m and n is an integer of 1 to 6, and Z represents an anion], or a hydrate or solvate thereof, and a pharmaceutically acceptable carrier.
(22) The pharmaceutical composition according to (21), which is a prophylactic and/or therapeutic agent for a disease in which the muscarinic M₃ receptor is involved.
(23) The pharmaceutical composition according to (21) or (22), which is a prophylactic and/or therapeutic agent for a respiratory disease, a urologic disease, a digestive system disease, or a central nervous system disease.
(24) The pharmaceutical composition according to (23), wherein the respiratory disease is chronic obstructive pulmonary disease, chronic bronchitis, asthma, chronic airway obstruction, pulmonary fibrosis, emphysema, diffuse panbronchiolitis, bronchiectasis, idiopathic interstitial pneumonia, or rhinitis.
(25) The pharmaceutical composition according to (23), wherein the urologic disease is neurogenic pollakiuria, neurogenic bladder dysfunction, enuresis nocturna, unstable bladder, bladder spasms, chronic cystitis, or urinary incontinence and/or pollakiuria in interstitial cystitis.
(26) The pharmaceutical composition according to (23), wherein the digestive system disease is irritable bowel syndrome, spasticity colitis, diverticulitis, functional diarrhea, esophageal achalasia, cardiac achalasia, or biliary spasm.
(27) The pharmaceutical composition according to (23), wherein the central nervous system disease is nausea or vomition caused by drug administration, kinesia, or Meniere's disease.
(28) A prophylactic and/or therapeutic agent for chronic obstructive pulmonary disease, which comprises 3-((1R,3S)-3-(carbamoyl(diphenyl)methyl)cyclopentyl)-1,2-dimethyl-4,5-dihydro-3H-imidazol-1-ium para-toluenesulfonate and a pharmaceutically acceptable carrier.
(29) A prophylactic and/or therapeutic agent for chronic obstructive pulmonary disease, which comprises 3-((1R,3S)-3-(carbamoyldiphenylmethyl)cyclopentan-1-yl)-1-(2-methoxyethyl)-2-methyl-3H-imidazol-1-ium mesylate and a pharmaceutically acceptable carrier.
(30) A prophylactic and/or therapeutic agent for chronic obstructive pulmonary disease, which comprises 3-((1S,3R)-3-(carbamoyldiphenylmethyl)cyclopentan-1-yl)-1-benzyl-2-methyl-3H-imidazol-1-ium bromide and a pharmaceutically acceptable carrier.
(31) A prophylactic and/or therapeutic agent for chronic obstructive pulmonary disease, which comprises 3-((1R,3S)-3-(carbamoyldiphenylmethyl)cyclopentan-1-yl)-2-methyl-1-(2-oxopropyl)-3H-imidazol-1-ium bromide and a pharmaceutically acceptable carrier.
(32) A prophylactic and/or therapeutic agent for chronic obstructive pulmonary disease, which comprises 3-((1R,3S)-3-(carbamoyl(diphenyl)methyl)cyclopentyl)-1-(3-phenylpropyl)-2-methyl-3H-imidazol-1-ium bromide and a pharmaceutically acceptable carrier.
(33) A prophylactic and/or therapeutic agent for chronic obstructive pulmonary disease, which comprises 3-((1R,3S)-3-(carbamoyldiphenylmethyl)cyclopentan-1-yl)-1-hydroxyethyl-2-methyl-3H-imidazol-1-ium bromide and a pharmaceutically acceptable carrier.
(34) A muscarinic M₃ receptor antagonist containing a nitrogen-containing heterocyclic derivative represented by the general formula (I):

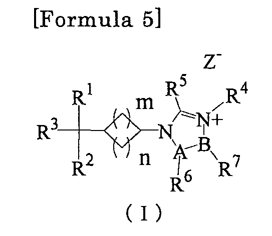

[wherein, in the formula, R¹ represents aryl of which ring may be substituted, R² represents aryl of which ring may be substituted, lower alkyl which may be substituted, or cycloalkyl which may be substituted, R³ represents hydrogen atom, cyano, a CONHR⁸ group (R⁸ represents hydrogen atom or lower alkyl), a CO₂R⁹ group (R⁹ represents hydrogen atom, aryl, lower alkyl, lower alkenyl, lower alkynyl, or aralkyl), hydroxyl group, a OCOR¹⁰ group (R¹⁰ represents aryl, lower alkyl, or aralkyl), or SONH₂ group, R⁴ represents alkyl which may be substituted or a CO₂R¹¹ group (R¹¹ represents aryl, lower alkyl, or aralkyl), R⁵ represents hydrogen atom, aryl, lower alkyl, cycloalkyl, or aralkyl, R⁶ and R⁷ independently do not exist, or represent hydrogen atom or lower alkyl, -A-B- represents -CH-CH-, -C=C-, -C=N-, or -N=C-, the sum of m and n is an integer of 1 to 6, and Z⁻ represents an anion], or a hydrate or solvate thereof, or a pharmaceutically acceptable salt thereof as an active ingredient.

### Effect of the Invention

The compounds of the present invention are useful as prophylactic and/or therapeutic agents for a respiratory disease, a urologic disease, a digestive system disease, or a central nervous system disease.

### Best Mode for Carrying out the Invention

Hereafter, the meanings of the terms used in this specification will be explained, and the present invention will be explained in more detail.

In the present invention, the "aryl" means aryl having 6 to 11 carbon atoms, and specific examples include phenyl, naphthyl, and the like.

The "alkyl" means, for example, linear or branched alkyl having 1 to 8 carbon atoms, and specific examples include methyl, ethyl, propyl, isopropyl, butyl, isobutyl, s-butyl, t-butyl, pentyl, isopentyl, neopentyl, t-pentyl, hexyl, heptyl, octyl, and the like.

The "lower alkyl" means linear or branched alkyl having 1 to 6 carbon atoms, and specific examples include methyl, ethyl, propyl, isopropyl, butyl, isobutyl, s-butyl, t-butyl, pentyl, isopentyl, neopentyl, t-pentyl, hexyl, and the like.

The "cycloalkyl" means an alicyclic hydrocarbon having 3 to 8 carbon atoms, and specific examples include cyclopropyl, cyclobutyl, cyclopentyl, cyclohexyl, cyclohexyl, and cyclooctyl.

The "lower alkenyl" means linear or branched alkenyl having 2 to 7 carbon atoms, and specific examples include vinyl, 1-propenyl, 2-propenyl, isopropenyl, 1-butenyl, 2-butenyl, 3-butenyl, 1-methyl-1-propenyl, 1-methyl-2-propenyl, 1-ethyl-1-ethenyl, 2-methyl-1-propenyl, 2-methyl-2-propenyl, 3-methyl-2-butenyl, 4-pentenyl, and the like.

The "lower alkynyl" means alkynyl having 2 to 7 carbon atoms, and specific examples include acetylenyl, 1-propynyl, 2-propynyl, 1-butynyl, 2-butynyl, 3-butynyl, pentynyl, hexynyl, heptynyl, and the like.

The "aralkyl" means phenyl which may be substituted and is bound with linear or branched alkylene having 1 to 6 carbon atoms, and specific examples include benzyl, phenylethyl, phenylpropyl, and the like.

Examples of the "substituent" that aryl, lower alkyl, cycloalkyl, alkyl, or phenyl may have include a halogen atom; lower alkyl; cycloalkyl; aryl which may be substituted with lower alkyl, a halogen atom, hydroxyl group, cyano, lower alkoxycarbonyl, or lower alkoxy; lower alkenyl which may be substituted with cycloalkyl, aryl, or lower alkoxycarbonyl; lower alkynyl; a heterocyclic group which may be substituted with lower alkoxycarbonyl; lower alkoxy which may be substituted with a halogen atom, hydroxyl group, or lower alkoxy; hydroxyl group; acyloxy; acyl; aryloxy, and the like. The aryl, the lower alkyl, the cycloalkyl, the lower alkenyl, and the lower alkynyl herein referred to have the same meanings as those described above.

The "halogen atom" means fluorine atom, chlorine atom, bromine atom, or iodine atom.

The "lower alkoxycarbonyl" is linear or branched alkoxycarbonyl having 2 to 7 carbon atoms, and specific examples include methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentyloxycarbonyl, isopentyloxycarbonyl, neopentyloxycarbonyl, t-pentyloxycarbonyl, hexyloxycarbonyl, and the like.

The "lower alkoxy" is linear or branched alkoxy having 1 to 6 carbon atoms, and specific examples include methoxy, ethoxy, propoxy, isopropoxy, butoxy, isobutoxy, s-butoxy, t-butoxy, pentyloxy, isopentyloxy, neopentyloxy, t-pentyloxy, hexyloxy, and the like.

The "heterocyclic group" means a saturated or unsaturated stable 5- to 7-membered monocyclic ring consisting of carbon atoms and 1 to 3 heteroatoms selected from the group consisting of nitrogen atom, oxygen atom and sulfur atom, and the nitrogen atom and sulfur atom may be oxidized as desired. Specific examples include oxirane, oxetane, tetrahydrofuran, tetrahydropyran, tetrahydrothiophene, propiolactone, butyrolactone, valerolactone, caprolactone, fural, thiophene, pyrrole, isoxazole, thiazole, and the like.

The "acyloxy" is linear, branched or cyclic acyloxy having 2 to 7 carbon atoms, and specific examples include acetoxy, ethylcarbonyloxy, propylcarbonyloxy, isopropylcarbonyloxy, butylcarbonyloxy, isobutylcarbonyloxy, s-butylcarbonyloxy, t-butylcarbonyloxy, n-pentylcarbonyloxy, neopentylcarbonyloxy, n-hexylcarbonyloxy, cyclopentylcarbonyloxy, cyclohexylcarbonyloxy, benzoyloxy, and the like.

The "acyl" is linear or branched alkanoyl having 2 to 7 carbon atoms, cycloalkylcarbonyl having 4 to 7 carbon atoms, aroyl or heteroarylcarbonyl having 7 to 11 carbon atoms, and specific examples include acetyl, propionyl, butyryl, isobutyryl, valeryl, isovaleryl, hexanoyl, cyclopropylcarbonyl, cyclopentylcarbonyl, cyclohexylcarbonyl, benzoyl, nicotenoyl, thenoyl, furoyl, and the like. The heteroaryl herein means heteroaryl containing 1 to 3 nitrogen atoms, sulfur atoms or oxygen atoms as ring-constituting atoms, and containing 5 to 9 ring-constituting atoms.

The "aryloxy" means aryloxy having 6 to 11 carbon atoms, and specific examples include phenyloxy, naphthyloxy, and the like.

The "anion" means an anion formed from a halogen atom, an inorganic acid, an organic sulfonic acid, a carboxylic acid, or the like, and specific examples include chloride ion, bromide ion, iodide ion, tosylate ion, mesylate ion, and the like.

Preferred examples of R¹ in the general formula (I) include phenyl which may be substituted, and more preferred examples include phenyl.

Preferred examples of R² include phenyl which may be substituted, and more preferred examples include phenyl.

Preferred examples of R³ include cyano, a CONHR⁸ group, carboxy, methoxycarbonyl, ethoxycarbonyl, hydroxyl group, methylcarbonyloxy, ethylcarbonyloxy, and SONH₂ group, more preferred examples include cyano and a CONHR⁸ group, and still more preferred examples include a CONHR⁸ group.

Preferred examples of R⁸ include hydrogen atom, methyl, and ethyl, and more preferred examples include hydrogen atom.

Preferred examples of R⁴ include alkyl having 1 to 8 carbon atoms which may be substituted. Specific examples of the substituent that the alkyl having 1 to 8 carbon atoms may have include aryl which may be substituted with lower alkyl, a halogen atom, cyano, lower alkoxycarbonyl or lower alkoxy; linear or branched alkanoyl having 2 to 7 carbon atoms; aroyl having 7 to 11 carbon atoms; cycloalkyl; lower alkoxy which may be substituted with lower alkoxy; a 3- to 6-membered monocyclic heterocyclic group containing one oxygen atom or sulfur atom as heteroatom, which may be substituted with lower alkoxycarbonyl; aryloxy; acyloxy; a halogen atom; lower alkenyl which may be substituted with aryl, cycloalkyl, or lower alkoxycarbonyl; hydroxyl group; and lower alkynyl, preferred examples include phenyl; phenyl substituted with lower alkyl, a halogen atom, cyano, lower alkoxycarbonyl, or lower alkoxy; naphthyl; acetyl; propionyl; 2,2-dimethylpropionyl; benzoyl; cyclopropyl; cyclobutyl; cyclohexyl; methoxy; ethoxy; methoxyethoxy; oxiranyl; furyl which may be substituted with lower alkoxycarbonyl; thienyl; phenoxy; acetoxy; fluorine atom; ethenyl which may be substituted with aryl, cycloalkyl, or lower alkoxycarbonyl; 2-methylpropenyl; butenyl; hydroxyl group; propynyl; and heptynyl, and more preferred examples include methoxy, phenyl, acetyl, and hydroxyl group. Further, when the lower alkyl is unsubstituted, the carbon number is preferably 1 to 6, more preferably 1 to 5, still more preferably 1 to 3, further preferably 1 or 2, most preferably 1. When the lower alkyl is substituted, the carbon number is preferably 1 to 5, more preferably 1 to 3.

Specific examples of R⁴ include methyl, linear or branched butyl, linear or branched pentyl, heptyl, octyl, benzyl, methylbenzyl, fluorobenzyl, chlorobenzyl, cyanobenzyl, methoxycarbonylbenzyl, methoxybenzyl, naphthylmethyl, phenethyl, phenylpropyl, acetylmethyl, propionylmethyl, t-butylcarbonylmethyl, benzoylmethyl, cyclopropylmethyl, cyclobutylmethyl, cyclohexylmethyl, cyclohexylethyl, methoxyethyl, ethoxyethyl, methoxypropyl, methoxyethoxyethyl, oxiranylmethyl, oxiranylethyl, thienylmethyl, ethoxycarbonylfurylmethyl, phenoxyethyl, phenoxypropyl, acetoxyethyl, acetoxypropyl, acetoxypentyl, difluoromethyl, trifluoropropyl, trifluorobutyl, phenylethenylmethyl, cyclohexylethenylmethyl, methoxycarbonylethenylmethyl, ethoxycarbonylethenylmethyl, propenylmethyl, 2-methylpropenylmethyl, butenylmethyl, hydroxyethyl, hydroxypropyl, propynylmethyl, and heptynylmethyl, preferred examples include methyl, butyl, 2-methylpropyl, pentyl, 5-methylhexyl, octyl, 3-methylbutyl, pentyl, benzyl, 2-methylbenzyl, 3-methylbenzyl, 4-methylbenzyl, 2-fluorobenzyl, 3-fluorobenzyl, 4-fluorobenzyl, 2,3,4,5,6-pentafluorobenzyl, 4-chlorobenzyl, 4-cyanobenzyl, 3-methoxycarbonylbenzyl, 4-methoxycarbonylbenzyl, 3-methoxybenzyl, 1-naphthylmethyl, phenethyl, 3-phenylpropyl, acetylmethyl, propionylmethyl, t-butylcarbonylmethyl, benzoylmethyl, cyclopropylmethyl, cyclobutylmethyl, cyclohexylmethyl, 2-cyclohexylethyl, 2-methoxyethyl, 2-ethoxyethyl, 3-methoxypropyl, 2-(2-methoxyethoxy)ethyl, oxiranylmethyl, 2-oxiranylethyl, 3-thienylmethyl, 5-ethoxycarbonyl-furan-2-ylmethyl, 2-phenoxyethyl, 3-phenoxypropyl, 2-acetoxyethyl, 3-acetoxypropyl, 5-acetoxypentyl, difluoromethyl, 3,3,3-trifluoropropyl, 4,4,4-trifluorobutyl, 2-phenylethenylmethyl, 2-cyclohexylethenylmethyl, 2-methoxycarbonylethenylmethyl, 2-ethoxycarbonylethenylmethyl, ethoxy-1-propenylmethyl, 3-methyl-but-2-enyl, 1-butenylmethyl, 2-hydroxyethyl, 3-hydroxypropyl, 1-propynylmethyl, and 1-heptynylmethyl, and more preferred examples include methyl, benzyl, 3-phenylpropyl, acetylmethyl, 2-methoxyethyl, and 2-hydroxyethyl.

Preferred examples of R⁵ include hydrogen atom and lower alkyl, more preferred examples include hydrogen atom and alkyl having 1 to 3 carbon atoms such as methyl and ethyl, more preferred examples include hydrogen atom, methyl and ethyl, and still more preferred examples include methyl.

R⁶ preferably does not exist, or is preferably hydrogen atom, methyl, or ethyl, more preferably it does not exist, or is hydrogen atom or methyl, still more preferably it does not exist, or is hydrogen atom, and still further preferably is hydrogen atom.

R⁷ preferably does not exist, or is preferably hydrogen atom, methyl, or ethyl, more preferably it does not exist, or is hydrogen atom or methyl, still more preferably it does not exist, or is hydrogen atom, and still further preferably is hydrogen atom.

Preferred examples of -A-B- include -CH-CH-. Preferred examples of -A-B-also include -C=C-.

Preferred examples of the sum of m and n include 3 and 4, and more preferred examples include 3.

Preferred examples of the anion include an anion formed from a halogen atom or an organic sulfonic acid, more preferred examples include chloride ion, bromide ion, iodide ion, tosylate ion, and mesylate ion, and still more preferred examples include bromide ion, tosylate ion, and mesylate ion.

In the present invention, preferred examples of the compounds represented by the general formula (I) include the compounds having a value exceeding 0.6 as a difference of the pA₂ value for muscarinic M₃ (see, Test Example 1 described later) and the pA₂ value for muscarinic M₂ receptor (see, Test Example 2 described later: a value obtained by subtracting the pA₂ value for muscarinic M₂ receptor from the pA₂ value for muscarinic M₃ receptor), and more preferred examples include the compounds having a value of 1.0 or larger as the aforementioned value. Preferred examples of the compounds from another aspect include the compounds that inhibit, 24 hours after the administration described in the test of Test Example 3 mentioned later, the bronchoconstriction induced with acetylcholine by 50% or more as compared with that observed in the control group.

Hydrates or solvates of the compounds of the present invention also fall within the scope of the compounds of the present invention. These substances can be obtained by well-known methods. Further, stereoisomers such as optical isomers, diastereoisomers and geometrical isomers or tautomers of the compounds of the present invention may exist depending on types of the substituent, and any of these optical isomers, tautomers and mixtures thereof also fall within the scope of the compounds of the present invention.

The compounds of the present invention represented by the general formula (I) can be prepared by, for example, the methods described in the preparation methods mentioned below or examples, and the like. However, the preparation methods of the compounds of the present invention are not limited to these methods.

### Preparation Method 1

By reacting a compound represented by the general formula (II):

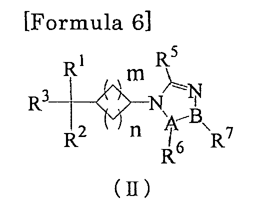

[wherein, in the formula, R¹, R², R³, R⁵, R⁶, R⁷, A, B, m and n have the same meanings as those defined above] or a salt thereof with a compound represented by the general formula (III):

R⁴-X¹ (III)

[wherein, in the formula, X¹ represents a leaving group, and R⁴ has the same meaning as that defined above], the compound represented by the general formula (I)

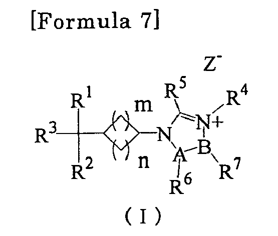

[wherein, in the formula, R¹, R², R³, R⁴, R⁵, R⁶, R⁷, A, B, m, n and Z have the same meanings as those defined above] can be prepared.

The "salt" of the compound represented by the general formula (II) means an acid addition salt at a basic nitrogen atom, and examples include, for example, inorganic acid salts such as hydrochloride, sulfate, nitrate, and phosphate, organic acid salts such as acetate, citrate, fumarate and tartrate, sulfonic acid salts such as methanesulfonate and tosylate, and the like.

Examples of the leaving group X¹ in the general formula (III) include halogen atoms such as chlorine atom, bromine atom and iodine atom, sulfonyloxy such as p-toluenesulfonyloxy, benzenesulfonyloxy, methanesulfonyloxy, and trifluoromethanesulfonyloxy, and the like, and chlorine atom, bromine atom, iodine atom, p-toluenesulfonyloxy, and methanesulfonyloxy are particularly preferred.

The reaction of the compound represented by the general formula (II) or a salt thereof and the compound represented by the general formula (III) is usually performed in an inert solvent having no influence on the reaction. Examples of the inert solvent include ethers such as tetrahydrofuran, diethyl ether and dioxane, aromatic hydrocarbons such as benzene, toluene and xylene, halogenated compound type solvents such as chloroform and dichloromethane, aprotic polar solvents such as acetone, acetonitrile, dimethyl sulfoxide and N,N-dimethylformamide, mixed solvents thereof, and the like, and tetrahydrofuran, dioxane, acetone and acetonitrile are particularly preferred.

The compound represented by the general formula (III) is usually used in an amount of 1 mole to excessive moles, preferably 1 to 10 moles, with 1 mole of the compound represented by the general formula (II).

As the reaction temperature, a temperature of from room temperature to the boiling point of the solvent is usually applied, and as for the reaction time, the reaction is usually performed for 10 minutes to 48 hours. However, a reaction temperature above or below the aforementioned range, and a reaction time longer or shorter than the aforementioned range may also be applied as required.

For smooth advance of the aforementioned reaction, the reaction may also be performed in the presence of a base.

Examples of the base include, for example, inorganic bases such as sodium hydrogencarbonate, potassium hydrogencarbonate, sodium carbonate, potassium carbonate and potassium tert-butoxide, organic bases such as triethylamine, N-methylmorpholine, N,N-diisopropylethylamine and 1,8-diazabicyclo[5.4.0]undec-7-ene (DBU), and the like.

The base is usually used in an amount of 1 mole to excessive moles, preferably 1 to 10 moles, with 1 mole of the compound represented by the general formula (II).

After completion of the reaction, the compound represented by the general formula (I) can be obtained by performing an ordinary treatment.

### Preparation Method 2

By reacting a compound represented by the general formula (IV)

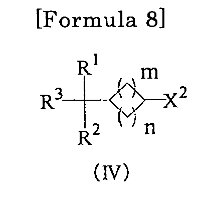

[wherein, in the formula, X² represents a leaving group, and R¹, R², R³, m and n have the same meanings as those defined above], with a compound represented by the general formula (V):

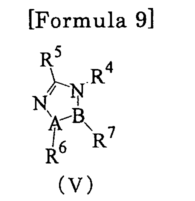

[wherein, in the formula, R⁴, R⁵, R⁶, R⁷, A and B have the same meanings as those defined above] or a salt thereof, the compound represented by the general formula (I):

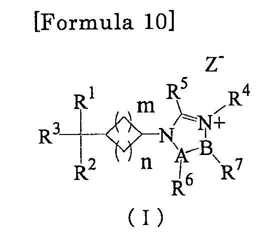

[wherein, in the formula, R¹, R², R³, R⁴, R⁵, R⁶, R⁷, A, B, m, n and Z have the same meanings as those defined above] can be prepared.

The "salt" of the compound represented by the general formula (V) means an acid addition salt at a basic nitrogen atom, and examples include, for example, inorganic acid salts such as hydrochloride, sulfate, nitrate, and phosphate, organic acid salts such as acetate, citrate, fumarate and tartrate, sulfonic acid salts such as methanesulfonate and tosylate, and the like.

Examples of the leaving group X² in the general formula (IV) include halogen atoms such as chlorine atom, bromine atom and iodine atom, sulfonyloxy such as p-toluenesulfonyloxy, benzenesulfonyloxy, methanesulfonyloxy and trifluoromethanesulfonyloxy, and the like, and chlorine atom, bromine atom, iodine atom, p-toluenesulfonyloxy and methanesulfonyloxy are particularly preferred.

The reaction of the compound represented by the general formula (IV) and the compound represented by the general formula (V) or.a salt thereof is usually performed in an inert solvent having no influence on the reaction. Examples of the inert solvent include ethers such as tetrahydrofuran, diethyl ether and dioxane, aromatic hydrocarbons such as benzene, toluene and xylene, halogenated compound type solvents such as chloroform and dichloromethane, aprotic polar solvents such as acetone, acetonitrile, dimethyl sulfoxide and N,N-dimethylformamide, mixed solvents thereof, and the like, and tetrahydrofuran, dioxane, acetone and acetonitrile are particularly preferred.

The compound represented by the general formula (V) is usually used in an amount of 1 mole to excessive moles, preferably 1 mole, with 1 mole of the compound represented by the formula (IV).

As the reaction temperature, a temperature of from room temperature to the boiling point of the solvent is usually applied, and as for the reaction time, the reaction is usually performed for 10 minutes to 48 hours. However, a reaction temperature above or below the aforementioned range, and a reaction time longer or shorter than the aforementioned range may also be applied as required.

For smooth advance of the aforementioned reaction, the reaction may also be performed in the presence of a base.

Examples of the base include, for example, inorganic bases such as sodium hydrogencarbonate, potassium hydrogencarbonate, sodium carbonate, potassium carbonate and potassium tert-butoxide, organic bases such as triethylamine, N-methylmorpholine, N,N-diisopropylethylamine and 1,8-diazabicyclo[5.4.0]undec-7-ene (DBU), and the like.

The base is usually used in an amount of 1 mole to excessive moles, preferably 1 to 10 moles, with 1 mole of the compound represented by the general formula (IV).

After completion of the reaction, the compound represented by the general formula (I) can be obtained by performing an ordinary treatment.

Isolation and purification of the compound represented by the general formula (I) or a salt thereof obtained by the aforementioned method can be achieved by, for example, any one or any suitable combination of conventional separation means such as column chromatography using silica gel, adsorption resin, or the like, liquid chromatography, solvent extraction, recrystallization and reprecipitation.

The anion represented by Z in the compound represented by the general formula (I) can be replaced with another kind of anion by a conventional means.

Examples of the method for replacing the aforementioned anion include, for example, a method of adsorbing the compound represented by the general formula (I) having a certain kind of anion on a column filled with a suitable carrier, treating the compound with an excessive amount of a salt of acid that can supply desired type of anion, and then eluting the produced compound having the desired anion, and the like.

As the compounds represented by the general formula (II), (III), (IV) or (V), for example, commercial products may be used, or they can be prepared by known methods, methods described in literature (Patent document 2), methods similar to the foregoing methods, the methods described below, the methods described in the examples and the reference examples, and the like.

The compound represented by the general formula (II):

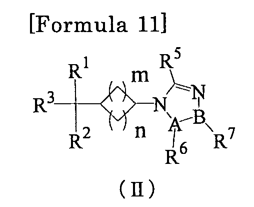

[wherein, in the formula, R¹, R², R³, R⁵, R⁶, R⁷, A, B, m and n have the same meanings as those defined above] can be prepared by the methods described below.

### Preparation Method A

By reacting a compound represented by the general formula (IV):

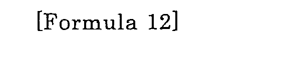

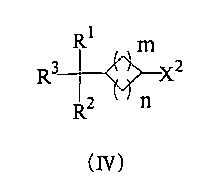

[wherein, in the formula, R¹, R², R³, m, n and X² have the same meanings as those defined above] with a compound represented by the general formula (VI):

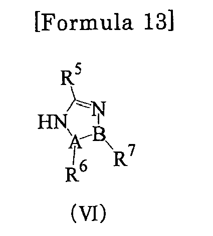

[wherein, in the formula, R⁵, R⁶, R⁷, A or B have the same meanings as those defined above] or a salt thereof, the compound represented by the general formula (II) can be prepared.

The "salt" of the compound represented by the general formula (VI) means an acid addition salt at a basic nitrogen atom, and examples include, for example, inorganic acid salts such as hydrochloride, sulfate, nitrate, and phosphate, organic acid salts such as acetate, citrate, fumarate, and tartrate, sulfonic acid salts such as methanesulfonate and tosylate, and the like.

The reaction of the compound represented by the general formula (IV) and the compound represented by the general formula (VI) or a salt thereof is usually performed in an inert solvent having no influence on the reaction. Examples of the inert solvent include ethers such as tetrahydrofuran, diethyl ether and dioxane, aromatic hydrocarbons such as benzene, toluene and xylene, halogenated compound type solvents such as chloroform and dichloromethane, aprotic polar solvents such as acetone, acetonitrile, dimethyl sulfoxide and N,N-dimethylformamide, mixed solvents thereof, and the like, and tetrahydrofuran, dioxane, toluene, acetone, acetonitrile, dimethyl sulfoxide and N,N-dimethylformamide are particularly preferred.

The compound represented by the general formula (VI) is usually used in an amount of 1 mole to excessive moles, preferably 1 mole to 10 moles, with 1 mole of the compound represented by the general formula (IV).

As the reaction temperature, a temperature of from room temperature to the boiling point of the solvent is usually applied, and as for the reaction time, the reaction is usually performed for 10 minutes to 48 hours. However, a reaction temperature above or below the aforementioned range, and a reaction time longer or shorter than the aforementioned range may also be applied as required.

For smooth advance of the aforementioned reaction, the aforementioned reaction may also be performed in the presence of a base.

Examples of the base include, for example, inorganic bases such as sodium hydrogencarbonate, potassium hydrogencarbonate, sodium carbonate, potassium carbonate, potassium tert-butoxide and sodium hydride, organic bases such as triethylamine, N-methylmorpholine, N,N-diisopropylethylamine, 1,8-diazabicyclo[5.4.0]undec-7-ene (DBU), pyridine and 4-dimethylaminopyridine, and the like.

The base is usually used in an amount of 1 mole to excessive moles, preferably 1 to 10 moles, with 1 mole of the compound represented by the general formula (VI).

After completion of the reaction, the compound represented by the general formula (II) can be obtained by performing an ordinary treatment.

### Preparation Method B

By reacting a compound represented by the general formula (VII):

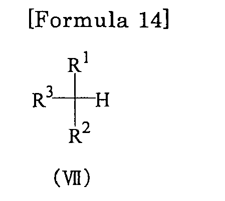

[wherein, in the formula, R¹, R² and R³ have the same meanings as those defined above] with a compound represented by the general formula (VIII):

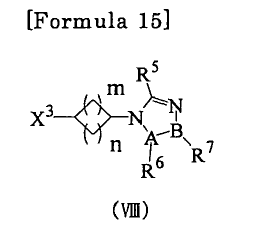

[wherein, in the formula, X³ represents a leaving group, and R⁵, R⁶, R⁷, A, B, m and n have the same meanings as those defined above] or a salt thereof in the presence of a base, the compound represented by the general formula (II) can be prepared.

Examples of the leaving group X³ in the compound represented by the general formula (VIII) include halogen atoms such as chlorine atom, bromine atom and iodine atom, sulfonyloxy such as p-toluenesulfonyloxy, benzenesulfonyloxy, methanesulfonyloxy and trifluoromethanesulfonyloxy, and the like, and chlorine atom, bromine atom, iodine atom, p-toluenesulfonyloxy and methanesulfonyloxy are particularly preferred.

The "salt" of the compound represented by the general formula (VIII) means an acid addition salt at a basic nitrogen atom, and examples include, for example, inorganic acid salts such as hydrochloride, sulfate, nitrate, and phosphate, organic acid salts such as acetate, citrate, fumarate, and tartrate, sulfonic acid salts such as methanesulfonate and tosylate, and the like.

Examples of the base include, for example, inorganic bases such as sodium hydrogencarbonate, potassium hydrogencarbonate, sodium carbonate, potassium carbonate, potassium tert-butoxide, sodium hydride, potassium hydroxide, sodium hydroxide and potassium hydroxide, organic bases such as triethylamine, N-methylmorpholine, N,N-diisopropylethylamine, 1,8-diazabicyclo[5.4.0]undec-7-ene (DBU), pyridine, 4-dimethylaminopyridine, methyllithium, n-butyllithium, s-butyllithium, t-butyllithium, phenyllithium, sodium amide and lithium diisopropylamide, and the like.

The base is usually used in an amount of 0.001 mole to excessive moles, preferably 0.1 mole to 10 moles, with 1 mole of the compound represented by the general formula (VII).

The reaction of the compound represented by the general formula (VII) and the compound represented by the general formula (VIII) or a salt thereof is usually performed in an inert solvent having no influence on the reaction. Examples of the inert solvent include ethers such as tetrahydrofuran, diethyl ether and dioxane, aromatic hydrocarbons such as benzene, toluene and xylene, halogenated compound type solvents such as chloroform and dichloromethane, aprotic polar solvents such as acetone, acetonitrile, dimethyl sulfoxide and N,N-dimethylformamide, mixed solvents thereof, and the like, and tetrahydrofuran, dioxane, toluene, acetone, acetonitrile, dimethyl sulfoxide and N,N-dimethylformamide are particularly preferred.

The compound represented by the general formula (VII) is usually used in an amount of 1 mole to excessive moles, preferably 1 mole, with 1 mole of the compound represented by the general formula (VIII).

As the reaction temperature, a temperature of from room temperature to the boiling point of the solvent is usually applied, and as for the reaction time, the reaction is usually performed for 10 minutes to 48 hours. However, a reaction temperature above or below the aforementioned range, and a reaction time longer or shorter than the aforementioned range may also be applied as required.

After completion of the reaction, the compound represented by the general formula (II) can be obtained by performing an ordinary treatment.

### Preparation Method C

As for the compound represented by the general formula (II):

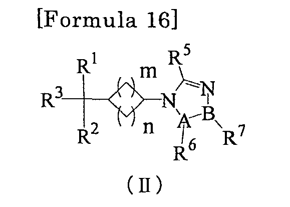

[wherein, in the formula, R¹, R², R³, R⁵, R⁶, R⁷, A, B, m and n have the same meanings as those defined above], wherein -A-B- is -CH₂-CH₂-, by treating a compound represented by the general formula (IX)

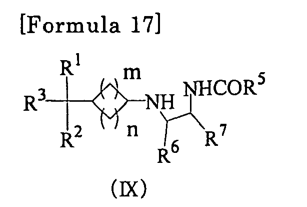

[wherein, in the formula, R¹, R², R³, R⁵, R⁶, R⁷, m and n have the same meanings as those defined above] with a halogenating agent, a compound represented by the general formula (II) [wherein, in the formula, A-B represents -CH₂-CH₂-, and R¹, R², R³, R⁵, R⁶, R⁷, m and n have the same meanings as those defined above] can be prepared.

Examples of the halogenating agent include, for example, chlorinating agents such as phosphorous oxychloride, phosphorous trichloride, sulfonyl chloride, oxalyl chloride, and carbon tetrachloride/triphenylphosphine, brominating agents such as phosphorous oxybromide, and phosphorous tetrabromide, and the like.

The reaction of the compound represented by the general formula (IX) and the halogenating agent is performed without solvent or in an inert solvent having no influence on the reaction. Examples of the inert solvent include ethers such as tetrahydrofuran, diethyl ether and dioxane, aromatic hydrocarbons such as benzene, toluene and xylene, halogenated compound type solvents such as chloroform and dichloromethane, aprotic polar solvents such as acetonitrile, mixed solvents thereof, and the like.

The halogenating agent is usually used in an amount of 1 mole to excessive moles, preferably 1 mole to 10 moles, with 1 mole of the compound represented by the general formula (IX).

As the reaction temperature, a temperature of from room temperature to the boiling point of the solvent is usually applied, and as for the reaction time, the reaction is usually performed for 10 minutes to 48 hours. However, a reaction temperature above or below the aforementioned range, and a reaction time longer or shorter than the aforementioned range may also be applied as required.

After completion of the reaction, the compound represented by the general formula (II) can be obtained by performing an ordinary treatment.

Isolation and purification of the compound represented by the general formula (II) or a salt thereof obtained by the aforementioned method can be achieved by, for example, any one or any suitable combination of conventional separation means such as column chromatography using silica gel, adsorption resin, or the like, liquid chromatography, solvent extraction, recrystallization and reprecipitation.

When the compounds of the present invention are used as medicaments, they can be orally or parenterally administered in the form of a pharmaceutical composition or a preparation (for example, tablet, solution and the like) obtained by mixing the compounds of the present invention with a pharmaceutically acceptable carrier (excipient, binder, disintegrating agent and the like). The pharmaceutical composition can be made into a pharmaceutical preparation by an ordinary method.

The compounds of the present invention have a selective antagonistic action against the muscarinic M₃ receptor, and accordingly, the compounds can be used as prophylactic and/or therapeutic agents for a disease in which the muscarinic M₃ receptor is involved. Examples of the disease in which the muscarinic M₃ receptor is involved include, for example, respiratory diseases such as chronic obstructive pulmonary disease, chronic bronchitis, asthma, chronic airway obstruction, pulmonary fibrosis, emphysema, diffuse panbronchiolitis, bronchiectasis, idiopathic interstitial pneumonia and rhinitis, preferably respiratory diseases accompanied by air flow obstruction; urologic diseases such as neurogenic pollakiuria, neurogenic bladder dysfunction, enuresis nocturna, unstable bladder, bladder spasms, chronic cystitis, urinary incontinence and/or pollakiuria in interstitial cystitis; digestive system diseases such as irritable bowel syndrome, spasticity colitis, diverticulitis, functional diarrhea, esophageal achalasia, cardiac achalasia, and biliary spasm; and central nervous system diseases such as nausea and vomition caused by drug administration, kinesia, and Meniere's disease. According to the present invention, a preferred example includes chronic obstructive pulmonary disease.

The dose may be determined depending on age, body weight, general health condition, sexuality, dietary, administration period, administration method, excretion velocity, combination with other medicaments, and severity of pathological condition of a patient under current treatment, in consideration of these factors and others. The compounds of the present invention have low toxicity and can be safely used. A daily dose may be varied depending on conditions and body weight of a patient, a type of the compound, a route of administration, and the like. However, the daily dose may be, for example, 0.01 to 1,000 mg/kg weight per day for oral administration, which is daily administered once or as several divided portions, or 0.01 to 100 mg/kg weight per day for parenteral administration, which is daily administered once or as several divided portions.

In the specification, the "prophylactic agent" is a medicament to be administered to healthy individuals whom the onset of a disease is not observed, for example, a medicament to be administered in order to prevent the onset of a disease. The "therapeutic agent" is a medicament to be administered to individuals (patients) whom the onset of a disease is diagnosed by medical practitioners, for example, a medicament to be administered for the purpose of relieving a disease or symptoms, or recovering health. Further, if a purpose of administration is prevention of aggravation of a disease or symptoms, or prevention of attacks, the medicament is a therapeutic agent so far that the medicament is administered to a patient.

### Examples

The present invention will be explained with reference to examples. However, the present invention is not limited by these examples.

### Reference Example 1

### ((3-Oxo)cyclopentyl)diphenylacetonitrile

A solution of diphenylacetonitrile (98.6 g) and 2-cyclopenten-1-one (41.9 ml) in THF (11) was added with potassium t-butoxide (0.56 g) at -10°C under an argon flow, and the mixture was stirred for 30 minutes. The reaction mixture was added with 6 N hydrochloric acid (0.83 ml), the solvent was evaporated under reduced pressure, and the resulting residue was added with water, and extracted with ethyl acetate. The organic layer was washed with saturated brine, and then dried over anhydrous sodium sulfate. The solvent was evaporated under reduced pressure, and the resulting residue was added with isopropyl ether (250 ml), and the precipitates were collected by filtration and dried to obtain 122 g (yield: 88%) of the title compound as white crystalline powder.

¹H-NMR (CDCl₃, δ PPM): 2.00 (2H, m), 2.49-2.16 (4H, m), 3.36 (1H, m), 7.51-7.29 (10H, m)

### Reference Example 2

### (cis-(3-Hydroxy)cyclopentyl)diphenylacetonitrile

A suspension of ((3-oxo)cyclopentyl)diphenylacetonitrile (22.0 g) obtained in Reference Example 1 in methanol (150 ml) was added with sodium borohydride (3.09 g) under ice cooling, the mixture was stirred for 1.5 hours, then the ice bath was removed, and the reaction mixture was left overnight at room temperature. The reaction mixture was added with ice and then with 6 N hydrochloric acid (5 ml), then the solvent was concentrated under reduced pressure, and the residue was added with water, and extracted with ethyl acetate. The organic layer was washed with water and then with saturated brine, and dried over anhydrous potassium carbonate, and then the solvent was evaporated under reduced pressure to obtain 22.3 g (yield: quantitative) of the title compound as colorless crystalline powder.

¹H-NMR (CDCl₃, δ PPM): 1.54-1.89 (5H, m), 2.07-2.17 (1H, m), 3.07-3.13 (1H, m), 4.27-4.33 (1H, m), 7.23-7.37 (6H, m), 7.43-7.46 (4H, m)

### Reference Example 3

### para-Toluenesulfonic acid (trans-3-(cyano(diphenyl)methyl)cyclopentyl) ester

A solution of (cis-(3-hydroxy)cyclopentyl)diphenylacetonitrile (15.4 g, 55.6 mmol) obtained in Reference Example 2, triphenylphosphine (21.0 g, 80.1 mmol), and para-toluenesulfonic acid ethyl ester (12.1 ml, 80.1 mmol) in tetrahydrofuran (240 ml) was added dropwise with azodicarboxylic acid diethyl ester (14.5 ml, 92.4 mmol) at - 10°C under an argon atmosphere, and the mixture was stirred at a temperature below 0°C for 2 hours, and then stirred at room temperature for 14 hour. The reaction mixture was concentrated under reduced pressure, and the resulting residue was purified by silica gel column chromatography (developing solvent: toluene) to obtain oil. The resulting oil was added with isopropyl ether and crystallized to obtain 16.7 g (yield: 70%) of the title compound as white crystalline powder.

¹H-NMR (CDCl₃, δ PPM): 1.56-2.14 (6H, m), 2.43 (3H, s), 3.28-3.52 (1H, m), 4.88-5.06 (1H, m), 7.10-7.54 (12H, m), 7.76 (2H, brd, J=8.25Hz)

### Reference Example 4

### (cis-3-(2-Methylimidazol-1-yl)cyclopentyl)diphenylacetonitrile

A suspension of para-toluenesulfonic acid (trans-3-(cyano(diphenyl)methyl)cyclopentyl) ester (66.0 g, 0.15 mol) obtained in Reference Example 3, 2-methylimidazole (25.5 g, 0.31 mol), and potassium carbonate (23.2 g, 0.17 mol) in acetonitrile (300 ml) was stirred for 16 hours under reflux by heating. The reaction mixture was concentrated under reduced pressure, added with water, and extracted with ethyl acetate, and then the organic layer was washed twice with water. The organic layer was dried over anhydrous sodium sulfate, then the solvent was evaporated under reduced pressure, and the resulting residue was purified by silica gel column chromatography (developing solvent: chloroform/methanol = 20/1 to 10/1) to obtain 36.3 g (yield: 69%) of the title compound as pale brown oil.

¹H-NMR (CDCl₃, δ PPM): 1.62-2.07 (4H, m), 2.11-2.47 (5H, m), 3.32-3.55 (1H, m), 4.57-4.73 (1H, m), 6.88 (1H, brs), 6.92 (1H, brs), 7.14-7.58 (10H, m)
MS (ESI) m/z: 342 [M+H]⁺

### Reference Example 5

### 2-(cis-3-(2-Methylimidazol-1-yl)cyclogentyl)-2,2-diphenylacetamide

A solution of (cis-3-(2-methylimidazol-1-yl)cyclopentyl)diphenylacetonitrile (36.3 g, 0.11 mol) obtained in Reference Example 4 was added with 70% aqueous sulfuric acid (57 ml), and the mixture was stirred at 140°C for 4 hours. The reaction mixture was cooled, then made basic by addition of aqueous sodium hydroxide, and then extracted with chloroform, and the organic layer was washed with saturated brine, and then dried over anhydrous sodium sulfate. The solvent was evaporated under reduced pressure, and the resulting concentrate was added with acetone (75 ml), and stirred under reflux by heating. The reaction mixture was left to cool to room temperature, and the produced precipitates were collected by filtration to obtain 18.5g (48%) of the title compound as a white crystalline compound.

¹H-NMR (CDCl₃, δ PPM): 1.52-2.14 (6H, m), 2.21 (3H, s), 3.55-3.77 (1H, m), 3.83-4.10 (1H, m), 5.44 (2H, brs), 6.90 (1H, brs), 6.96 (1H, brs), 7.15-7.49 (10H, m)
MS (ESI) m/z: 360 [M+H]⁺

### Reference Example 6

### (cis-3-(2-Isopropylimidazol-1-yl)cyclopentyl)diphenylacetonitrile

By using para-toluenesulfonic acid (trans-3-(cyano(diphenyl)methyl)cyclopentyl) ester (2.00 g, 4.63 mmol) obtained in Reference Example 3 and 2-isopropylimidazole (0.51 mg, 4.63 mmol), 1.17 g (yield: 68%) of the title compound was obtained as a colorless amorphous compound in the same manner as that of Reference Example 4.

¹H-NMR (CDCl₃, δ PPM): 1.30 (6H, t, J=7.2Hz),1.90-2.29 (6H, m), 2.92-3.05 (1H, m), 3.22-3.34 (1H, m), 4.45-4.56 (1H, m), 6.96 (1H, brs), 7.02 (1H, brs), 7.23-7.54 (10H, m)

### Reference Example 7

### 2-(cis-3-(2-Isopropylimidazol-1-yl)cyclopentyl)-2,2-diphenylacetamide

(cis-3-(2-Isopropylimidazol-1-yl)cyclopentyl)diphenylacetonitrile (0.59 g, 1.59 mmol) obtained in Reference Example 6 was used and added with 70% aqueous sulfuric acid (5.7 ml), and the mixture was stirred at 140°C for 4 hours. The reaction mixture was cooled, then made basic by addition of aqueous sodium hydroxide, and then extracted with chloroform, and the organic layer was washed with saturated brine, and then dried over anhydrous sodium sulfate. The solvent was evaporated under reduced pressure, and the resulting residue was purified by silica gel column chromatography (developing solvent: chloroform/methanol = 10/1) to obtain 0.32 g (yield: 53%) of the title compound as a colorless amorphous compound.

¹H-NMR (CDCl₃, δ PPM): 1.26-1.29 (6H, m), 1.42-1.55 (1H, m), 1.62-1.78 (2H, m), 1.94-2.14 (2H, m), 2.25-2.36 (1H, m), 2.93-3.04 (1H, m), 3.49-3.61 (1H, m), 4.42-4.53 (1H, m), 5.43 (2H, brs), 6.40 (1H, brs), 6.81 (1H, brs), 7.29-7.42 (10H, m)
MS (ESI) m/z: 388 [M+H]⁺

### Reference Example 8

### (cis-3-(4-Methylimidazol-1-yl)cyclopentyl)diphenylacetonitrile

By using para-toluenesulfonic acid (trans-3-(cyano(diphenyl)methyl)cyclopentyl) ester (2.00 g, 4.63 mmol) obtained in Reference Example 3 and 4-methylimidazole (0.38 g, 4.63 mmol), the title compound was obtained as a colorless amorphous compound in the same manner as that of Reference

### Example 4.

¹H-NMR (CDCl₃, δ PPM): 1.85-2.30 (9H, m), 3.19-3.33 (1H, m), 4.34-4.45 (1H, m), 6.72 (1H, s), 7.24-7.53 (11H, m)

### Reference Example 9

### 2-(cis-3-(2-Methylimidazol-1-yl)cyclopentyl)-2,2-diphenylacetamide

By using (cis-3-(4-methylimidazol-1-yl)cyclopentyl)diphenylacetonitrile obtained in Reference Example 8, 0.33 g (yield: 20%) of the title compound was obtained as a colorless amorphous compound in the same manner as that of Reference Example 7.

¹H-NMR (CDCl₃, δ PPM): 1.43-1.56 (1H, m), 1.58-1.76 (2H, m), 1.96-2.08 (2H, m), 2.12 (3H, s), 2.32-2.45 (1H, m), 3.46-3.64 (1H, m), 4.23-4.48 (1H, m), 5.44 (2H, brs), 6.35 (1H, brs), 7.15 (1H, brs), 7.26-7.44 (10H, m)
MS (ESI) m/z: 360 [M+H]⁺

### Reference Example 10

### (cis-3-(Imidazol-1-yl)cyclopentyl)diphenylacetonitrile

By using para-toluenesulfonic acid (trans-3-(cyano(diphenyl)methyl)cyclopentyl) ester (1.45 g, 3.36 mmol) obtained in Reference Example 3 and imidazole (0.35 g, 5.14 mmol), 0.71 g (yield: 65%) of the title compound was obtained as a colorless amorphous compound in the same manner as that of Reference Example 4.

¹H-NMR (CDCl₃, δ PPM): 1.92-2.05 (3H, m), 2.06-2.35 (3H, m), 3.22-3.34 (1H, m), 4.42-4.54 (1H, m), 7.01 (1H, brs), 7.04 (1H, brs), 7.26-7.53 (11H, m)

### Reference Example 11

### 2-(cis-3-(Imidazol-1-yl)cyclopentyl)-2,2-diphenylacetamide

By using (cis-3-(imidazol-1-yl)cyclopentyl)diphenylacetonitrile (0.71 g, 2.17 mmol) obtained in Reference Example 10, 0.42 g (yield: 56%) of the title compound was obtained as a colorless amorphous compound in the same manner as that of Reference Example 7.

¹H-NMR (CDCl₃, δ PPM): 1.45-1.58 (1H, m), 1.62-1.83 (3H, m), 1.99-2.15 (2H, m), 2.37-2.45 (1H, m), 3.50-3.62 (1H, m), 4.35-4.46 (1H, m), 5.44 (1H, brs), 5.47 (1H, brs), 6.66 (1H, brs), 6.92 (1H, brs), 7.26-7.46 (11H, m)
MS (ESI) m/z: 346 [M+H]⁺

### Reference Example 12

### (cis-3-(2-Methyl-4,5-dihydroimidazol-1-yl)cyclopentyl)diphenylacetonitrile

A solution of para-toluenesulfonic acid (trans-3-(cyano(diphenyl)methyl)cyclopentyl) ester (16.7 g, 38.8 mmol) obtained in Reference Example 3 and 2-methyl-4,5-dihydroimidazole (16.3 g, 0.19 mol) in acetonitrile (150 ml) was stirred for 7 hours under reflux by heating. The reaction mixture was concentrated under reduced pressure, added with water, and extracted with chloroform, and then the organic layer was washed twice with water. The organic layer was dried over anhydrous sodium sulfate, then the solvent was evaporated under reduced pressure, and the resulting residue was purified by silica gel column chromatography (developing solvent: chloroform/methanol = 3/1 to chloroform/methanol (0.5% triethylamine) = 2/1) to obtain oil. The resulting oil was added with isopropyl ether, and the produced white crystalline powder was collected by filtration to obtain 8.47 g (yield: 64%) of the title compound.

¹H-NMR (CDCl₃, δ PPM): 1.58-2.02 (9H, m), 3.04-3.22 (1H, m), 3.24-3.44 (2H, m), 3.52-3.74 (2H, m), 3.82-4.04 (1H, m), 7.12-7.54 (10H, m)
MS (ESI) m/z: 344 [M+H]⁺

### Reference Example 13

### 2-(cis-3-(2-Methyl-4,5-dihydroimidazol-1-yl)cyclopentyl)-2,2-diphenylacetamide

(cis-3-(2-Methyl-4,5-dihydroimidazol-1-yl)cyclopentyl)diphenylacetonitrile (0.26 g, 0.76 mmol) obtained in Reference Example 12 was used and reacted in the same manner as that of Reference Example 7, and the resulting residue was purified by silica gel column chromatography (developing solvent: chloroform/methanol = 3/1 to chloroform/methanol (0.5% triethylamine) = 2/1) to obtain 0.07 g (yield: 26%) of the title compound as a colorless amorphous compound.

¹H-NMR (CDCl₃, δ PPM): 1.18-1.57 (3H, m), 1.58-1.74 (1H, m), 1.78-2.04 (5H, m), 2.82-3.04 (2H, m), 3.30-3.57 (3H, m), 3.80-4.00 (1H, m), 5.45 (2H, brs), 7.14-7.48 (10H, m)
MS (ESI) m/z: 362 [M+H]⁺

### Reference Example 14

### (cis-3-(2-Ethylimidazol-1-yl)cyclopentyl)diphenylacetonitrile

A solution of para-toluenesulfonic acid (trans-3-(cyano(diphenyl)methyl)cyclopentyl) ester (4.3 g, 10.0 mmol) obtained in Reference Example 3, 2-ethylimidazole (1.4 g, 15.0 mmol) and potassium tert-butoxide (1.2 g, 11.0 mmol) in acetonitrile (20 ml) was stirred overnight at room temperature. The reaction mixture was concentrated under reduced pressure, added with aqueous sodium hydrogencarbonate, and extracted with ethyl acetate, and then the organic layer was washed twice with water. The organic layer was dried over anhydrous sodium sulfate, then the solvent was evaporated under reduced pressure, and the resulting residue was purified by silica gel column chromatography (developing solvent: chloroform/methanol = 100/0 to chloroform/methanol = 90/10) to obtain 0.5 g (yield: 14%) of the title compound as a pale yellow amorphous compound.

¹H-NMR (CDCl₃, δ PPM): 1.30 (3H, t, J=7.5Hz),1.83-2.28 (6H, m), 2.66 (2H, dd, J=15.1, 7.5Hz),3.20-3.35 (1H, m), 4.34-4.59 (1H, m), 6.94 (1H, d, J=1.4Hz),7.03 (1H, d, J=1.4Hz),7.16-7.55 (10H, m)
MS (ESI) m/z: 356 [M+H]⁺

### Reference Example 15

### 2-(cis-3-(2-Ethylimidazol-1-yl)cyclopentyl)-2,2-diphenylacetamide

(cis-3-(2-Ethylimidazol-1-yl)cyclopentyl)diphenylacetonitrile (5.6 g, 15.8 mmol) obtained in Reference Example 14 was used and added with 75% aqueous sulfuric acid (11 ml), and the mixture was stirred at 140°C for 2 hours. The reaction mixture was cooled, then made basic by addition of aqueous sodium hydroxide, and then extracted with chloroform, and the organic layer was washed with saturated brine, and then dried over anhydrous sodium sulfate. The solvent was evaporated under reduced pressure, and the resulting residue was purified by silica gel column chromatography (developing solvent: chloroform/methanol). The resulting concentrate was dissolved in ethyl acetate (5 ml), then the solution was added with isopropyl ether (5 ml), and the precipitated white crystals were collected by filtration to obtain 4.4 g (yield: 74%) of the title compound.

¹H-NMR (CDCl₃, δ PPM): 1.28 (3H, t, J=7.5Hz),1.35-1.56 (1H, m), 1.57-1.76 (2H, m), 1.90-2.10 (2H, m), 2.21-2.36 (1H, m), 2.52-2.70 (2H, m), 3.43-3.63 (1H, m), 4.32-4.48 (1H, m), 5.44 (2H, brs), 6.44 (1H, brs), 6.80 (1H, brs), 7.23-7.46 (10H, m)
MS (ESI) m/z: 374 [M+H]⁺

### Reference Example 16

### Diphenyl(cis-3-[1,2,4]triazol-4-yl-cyclopentyl)acetonitrile

A solution of para-toluenesulfonic acid (trans-3-(cyano(diphenyl)methyl)cyclopentyl) ester (4.32 g, 10.0 mmol) obtained in Reference Example 3 and 1,2,4-triazole (3.45 g, 50.0 mmol) in acetonitrile (20 ml) was stirred for 9 hours under reflux by heating. The reaction mixture was concentrated under reduced pressure, added with water, and then extracted with ethyl acetate, and then the organic layer was washed twice with water. The organic layer was dried over anhydrous sodium sulfate, then the solvent was evaporated under reduced pressure, and the resulting residue was purified by silica gel column chromatography [elution solvent: chloroform/methanol = 99/1 to chloroform/methanol = 95/5] to obtain 0.85 g (yield: 26%) of the 4-yl compound showing an RF value of 0.3 in silica gel TLC (developing solvent: chloroform/methanol = 10/1) as a white amorphous substance.

¹H-NMR (CDCl₃, δ PPM): 1.90-2.41 (6H, m), 3.35 (1H, m), 4.54 (1H, m), 7.26-7.50 (10H, m), 8.21 (2H, s)
MS (ESI) m/z: 329 [M+H]⁺

### Reference Example 17

### Diphenyl(cis-3-[1,2,4]triazol-1-yl-cyclopentyl)acetonitrile

The 1-yl compound was obtained as 1.20 g (yield: 37%) of a white amorphous substance showing an RF value of 0.5 in the silica gel column chromatography in Reference Example 16 [elution solvent: chloroform/methanol = 99/1 to chloroform/methanol = 95/5].

¹H-NMR (CDCl₃, δ PPM): 1.99-2.34 (6H, m), 3.31 (1H, m), 4.70 (1H, m), 7.27-7.51 (10H, m), 7.92 (1H, s), 8.11 (1H, s)
MS (ESI) m/z: 329 [M+H]⁺

### Reference Example 18

### 2,2-Diphenyl-2-(cis-3-[1,2,4]triazol-4-yl-cyclopentyl)acetamide

Diphenyl(cis-3-[1,2,4]triazol-4-yl-cyclopentyl)acetonitrile (0.90 g, 2.74 mmol) obtained in Reference Example 16 was added with 75% sulfuric acid (2 ml), and the mixture was heated to 140°C, and stirred for 2 hours. The reaction mixture was cooled, then diluted with water, made alkaline with 4 M/L sodium hydroxide, and then extracted with ethyl acetate. Then the organic layer was washed twice with water and with saturated brine. The organic layer was dried over anhydrous sodium sulfate, then the solvent was evaporated under reduced pressure, and the resulting residue was purified by silica gel column chromatography [elution solvent: chloroform/methanol = 99/1 to chloroform/methanol = 90/10] to obtain 0.68 g (yield: 71%) of the title compound as a white amorphous substance.

¹H-NMR (CDCl₃, δ PPM): 1.53 (1H, m), 1.67-1.80 (2H, m), 2.04-2.21 (2H, m), 2.49 (1H, m), 3.60 (1H, m), 4.47 (1H, m), 5.47 (1H, brs),5.63 (1H, brs),7.23-7.42 (10H, m), 7.89 (2H, s)
MS (ESI) m/z: 347 [M+H]⁺

### Reference Example 19

### 2,2-Diphenyl-2-(cis-3-[1,2,4]triazol-1-yl-cyclopentyl)acetamide

Diphenyl(cis-3-[1,2,4]triazol-1-yl-cyclopentyl)acetonitrile (1.55 g, 4.72 mmol) obtained in Reference Example 17 was added with 75% sulfuric acid (3 ml), and the mixture was heated to 140°C, and stirred for 3 hours. The reaction mixture was cooled, then diluted with water, made alkaline with 4 M/L sodium hydroxide, and then extracted with chloroform. Then, the organic layer was washed twice with water, and washed once with saturated brine. The organic layer was dried over anhydrous sodium sulfate, then the solvent was evaporated under reduced pressure, and the resulting residue was purified by silica gel column chromatography [elution solvent: chloroform/methanol = 99/1 to chloroform/methanol = 97/3] to obtain a white amorphous substance. The resulting white amorphous substance was added with ethyl acetate, and the produced white crystalline powder was collected by filtration to obtain 0.57 g (yield: 35%) of the title compound.

¹H-NMR (CDCl₃, δ PPM): 1.61-1.86 (3H, m), 2.05-2.16 (2H, m), 2.50 (1H, m), 3.57 (1H, m), 4.67 (1H, m), 5.44 (1H, brs),5.48 (1H, brs),7.27-7.42 (10H, m), 7.74 (1H, s), 7.81 (1H, s)
MS (ESI) m/z: 347 [M+H]⁺

### Reference Example 20

### ((1S,3R)-3-(2-Methylimidazol-1-yl)cyclopentyl)diphenylacetonitrile derived from (+)-dibenzoyl-D-tartaric acid

A mixture of (cis-3-(2-methylimidazol-1-yl)cyclopentyl)diphenylacetonitrile (10.0 g, 29.3 mmol) obtained in Reference Example 4 and (+)-dibenzoyl-D-tartaric acid (10.5 g, 29.3 mmol) were dissolved in ethanol (200 ml) under reflux by heating, then the solution was left at room temperature, and the produced white powder was collected by filtration (5.24 g, yield: 26%). The powder was dissolved in ethanol (100 ml) under reflux by heating, then the solution was left at room temperature, and the produced white powder was collected by filtration to obtain 4.13 g (yield: 20%, 99.7%ee) of (+)-dibenzoyl-D-tartrate of the title compound. The optical purity was determined,by high performance liquid chromatography under the following conditions.
Optical resolution column: CHIRALCEL OD, 0.46 cm Φ × 25 cm (DAICEL CHEMICAL IND., LTD.)
Eluent: n-hexane:ethanol:diethylamine = 100:10:1
Flow rate: 1.0 ml/min
Detection wavelength: 254 nm
Column temperature: 40.0°C
(+)-Dibenzoyl-D-tartrate of the title compound (4.1 g) was made basic by addition of aqueous sodium hydroxide, and then extracted with chloroform, and the organic layer was washed with saturated brine, and then dried over anhydrous magnesium sulfate. The organic layer was concentrated under reduced pressure to obtain 2.0 g (yield: quantitative) of the title compound as a colorless amorphous compound.

¹H-NMR (CDCl₃, δ PPM): 1.62-2.07 (4H, m), 2.11-2.47 (5H, m), 3.32-3.55 (1H, m), 4.57-4.73 (1H, m), 6.88 (1H, brs), 6.92 (1H, brs), 7.14-7.58 (10H, m)
MS (ESI) m/z: 342 [M+H]⁺

### Reference Example 21

### ((1R,3S)-3-(2-Methylimidazol-1-yl)cyclopentyl)diphenylacetonitrile derived from (-)-dibenzoyl-L-tartaric acid

Concentrate of the filtrate obtained in Reference Example 20 was made basic with aqueous sodium hydroxide, and then extracted with chloroform, the organic layer was dried over anhydrous sodium sulfate, and then the solvent was evaporated under reduced pressure. The resulting residue and (-)-dibenzoyl-L-tartaric acid (10.5 g, 29.3 mmol) were dissolved in ethanol (200 ml) under reflux by heating, and then the solution was left at room temperature. The produced white powder was collected by filtration, and obtained in an amount of 9.4 g. The powder was dissolved in ethanol (100 ml) under reflux by heating, then the solution was left at room temperature, and the produced white powder was collected by filtration, and obtained in an amount of 6.5 g. The same procedure was repeated by using ethanol (50 ml) to obtain 6.5 g (yield: 32%, 98.3%ee) of (-)-dibenzoyl-L-tartrate of the title compound as white crystalline powder.
The optical purity was determined under the same conditions as those of Reference Example 20.

(-)-Dibenzoyl-L-tartrate of the title compound (6.5 g) was made basic by addition of aqueous sodium hydroxide, and then extracted with chloroform, and the organic layer was washed with saturated brine, and then dried over anhydrous magnesium sulfate. The solvent was concentrated under reduced pressure to obtain 3.2 g (yield: quantitative) of the title compound as a colorless amorphous compound.

¹H-NMR (CDCl₃, δ PPM): 1.62-2.07 (4H, m), 2.11-2.47 (5H, m), 3.32-3.55 (1H, m), 4.57-4.73 (1H, m), 6.88 (1H, brs), 6.92 (1H, brs), 7.14-7.58 (10H, m)
MS (ESI) m/z: 342 [M+H]⁺

### Reference Example 22

### 2-((1S,3R)-3-(2-Methylimidazol-1-yl)cyclopentyl)-2,2-diphenylacetamide derived from (+)-dibenzoyl-D-tartaric acid

((1S,3R)-3-(2-Methylimidazol-1-yl)cyclopentyl)diphenylacetonitrile (98 g, 0.29 mol) obtained in Reference Example 20 and 80% sulfuric acid (200 ml) were stirred at 140°C for 5.5 hours. The reaction mixture was cooled, then made basic by addition of aqueous sodium hydroxide, and then extracted with ethyl acetate, and the organic layer was washed with saturated brine, and then dried over anhydrous sodium sulfate. The solvent was evaporated under reduced pressure, and a solution of the resulting residue in toluene (1000 ml) was purified to obtain 60.7 g (yield: 58%) of the title compound as a white crystalline compound.

¹H-NMR (CDCl₃, δ PPM): 1.52-2.14 (6H, m), 2.21 (3H, s), 3.55-3.77 (1H, m), 3.83-4.10 (1H, m), 5.44 (2H, brs), 6.90 (1H, brs), 6.96 (1H, brs), 7.15-7.49 (10H, m)
MS (ESI) m/z: 360 [M+H]⁺

### Reference Example 23

### 2-((1R,3S)-3-(2-Methylimidazol-1-yl)cyclopentyl)-2,2-diphenylacetamide derived from (-)-dibenzoyl-L-tartaric acid

By using ((1R,3S)-3-(2-methylimidazol-1-yl)cyclopentyl)diphenylacetonitrile (11 g, 32 mmol) obtained in Reference Example 21 and 75% sulfuric acid (54 ml), 9.1 g (yield: 80%) of the title compound was obtained in the same manner as that of Reference Example 7.

¹H-NMR (CDCl₃, δ PPM): 1.52-2.14 (6H, m), 2.21 (3H, s), 3.55-3.77 (1H, m), 3.83-4.10 (1H, m), 5.44 (2H, brs), 6.90 (1H, brs), 6.96 (1H, brs), 7.15-7.49 (10H, m)
MS (ESI) m/z: 360 [M+H]⁺

### Reference Example 24

### ((1S,3R)-3-(2-Methyl-4,5-dihydroimidazol-1-yl)cyclopentyl)diphenylacetonitrile derived from (+)-dibenzoyl-D-tartaric acid

Concentrate of the filtrate obtained in Reference Example 25 was made basic with aqueous sodium hydroxide, and then extracted with chloroform, the organic layer was dried over anhydrous sodium sulfate, and then the solvent was evaporated under reduced pressure. The resulting residue and (+)-dibenzoyl-D-tartaric acid (3.32 g, 18.5 mmol) were dissolved in ethanol (375 ml) under reflux by heating, then the solution was left at room temperature, and the produced white powder was collected by filtration to 2.77 g (yield: 30%, 76.7%ee) of the powder. The powder was dissolved in ethanol (180 ml) under reflux by heating, then the solution was left at room temperature, and the produced white powder was collected by filtration to obtain 1.66 g of the powder. The same procedure was repeated by using ethanol (100 ml) to obtain 1.08 g (yield: 8%, 99.9%ee) of (+)-dibenzoyl-D-tartrate of the title compound as colorless crystalline powder.
The optical purity was determined under the same conditions as those of Reference Example 20.

(+)-Dibenzoyl-D-tartrate of the title compound (1.06 g, 1.51 mmol) was made basic by addition of aqueous sodium hydroxide, and then extracted with chloroform, and the organic layer was washed with saturated brine, and then dried over anhydrous magnesium sulfate. The organic layer was concentrated under reduced pressure to obtain 0.52 g (yield: quantitative) of the title compound as a colorless amorphous compound.

¹H-NMR (CDCl₃, δ PPM): 1.58-2.02 (9H, m), 3.04-3.22 (1H, m), 3.24-3.44 (2H, m), 3.52-3.74 (2H, m), 3.82-4.04 (1H, m), 7.12-7.54 (10H, m), 10.5 (2H, brs)
MS (ESI) m/z: 344 [M+H]⁺

### Reference Example 25

### ((1R,3S)-3-(2-Methyl-4,5-dihydroimidazol-1-yl)cyclopentyl)diphenylacetonitrile derived from (-)-dibenzoyl-L-tartaric acid

A mixture of (cis-3-(2-methyl-4,5-dihydroimidazol-1-yl)cyclopentyl)diphenylacetonitrile (6.36 g, 18.5 mmol) obtained in Reference Example 12 and (-)-dibenzoyl-L-tartaric acid (3.32 g, 18.5 mmol) was dissolved in ethanol (400 ml) under reflux by heating, and then the solution was left at room temperature. The produced white powder was collected by filtration to obtain 3.73 g of the powder. The powder was dissolved in ethanol (400 ml) under reflux by heating, and then the solution was left at room temperature. The produced white powder was collected by filtration to obtain 1.84 g of the powder. The same procedure was repeated by using ethanol (180 ml) to obtain 0.98 g (yield: 8%, 98.5%ee) of (-)-dibenzoyl-L-tartrate of the title compound as colorless crystalline powder.
The optical purity was determined under the same conditions as those of Reference Example 20.
(-)-Dibenzoyl-L-tartrate of the title compound (0.98 g, 1.38 mmol) was made basic by addition of aqueous sodium hydroxide, and then extracted with chloroform, and the organic layer was washed with saturated brine, and then dried over anhydrous magnesium sulfate. The solvent was concentrated under reduced pressure to obtain 0.48 g (yield: quantitative) of the title compound as a colorless amorphous compound.

¹H-NMR (CDCl₃, δ PPM): 1.58-2.02 (9H, m), 3.04-3.22 (1H, m), 3.24-3.44 (2H, m), 3.52-3.74 (2H, m), 3.82-4.04 (1H, m), 7.12-7.54 (10H, m)
MS (ESI) m/z: 344 [M+H]⁺

### Reference Example 26

### ((1S,3R)-3-(2-Methyl-4,5-dihydroimidazol-1-yl)cyclopentyl)diphenylacetamide derived from (+)-dibenzoyl -D-tartaric acid

A solution of ((1S,3R)-3-(2-methyl-4,5-dihydroimidazol-1-yl)cyclopentyl)diphenylacetonitrile (0.52 g, 1.51 mmol) obtained in Reference Example 24 in acetic acid (5 ml) was added with 70% aqueous sulfuric acid (11.6 ml), and stirred at 140°C for 4 hours. The reaction mixture was cooled, then made basic by addition of aqueous sodium hydroxide, and then extracted with chloroform, and the organic layer was washed with saturated brine, and then dried over anhydrous sodium sulfate. The solvent was evaporated under reduced pressure, and the resulting residue was purified by silica gel column chromatography (developing solvent: chloroform/methanol = 3/1 to chloroform/methanol (0.5% triethylamine) = 1/1 to 1/10) to obtain 0.5 g (yield: 92%) of the title compound as a colorless amorphous compound.

¹H-NMR (CDCl₃, δ PPM): 1.18-1.57 (3H, m), 1.58-1.74 (1H, m), 1.78-2.04 (5H, m), 2.82-3.04 (2H, m), 3.30-3.57 (3H, m), 3.80-4.00 (1H, m), 5.45 (2H, brs), 7.14-7.48 (10H, m)
MS (ESI) m/z: 362 [M+H]⁺

### Reference Example 27

### 2-((1R,3S)-3-(2-Methyl-4,5-dihydroimidazol-1-yl)cyclopentyl)-2,2-diphenylacetamide derived from (-)-dibenzoyl-L-tartaric acid

A solution of ((1R,3S)-3-(2-methyl-4,5-dihydroimidazol-1-yl)cyclopentyl)diphenylacetonitrile (0.48 g, 1.38 mmol) obtained in Reference Example 25 in acetic acid (5 ml) was added with 70% aqueous sulfuric acid (11.6 ml), and the mixture was stirred at 140°C for 4 hours. The reaction mixture was cooled, then made basic by addition of aqueous sodium hydroxide, and then extracted with chloroform, and the organic layer was washed with saturated brine, and then dried over anhydrous sodium sulfate. The solvent was evaporated under reduced pressure, and the resulting residue was purified by silica gel column chromatography (developing solvent: chloroform/methanol = 3/1 to chloroform/methanol (0.5% triethylamine) = 1/1 to 1/10) to obtain 0.43 g (yield: 86%) of the title compound as a colorless amorphous compound.

¹H-NMR (CDCl₃, δ PPM): 1.18-1.57 (3H, m), 1.58-1.74 (1H, m), 1.78-2.04 (5H, m), 2.82-3.04 (2H, m), 3.30-3.57 (3H, m), 3.80-4.00 (1H, m), 5.45 (2H, brs), 7.14-7.48 (10H, m)
MS (ESI) m/z: 362 [M+H]⁺

### Reference Example 28

### para-Toluenesulfonic acid (cis-3-(cyano(diphenyl)methyl)cyclopentyl) ester

A solution.of (cis-(3-hydroxy)cyclopentyl)diphenylacetonitrile (27.7 g, 100 mmol) obtained in Reference Example 2 and para-toluenesulfonyl chloride (23 g, 120 mmol) in pyridine (100 ml) was stirred at room temperature for 6 hours. The reaction mixture was added with water, and extracted with ethyl acetate, and the organic layer was washed with 1 N hydrochloric acid, and then dried over anhydrous magnesium sulfate. The organic layer was filtered, and then concentrated under reduced pressure, and the resulting powder was washed with a mixed solvent ethyl acetate/isopropyl ether to obtain 32.0 g (yield: 74%) of the title compound.

¹H-NMR (CDCl₃, δ PPM): 1.58-2.09 (6H, m), 2.42 (3H, s), 2.90-3.06 (1H, m), 4.88-5.01 (1H, m), 7.16-7.44 (12H, m), 7.77 (2H, d, J=8.3Hz).

### Reference Example 29

### (trans-3-(2-Methylimidazol-1-yl)cyclopentyl)diphenylacetonitrile

A solution of para-toluenesulfonic acid (cis-3-(cyano(diphenyl)methyl)cyclopentyl) ester (32 g, 74.0 mmol) obtained in Reference Example 28 and 2-methylimidazole (30.4 g, 370 mmol) in acetonitrile (150 ml) was stirred for 6 hours under reflux by heating. The reaction mixture was concentrated under reduced pressure, added with water, and extracted with ethyl acetate, and then the organic layer was washed twice with water. The organic layer was dried over anhydrous magnesium sulfate, and then the solvent was evaporated under reduced pressure. The resulting residue was purified by silica gel column chromatography (developing solvent: chloroform/methanol = 20/1) to obtain 18.5 g (yield: 73%) of the title compound as a colorless amorphous compound.

¹H-NMR (CDCl₃, δ PPM): 1.70-2.04 (4H, m), 2.11-2.38 (5H, m), 3.32-3.50 (1H, m), 4.52-4.68 (1H, m), 6.88 (1H, s), 6.93 (1H, s), 7.18-7.52 (10H, m)

### Reference Example 30

### 2-(trans-3-(2-Methylimidazol-1-yl)cyclopentyl)-2,2-diphenylacetamide

(trans-3-(2-Methylimidazol-1-yl)cyclopentyl)diphenylacetonitrile (14.6 g, 42.7 mmol) obtained in Reference Example 29 was added with 75% aqueous sulfuric acid (20 ml), and the mixture was stirred at 140°C for 6 hours. The reaction mixture was cooled, then made basic by addition of aqueous sodium hydroxide, and then extracted with ethyl acetate. The organic layer was washed with saturated brine, and then dried over anhydrous magnesium sulfate. The solvent was evaporated under reduced pressure, and the resulting residue was purified by silica gel column chromatography (developing solvent: chloroform/methanol = 20/1) to obtain 11.7 g (yield: 76%) of the title compound as a colorless amorphous compound.

¹H-NMR (CDCl₃, δ PPM): 1.56-1.84 (2H, m), 1.86-2.14 (4H, m), 2.20 (3H, s), 3.56-3.76 (1H, m), 3.82-3.99 (1H, m), 5.45 (1H, brs), 5.64 (1H, brs), 6.88 (1H, brs), 6.95 (1H, brs), 7.22-7.42 (10H, m)

### Reference Example 31

### (trans-3-(4-Methylimidazol-1-yl)cyclopentyl)diphenylacetonitrile

A suspension of para-toluenesulfonic acid (cis-3-(cyano(diphenyl)methyl)cyclopentyl) ester (21.6 g, 50.0 mmol) obtained in Reference Example 28, 4-methylimidazole (20.5 g, 250 mmol), and potassium carbonate (7 g) in acetonitrile (150 ml) was stirred for 6 hours under reflux by heating. The reaction mixture was concentrated under reduced pressure, added with water, and extracted with ethyl acetate, and then the organic layer was washed twice with water. The organic layer was dried over anhydrous magnesium sulfate, then the solvent was evaporated under reduced pressure, and the resulting residue was purified by silica gel column chromatography (developing solvent: ethyl acetate/methanol) to obtain 13 g (yield: 76%) of the title compound as colorless oil.

¹H-NMR (CDCl₃, δ PPM): 1.69-2.42 (9H, m), 3.26-3.46 (1H, m), 4.43-4.60 (1H, m), 6.64 (1H, s), 7.11-7.48 (11H, m)

### Reference Example 32

### 2-(trans-3-(4-Methylimidazol-1-yl)cyclopentyl)-2,2-diphenylacetamide

(trans-3-(4-Methylimidazol-1-yl)cyclopentyl)diphenylacetonitrile (13 g, 38.1 mmol) obtained in Reference Example 31 was added with 80% aqueous sulfuric acid (36 ml), and the mixture was stirred at 140°C for 6 hours. The reaction mixture was cooled, then made basic by addition of aqueous sodium hydroxide, and then extracted with ethyl acetate, and the organic layer was washed with saturated brine, and then dried over anhydrous magnesium sulfate. The solvent was evaporated under reduced pressure, and the resulting powder was washed with ethanol to obtain 3.4 g (yield: 25%) of the title compound as a white crystalline compound.

¹H-NMR (CDCl₃, δ PPM): 1.22-1.38 (1H, m), 1.58-1.86 (3H, m), 1.87-2.10 (5H, m), 3.64-3.86 (2H, m), 6.83 (1H, brs), 6.88 (1H, s), 7.12 (1H, brs), 7.17-7.36 (10H, m), 7.42 (1H, s)

### Reference Example 33

### (trans-3-(Imidazol-1-yl)cyclopentyl)diphenylacetonitrile

A suspension of para-toluenesulfonic acid (cis-3-(cyano(diphenyl)methyl)cyclopentyl) ester (21.6 g, 50.0 mmol) obtained in Reference Example 28, imidazole (17.0 g, 250 mmol), and potassium carbonate (7 g) in tetrahydrofuran (150 ml) was stirred for 8.5 hours under reflux by heating. The reaction mixture was concentrated under reduced pressure, added with water, and extracted with ethyl acetate, and then the organic layer was washed twice with water. The organic layer was dried over anhydrous magnesium sulfate, and then the solvent was evaporated under reduced pressure. The resulting residue was purified by silica gel column chromatography (developing solvent: ethyl acetate/methanol) to obtain 14.4 g (yield: 88%) of the title compound as colorless oil.

¹H-NMR (CDCl₃, δ PPM): 1.68-2.42 (6H, m), 3.29-3.48 (1H, m), 4.51-4.70 (1H, m), 6.93 (1H, brs), 7.06 (1H, brs), 7.14-7.53 (11H, m)

### Reference Example 34

### 2-(trans-3-(Imidazol-1-yl)cyclopentyl)-2,2-diphenylacetamide

(trans-3-(Imidazol-1-yl)cyclopentyl)diphenylacetonitrile (14.4 g, 43.9 mmol) obtained in Reference Example 33 was added with 80% aqueous sulfuric acid (29 ml), and the mixture was stirred at 140°C for 6 hours. The reaction mixture was cooled, then made basic by addition of aqueous sodium hydroxide, and then extracted with ethyl acetate, and the organic layer was washed with saturated brine, and then dried over anhydrous magnesium sulfate. The solvent was evaporated under reduced pressure, the resulting residue was added with a mixed solution of ethyl acetate/isopropyl ether (1:1), and the precipitated powder was collected by filtration to obtain 11.6 g (yield: 76%) of the title compound as a white crystalline compound.

¹H-NMR (CDCl₃, δ PPM): 1.58-190 (2H, m), 1.92-2.24 (4H, m), 3.46-3.65 (1H, m), 4.01-4.16 (1H, m), 5.48 (1H, brs), 5.87 (1H, brs), 6.93 (1H, s), 7.01 (1H, s), 7.16-7.36 (10H, m), 7.46 (1H, s).

### Reference Example 35

### 2-(trans-3-(2-Methyl-4,5-dihydroimidazol-1-yl)cyclopentyl)-2,2-diphenylacetonitrile

A solution of para-toluenesulfonic acid (cis-3-(cyano(diphenyl)methyl)cyclopentyl) ester (77.2 g, 0.18 mol) obtained in Reference Example 28 and 2-methyl-4,5-dihydroimidazole (150 g, 1.8 mmol) in toluene (600 ml) was stirred for 2 hours under reflux by heating. The reaction mixture was added with water, and extracted with ethyl acetate, and then the organic layer was washed twice with water, and dried over anhydrous magnesium sulfate. Then, the solvent was evaporated under reduced pressure, the resulting residue was added with a mixed solution of ethyl acetate/hexane, and the precipitated powder was collected by filtration to obtain 36.3 g (yield: 59%) of the title compound as a pale yellow crystalline compound.

¹H-NMR (DMSO-d₆, δ PPM): 1.29-1.94 (9H, m), 3.12-3.65 (5H, m), 4.00-4.14 (1H, m), 7.25-7.34 (2H, m), 7.35-7.45 (4H, m), 7.46-7.58 (4H, m)
MS (ESI) m/z: 343 [M+H]⁺

### Reference Example 36

### (trans-3-(2-Methylimidazol-1-yl)cyclopentyl)diphenylacetonitrile derived from L-(+)-mandelic acid

(trans-3-(2-Methylimidazol-1-yl)cyclopentyl)diphenylacetonitrile (52 g, 152 mmol) obtained in Reference Example 29 and L-(+)-mandelic acid (23.1 g, 152 mmol) were dissolved in methanol (200 ml) under reflux by heating, and then the solution was concentrated. Then, the solvent was replaced with a solution of ethyl acetate/ethanol (10:1), and the residue was dissolved under reflux by heating. Then, the solution was left at room temperature. The produced white powder was collected by filtration to obtain L-(+)-mandelate of the title compound.

L-(+)-Mandelate of the title compound was made basic by addition of aqueous sodium hydroxide, and then extracted with ethyl acetate, and the organic layer was washed with saturated brine, and then dried over anhydrous magnesium sulfate. The organic layer was concentrated under reduced pressure to obtain 17 g (yield: 33%, 97%ee) of the title compound as a colorless amorphous compound.
The optical purity was determined under the same conditions as those of Reference Example 20.

¹H-NMR (CDCl₃, δ PPM): 1.70-2.04 (4H, m), 2.11-2.38 (5H, m), 3.32-3.50 (1H, m), 4.52-4.68 (1H, m), 6.88 (1H, s), 6.93 (1H, s), 7.18-7.52 (10H, m)

### Reference Example 37

### (trans-3-(2-Methylimidazol-1-yl)cyclopentyl)diphenylacetonitrile derived from D-(-)-mandelic acid

Concentrate of the filtrate obtained in Reference Example 36 was made basic with aqueous sodium hydroxide, and then extracted with chloroform, the organic layer was dried over anhydrous sodium sulfate, and then the solvent was evaporated under reduced pressure. The resulting residue and D-(-)-mandelic acid (25 g, 164 mmol) were dissolved in methanol (200 ml) under reflux by heating, and then the solution was concentrated. Then, the solvent was replaced with ethyl acetate, the solution was left in a refrigerator, and the produced white powder was collected by filtration to obtain 42 g (93%ee) of D-(-)-mandelate of the title compound. The resulting white powder was dissolved in an ethyl acetate/ethanol (10:1) solution, the solution was left at room temperature, and the precipitated crystals were collected by filtration to obtain D-(-)-mandelate of the title compound (> 99%ee). The resulting compound was made basic by addition of aqueous sodium hydroxide, and then extracted with ethyl acetate, and the organic layer was washed with saturated brine, and then dried over anhydrous magnesium sulfate. The organic layer was concentrated under reduced pressure to obtain 24 g (yield: 46%) of the title compound as yellow oil.
The optical purity was determined under the same conditions as those of Reference Example 20.

¹H-NMR (CDCl₃, δ PPM): 1.70-2.04 (4H, m), 2.11-2.38 (5H, m), 3.32-3.50 (1H, m), 4.52-4.68 (1H, m), 6.88 (1H, s), 6.93 (1H, s), 7.18-7.52 (10H, m)

### Reference Example 38

### 2-(trans-3-(2-Methylimidazol-1-yl)cyclopentyl)-2,2-diphenylacetamide derived from L-(+)-mandelic acid

(trans-3-(2-Methylimidazol-1-yl)cyclopentyl)diphenylacetonitrile (17 g, 49.7 mmol) obtained in Reference Example 36 and 75% sulfuric acid (40 ml) were stirred at 140°C for 4 hours. The reaction mixture was cooled, then made basic by addition of aqueous sodium hydroxide, and then extracted with ethyl acetate, and the organic layer was washed with saturated brine, and then dried over anhydrous sodium sulfate. The solvent was evaporated under reduced pressure, and the resulting residue was purified by silica gel column chromatography (developing solvent: chloroform/methanol = 30/1). Crystals of the purification product precipitated from toluene were collected by filtration, and washed with isopropyl ether to obtain 7.8 g (yield: 44%) of the title compound as a white crystalline compound.

¹H-NMR (CDCl₃, δ PPM): 1.56-1.84 (2H, m), 1.86-2.14 (4H, m), 2.20 (3H, s), 3.56-3.76 (1H, m), 3.82-3.99 (1H, m), 5.45 (1H, brs), 5.64 (1H, brs), 6.88 (1H, brs), 6.95 (1H, brs), 7.22-7.42 (10H, m)

MS (ESI) m/z: 360 [M+H]⁺

### Reference Example 39

### 2-(trans-3-(2-Methylimidazol-1-yl)cyclopentyl)-2,2-diphenylacetamide derived from D-(-)-mandelic acid

(trans-3-(2-Methylimidazol-1-yl)cyclopentyl)diphenylacetonitrile (24 g, 70.2 mmol) obtained in Reference Example 37 and 75% sulfuric acid (38 ml) were stirred at 140°C for 3.5 hours. The reaction mixture was cooled, then made basic by addition of aqueous sodium hydroxide, and then extracted with ethyl acetate, and the organic layer was washed with saturated brine, and then dried over anhydrous sodium sulfate. The solvent was evaporated under reduced pressure, and the resulting residue was purified by silica gel column chromatography (developing solvent: chloroform/methanol = 30/1). Crystals of the purification product precipitated from toluene were collected by filtration, and washed with isopropyl ether to obtain 15.3 g (yield: 61%) of the title compound as a white crystalline compound.

¹H-NMR (CDCl₃, δ PPM): 1.56-1.84 (2H, m), 1.86-2.14 (4H, m), 2.20 (3H, s), 3.56-3.76 (1H, m), 3.82-3.99 (1H, m), 5.45 (1H, brs), 5.64 (1H, brs), 6.88 (1H, brs), 6.95 (1H, brs), 7.22-7.42 (10H, m)

MS (ESI) m/z: 360 [M+H]⁺

### Reference Example 40

### (trans-3-(2-Methyl-4,5-dihydroimidazol-1-yl)cyclopentyl)diphenylacetonitrile derived from (+)-dibenzoyl-D-tartaric acid

A mixture of 2-(trans-3-(2-methyl-4,5-dihydroimidazol-1-yl)cyclopentyl)-2,2-diphenylacetonitrile (41.4 g, 121 mmol) obtained in Reference Example 35 and (+)-dibenzoyl-D-tartaric acid [(43.2 g, 121 mmol) were dissolved in ethanol (480 ml) under reflux by heating, and then the solution was left at room temperature. The produced white powder was collected by filtration, and washed with methanol to obtain 14.9 g (99%ee) of (+)-dibenzoyl-D-tartrate of the title compound.

(+)-Dibenzoyl-D-tartrate of the title compound (14.9 g) was made basic by addition of aqueous sodium hydroxide, and then extracted with chloroform, and the organic layer was washed with saturated brine, and then dried over anhydrous magnesium sulfate. The organic layer was concentrated under reduced pressure to obtain 10.7 g (yield: 26%) of the title compound as yellow oil.
The optical purity was determined under the same conditions as those of Reference Example 20.

¹H-NMR (DMSO-d₆, δ PPM): 1.29-1.94 (9H, m), 3.12-3.65 (5H, m), 4.00-4.14 (1H, m), 7.25-7.34 (2H, m), 7.35-7.45 (4H, m), 7.46-7.58 (4H, m)
MS (ESI) m/z: 343 [M+H]⁺

### Reference Example 41

### (trans-3-(2-Methyl-4,5-dihydroimidazol-1-yl)cyclopentyl)diphenylacetonitrile derived from (-)-dibenzoyl-L-tartaric acid

Concentrate of the filtrate obtained in Reference Example 40 was made basic with aqueous sodium hydroxide, and then extracted with chloroform, the organic layer was dried over anhydrous sodium sulfate, and then the solvent was evaporated under reduced pressure. The resulting residue and (-)-dibenzoyl-L-tartaric acid (36.1 g, 100 mmol) were dissolved in ethanol (400 ml) under reflux by heating, and then the solution was left at room temperature. The produced white powder was collected by filtration, and washed with methanol to obtain 12 g (99%ee) of (-)-dibenzoyl-L-tartrate the title compound.

(-)-Dibenzoyl-L-tartrate of the title compound (12 g) was made basic by addition of aqueous sodium hydroxide, and then extracted with chloroform, and the organic layer was washed with saturated brine, and then dried over anhydrous magnesium sulfate. The organic layer was concentrated under reduced pressure to obtain 8.5 g (yield: 25%) of the title compound as yellow oil.
The optical purity was determined under the same conditions as those of Reference Example 20.

¹H-NMR (DMSO-d₆, δ PPM): 1.29-1.94 (9H, m), 3.12-3.65 (5H, m), 4.00-4.14 (1H, m), 7.25-7.34 (2H, m), 7.35-7.45 (4H, m), 7.46-7.58 (4H, m)
MS (ESI) m/z: 343 [M+H]⁺

### Reference Example 42

### 2-(trans-3-(2-Methyl-4,5-dihydroimidazol-1-yl)cyclopentyl)-2,2-diphenylacetamide derived from (+)-dibenzoyl-D-tartaric acid

(trans-3-(2-Methyl-4,5-dihydroimidazol-1-yl)cyclopentyl)diphenylacetonitrile (10.7 g, 31.2 mmol) obtained in Reference Example 40 and 80% sulfuric acid (54 ml) were stirred at 140°C for 2 hours. The reaction mixture was cooled, then made basic by addition of aqueous sodium hydroxide, and then extracted with chloroform, and the organic layer was washed with saturated brine, and then dried over anhydrous sodium sulfate. The solvent was evaporated under reduced pressure to obtain 8.8 g (yield: 79%) of the title compound as a dark brown amorphous compound.

MS (ESI) m/z: 361 [M+H]⁺

### Reference Example 43

### 2-(trans-3-(2-Methyl-4,5-dihydroimidazol-1-yl)cyclopentyl)-2,2-diphenylacetamide derived from (-)-dibenzoyl-L-tartaric acid

(trans-3-(2-Methyl-4,5-dihydroimidazol-1-yl)cyclopentyl)diphenylacetonitrile (8.5 g, 24.9 mmol) obtained in Reference Example 41 and 80% sulfuric acid (43 ml) were stirred at 140°C for 2 hours. The reaction mixture was cooled, then made basic by addition of aqueous sodium hydroxide, and then extracted with chloroform, and the organic layer was washed with saturated brine, and then dried over anhydrous sodium sulfate. The solvent was evaporated under reduced pressure to obtain 6.8 g (yield: 76%) of the title compound as a dark brown amorphous compound.

MS (ESI) m/z: 361 [M+H]⁺

### Example 1

### 3-(cis-3-(Carbamoyl(diphenyl)methyl)cyclopentyl)-1,2-dimethyl-3H-imidazol-1-ium iodide

A solution of (cis-3-(2-methylimidazol-1-yl)cyclopentyl)diphenylacetamide obtained in Reference Example 5 and methyl iodide (0.2 ml) in acetone (5 ml) was stirred overnight at room temperature. The precipitates in the reaction mixture were collected by filtration to obtain 0.28 g (yield: 8%) of the title compound as a pale yellow amorphous compound.

¹H-NMR (CDCl₃, δ PPM): 1.64-2.38 (5H, m), 2.40-2.50 (1H, m), 2.84 (3H, s), 3.58-3.70 (1H, m), 3.90 (3H, s), 4.89-5.01 (1H, m), 5.70 (1H, brs), 5.89 (1H, brs), 6.96 (1H, brs), 7.20-7.42 (11H, m)
MS (ESI) m/z: 374 [M]⁺

### Example 2

### 3-(cis-3-(Cyano(diphenyl)methyl)cyclopentyl)-2-isopropyl-1-methyl-3H-imidazol-1-ium iodide

By using (cis-3-(2-isopropylimidazol-1-yl)cyclopentyl)diphenylacetonitrile (0.56 g, 1.51 mmol) obtained in Reference Example 6, 0.77 g (yield: 95%) of the title compound was obtained as a pale yellow amorphous compound in the same manner as that of Example 1.

¹H-NMR (CDCl₃, δ PPM): 1.47-1.55 (6H, m), 1.75-1.84 (1H, m), 1.85-1.95 (1H, m), 2.01-2.20 (2H, m), 2.42-2.54 (2H, m), 3.82-3.92 (1H, m), 4.01 (3H, s), 4.08-4.18 (1H, m), 5.58-5.66 (1H, m), 7.21-7.28 (2H, m), 7.31-7.36 (5H, m), 7.48 (1H, brs), 7.52-7.63 (4H, m)
MS (ESI) m/z: 384 [M]⁺

### Example 3

### 3-(cis-3-(Carbamoyl(diphenyl)methyl)cyclopentyl)-2-isopropyl-1-methyl-3H-imidazol-1-ium iodide

By using (cis-3-(2-isopropylimiazol-1-yl)cyclopentyl)diphenylacetamide (0.24 g, 0.63 mmol) obtained in Reference Example 7, 0.27 g (yield: 80%) of the title compound was obtained as a pale yellow amorphous compound in the same manner as that of Example 1.

¹H-NMR (CDCl₃, δ PPM): 1.49 (3H, s), 1.51 (3H, s), 1.63-2.03 (3H, m), 2.20-2.34 (2H, m), 2.37-2.50 (1H, m), 3.65-3.75 (1H, m), 3.80-3.93 (1H, m), 4.00 (3H, s), 5.01-5.12 (1H, m), 5.74 (1H, brs), 5.85 (1H, brs), 6.96 (1H, brs), 7.23-7.41 (10H, m), 7.55 (1H, brs)
MS (ESI) m/z: 402 [M]⁺

### Example 4

### 3-(cis-3-(Carbamoyl(diphenyl)methyl)cyclopentyl)-1,5-dimethyl-3H-imidazol-1-ium iodide

By using (cis-3-(5-methylimidazol-1-yl)cyclopentyl)diphenylacetamide (0.20 g, 0.56 mmol) obtained in Reference Example 9, 0.17 g (yield: 60%) of the title compound was obtained as a pale yellow amorphous compound in the same manner as that of Example 1.

¹H-NMR (CDCl₃, δ PPM): 1.81-2.50 (9H, m), 3.42-3.57 (1H, m), 3.86 (3H, s), 4.90-5.03 (1H, m), 5.55 (1H, brs), 5.68 (1H, brs), 6.89 (1H, s), 7.21-7.41 (10H, m), 10.0 (1H, s)
MS (ESI) m/z: 374 [M]⁺

### Example 5

### 3-(cis-3-(Carbamoyl(diphenyl)methyl)cyclopentyl)-1-methyl-3H-imidazol-1-ium iodide

By using (cis-3-(3-imidazol-1-yl)cyclopentyl)diphenylacetamide (0.30 g, 0.87 mmol) obtained in Reference Example 11, 0.18 g (yield: 42%) of the title compound was obtained as a colorless powdery compound in the same manner as that of Example 1.

¹H-NMR (DMSO-d₆, δ PPM): 1.26-1.52 (3H, m), 1.92-2.05 (1H, m), 2.08-2.19 (1H, m), 2.45-2.58 (1H, m), 3.64-3.74 (1H, m), 3.75 (3H, s), 4.60-4.73 (1H, m), 6.74 (1H, brs), 7.17 (1H, brs), 7.21-7.38 (10H, m), 7.39 (1H, brs), 7.59 (1H, brs), 8.89 (1H, brs)
MS (ESI) m/z: 360 [M]⁺

### Example 6

### 1-Benzyl-3-(cis-3-(carbamoyl(diphenyl)methyl)cyclopentyl)-2-methyl-1-3H-imidazol-1-ium bromide

A solution of (cis-3-(2-methylimidazol-1-yl)cyclopentyl)diphenylacetamide (0.30 g, 0.83 mmol) obtained in Reference Example 5 and benzyl bromide (0.20 ml) in acetone (3 ml) was stirred for 16 hours under reflux by heating. The reaction mixture was cooled to room temperature, and then the precipitates were collected by filtration to obtain 0.30 g (yield: 67%) of the title compound as a white crystalline compound.

¹H-NMR (DMSO-d₆, δ PPM): 1.24-1.55 (3H, m), 1.90-2.12 (2H, m), 2.36-2.47 (1H, m), 2.61 (3H, s), 3.58-3.72 (1H, m), 4.71-4.85 (1H, m), 5.35 (2H, brs), 6.76 (1H, brs), 6.96 (1H, brs), 7.19 (1H, brs), 7.21-7.43 (15H, m), 7.63 (1H, brs)
MS (ESI) m/z: 450 [M]⁺

### Example 7

### 3-(cis-3-(Carbamoyl(diphenyl)methyl)cyclopentyl)-1-(4-fluoro-benzyl)-2-methyl-1-3H-imidazol-1-ium bromide

By using (cis-3-(2-methylimidazol-1-yl)cyclopentyl)diphenylacetamide (0.30 g, 0.83 mmol) obtained in Reference Example 5 and 4-fluorobenzyl bromide (0.20 ml), 0.42 g (yield: 92%) of the title compound was obtained as a white crystalline compound in the same manner as that of Example 6.

¹H-NMR (DMSO-d₆, δ PPM): 1.24-1.54 (3H, m), 1.88-2.10 (2H, m), 2.36-2.48 (1H, m), 2.61 (3H, s), 3.59-3.73 (1H, m), 4.72-4.85 (1H, m), 5.33 (2H, brs), 6.75 (1H, brs), 6.96 (1H, d, J=2.1Hz),7.19 (1H, brs), 7.20-7.45 (14H, m), 7.62 (1H, brs)
MS (ESI) m/z: 468 [M]⁺

### Example 8

### 3-(cis-3-(Carbamoyl(diphenyl)methyl)cyclopentyl)-2-methyl-1-(3-methyl-benzyl)-3H-imidazol-1-ium bromide

By using (cis-3-(2-methylimidazol-1-yl)cyclopentyl)diphenylacetamide (0.30 g, 0.83 mmol) obtained in Reference Example 5 and 3-methylbenzyl bromide (0.20 ml), 0.43 g (yield: 96%) of the title compound was obtained as a white crystalline compound in the same manner as that of Example 6.

¹H-NMR (DMSO-d₆, δ PPM): 1.24-1.53 (3H, m), 1.89-2.12 (2H, m), 2.29 (3H, s), 2.35-2.46 (1H, m), 2.60 (3H, s), 3.58-3.72 (1H, m), 4.71-4.84 (1H, m), 5.29 (2H, brs), 6.75 (1H, brs), 6.96 (1H, brs), 7.06 (1H, d, J=7.6Hz),7.10-7.41 (14H, m), 7.61 (1H, brs)
MS (ESI) m/z: 464 [M]⁺

### Example 9

### 3-(cis-3-(Carbamoyl(diphenyl)methyl)cyclopentyl)-2-methyl-1-(2-methyl-benzyl)-3H-imidazol-1-ium bromide

By using (cis-3-(2-methylimidazol-1-yl)cyclopentyl)diphenylacetamide (0.30 g, 0.83 mmol) obtained in Reference Example 5 and 2-methylbenzyl bromide (0.20 ml), 0.32 g (yield: 70%) of the title compound was obtained as a white crystalline compound in the same manner as that of Example 6.

¹H-NMR (DMSO-d₆, δ PPM): 1.27-1.55 (3H, m), 1.92-2.15 (2H, m), 2.26 (3H, s), 2.39-2.52 (1H, m), 2.57 (3H, s), 3.61-3.74 (1H, m), 4.76-4.89 (1H, m), 5.34 (2H, brs), 6.77 (1H, brs), 6.79 (1H, brs), 6.99 (1H, brs), 7.12-7.43 (15H, m)
MS (ESI) m/z: 464 [M]⁺

### Example 10

### 3-(cis-3-(Carbamoyl(diphenyl)methyl)cyclopentyl)-1-(4-chloro-benzyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and 4-chlorobenzyl bromide (0.20 ml), 0.41 g (yield: 87%) of the title compound was obtained as a white crystalline compound in the same manner as that of Example 6.

¹H-NMR (DMSO-d₆, δ PPM): 1.25-1.52 (3H, m), 1.90-2.11 (2H, m), 2.36-2.46 (1H, m), 2.59 (3H, s), 3.59-3.72 (1H, m), 4.72-4.83 (1H, m), 5.34 (2H, brs), 6.75 (1H, brs), 6.97 (1H, d, J=2.1Hz),7.18 (1H, brs), 7.23-7.39 (12H, m), 7.46 (2H, d, J=8.5Hz),7.62 (1H, d, J=2.1Hz)
MS (ESI) m/z: 485 [M]⁺

### Example 11

### 3-(cis-3-(Carbamoyl(diphenyl)methyl)cyclopentyl)-2-methyl-1-(4-methyl-benzyl)-3H-imidazol-1-ium bromide

By using (cis-3-(2-methylimidazol-1-yl)cyclopentyl)diphenylacetamide (0.30 g, 0.83 mmol) obtained in Reference Example 5 and 4-methylbenzyl bromide (0.20 ml), 0.28 g (yield: 63%) of the title compound was obtained as a white crystalline compound in the same manner as that of Example 6.

¹H-NMR (DMSO-d₆, δ PPM): 1.25-1.52 (3H, m), 1.90-2.08 (2H, m), 2.29 (3H, s), 2.36-2.47 (1H, m), 2.59 (3H, s), 3.58-3.70 (1H, m), 4.70-4.81 (1H, m), 5.27 (2H, brs), 6.75 (1H, brs), 6.94 (1H, d, J=2.1Hz),7.13-7.39 (15H, m), 7.59 (1H, d, J=2.1Hz)
MS (ESI) m/z: 464 [M]⁺

### Example 12

### 3-(cis-3-(Carbamoyl(diphenyl)methyl)cyclopentyl)-1-(4-cyano-benzyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and 4-cyanobenzyl bromide (0.20 ml), 0.41 g (yield: 88%) of the title compound was obtained as a white crystalline compound in the same manner as that of Example 6.

¹H-NMR (DMSO-d₆, δ PPM): 1.25-1.54 (3H, m), 1.91-2.10 (2H, m), 2.35-2.46 (1H, m), 2.57 (3H, s), 3.58-3.73 (1H, m), 4.70-4.84 (1H, m), 5.46 (2H, brs), 6.75 (1H, brs),7.00 (1H, d, J=2.0Hz),7.19 (1H, brs), 7.24-7.39 (10H, m), 7.46 (2H, d, J=8.2Hz),7.65 (1H, brs), 7.88 (2H, d, J=8.2Hz)
MS (ESI) m/z: 475 [M]⁺

### Example 13

### 3-(cis-3-(Carbamoyl(diphenyl)methyl)cyclopentyl)-2-methyl-1-phenethyl-3H-imidazol-1-ium bromide

By using (cis-3-(2-methylimidazol-1-yl)cyclopentyl)diphenylacetamide (0.30 g, 0.83 mmol) obtained in Reference Example 5 and (2-bromoethyl)benzene (0.20 ml), 0.13 g (yield: 29%) of the title compound was obtained as a white crystalline compound in the same manner as that of Example 6.

¹H-NMR (DMSO-d₆, δ PPM): 1.18-1.54 (3H, m), 1.89-2.05 (2H, m), 2.27 (3H, s), 2.28-2.37 (1H, m), 2.99 (2H, t, J=7.1Hz),3.57-3.69 (1H, m), 4.30 (2H, t, J=7.1Hz),4.64-4.76 (1H.m),6.74 (1H, brs), 6.94 (1H, brs), 7.05-7.40 (16H, m), 7.56 (1H, brs)
MS (ESI) m/z: 464 [M]⁺

### Example 14

### 3-(cis-3-(Carbamoyl(diphenyl)methyl)cyclopentyl)-2-methyl-1-octyl-3H-imidazol-1-ium bromide

By using (cis-3-(2-methylimidazol-1-yl)cyclopentyl)diphenylacetamide (0.30 g, 0.83 mmol) obtained in Reference Example 5 and 1-bromooctane (0.20 ml), 0.27 g (yield: 59%) of the title compound was obtained as a white crystalline compound in the same manner as that of Example 6.

¹H-NMR (DMSO-d₆, δ PPM): 0.78-0.90 (3H, m), 1.15-1.52 (13H, m), 1.57-1.72 (2H, m), 1.90-2.09 (2H, m), 2.34-2.45 (1H, m), 2.57 (3H, s), 3.58-3.72 (1H, m), 3.95-4.05 (2H, m), 4.68-4.82 (1H, m), 6.74 (1H, brs), 6.92 (1H, brs), 7.18 (1H, brs), 7.22-7.38 (10H, m), 7.58 (1H, brs)
MS (ESI) m/z: 472 [M]⁺

### Example 15

### 3-(cis-3-(Carbamoyl(diphenyl)methyl)cyclopentyl)-2-methyl-1-(5-methyl-hexyl)-3H-imidazol-1-ium bromide

A solution of (cis-3-(2-methylimidazol-1-yl)cyclopentyl)diphenylacetamide (0.36 g, 1.00 mmol) obtained in Reference Example 5 and 1-bromo-5-methylhexane (1.49 g, 0.01 mol) in tetrahydrofuran (4 ml) was stirred for 16 hours under reflux by heating, and cooled to room temperature, and then the precipitates were collected by filtration to obtain 0.35 g (yield: 65%) of the title compound as a white crystalline compound.

¹H-NMR (DMSO-d₆, δ PPM): 0.84 (6H, d, J=6.7Hz),1.14-1.51 (8H, m), 1.62-1.66 (2H, m), 1.96-2.04 (2H, m), 2.39-2.42 (1H, m), 2.59 (3H, s), 3.65 (1H, m), 4.02 (2H, t, J=7.4Hz),4.74-4.76 (1H, m), 6.75 (1H, brs), 6.93 (1H, d, J=1.7Hz),7.19 (1H, brs), 7.27-7.35 (10H, m), 7.59 (1H, d, J=1.7Hz)
MS (ESI) m/z: 458 [M]⁺

### Example 16

### 3-(cis-3-(Carbamoyl(diphenyl)methyl)cyclopentyl)-2-methyl-1-pentyl-3H-imidazol-1-ium bromide

By using (cis-3-(2-methylimidazol-1-yl)cyclopentyl)diphenylacetamide (0.30 g, 0.83 mmol) obtained in Reference Example 5 and n-amyl bromide (0.20 ml), 0.15 g (yield: 36%) of the title compound was obtained as a white crystalline compound in the same manner as that of Example 6.

¹H-NMR (DMSO-d₆, δ PPM): 0.80-0.88 (3H, m), 1.15-1.52 (7H, m), 1.58-1.74 (2H, m), 1.88-2.10 (2H, m), 2.34-2.45 (1H, m), 2.57 (3H, s), 3.59-3.71 (1H, m), 4.02 (2H, t, J=7.4Hz),4.71-4.83 (1H, m), 6.75 (1H, brs), 6.92 (1H, brs), 7.18 (1H, brs), 7.22-7.38 (10H, m), 7.59 (1H, brs)
MS (ESI) m/z: 430 [M]⁺

### Example 17

### 3-(cis-3-(Carbamoyl(diphenyl)methyl)cyclopentyl)-2-methyl-1-(3-phenyl-propyl)-3H-imidazol-1-ium bromide

By using (cis-3-(2-methylimidazol-1-yl)cyclopentyl)diphenylacetamide (0.30 g, 0.83 mmol) obtained in Reference Example 5 and phenylpropyl bromide (0.20 ml), 0.21 g (yield: 44%) of the title compound was obtained as a white crystalline compound in the same manner as that of Example 6.

¹H-NMR (DMSO-d₆, δ PPM): 1.22-1.50 (3H, m), 1.89-2.09 (4H, m), 2.31-2.45 (1H, m), 2.54 (3H, s), 2.56-2.65 (2H, m), 3.58-3.70 (1H, m), 4.00-4.15 (2H, m), 4.65-4.79 (1H, m), 6.75 (1H, brs), 6.90 (1H, brs), 7.10-7.41 (16H, m), 7.60 (1H, d, J=2.0Hz)
MS (ESI) m/z: 478 [M]⁺

### Example 18

### 3-(cis-3-(Carbamoyl(diphenyl)methyl)cyclopentyl)-1-(3,3-dimethyl-2-oxo-butyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and 1-bromo-3,3-dimethyl-2-butanone (0.20 ml), 0.36 g (yield: 81%) of the title compound was obtained as a white crystalline compound in the same manner as that of Example 6.

¹H-NMR (DMSO-d₆, δ PPM): 1.19 (9H, s), 1.23-1.55 (3H, m), 1.92-2.14 (2H, m), 2.38-2.46 (4H, m), 3.60-3.72 (1H, m), 4.76-4.88 (1H, m), 5.53 (2H, brs), 6.76 (1H, brs), 6.97 (1H, brs), 7.20 (1H, brs), 7.23-7.38 (10H, m), 7.44 (1H, brs)
MS (ESI) m/z: 458 [M]⁺

### Example 19

### 3-(cis-3-(Carbamoyl(diphenyl)methyl)cyclopentyl)-1-isobutyl-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and isobutyl bromide (1.37 g, 0.01 mol), 0.02 g (yield: 5%) of the title compound was obtained as a white crystalline compound in the same manner as that of Example 15.

¹H-NMR (DMSO-d₆, δ PPM): 0.84 (6H, d, J=6.7Hz),1.28-1.48 (3H, m), 1.94-2.07 (3H, m), 2.40-2.44 (1H, m), 2.58 (3H, s), 3.63-3.68 (1H, m), 3.88 (2H, d, J=7.5Hz),3.74-3.78 (1H, m), 6.75 (1H, brs), 6.95 (1H, d, J=2.0Hz),7.18 (1H, brs), 7.27-7.35 (10H, m), 7.57 (1H, d, J=2.0Hz)
MS (ESI) m/z: 416 [M]⁺

### Example 20

### 3-(cis-3-(Carbamoyl(diphenyl)methyl)cyclopentyl)-2-methyl-1-(3-methyl-butyl)-3H-imidazol-1-ium bromide

By using (cis-3-(2-methylimidazol-1-yl)cyclopentyl)diphenylacetamide (0.36 g, 1.00 mmol) obtained in Reference Example 5 and isoamyl bromide (1.51 g, 0.01 mol), 0.28 g (yield: 55%) of the title compound was obtained as a white crystalline compound in the same manner as that of Example 15.

¹H-NMR (DMSO-d₆, δ PPM): 0.90 (6H, d, J=5.7Hz),1.28-1.57 (6H, m), 1.94-2.06 (2H, m), 2.39-2.42 (1H, m), 2.57 (3H, s), 3.63-3.68 (1H, m), 4.01-4.05 (2H, m), 4.74-4.78 (1H, m), 6.75 (1H, brs), 6.93 (1H, d, J=1.2Hz),7.19 (1H, brs), 7.28-7.35 (10H, m), 7.59 (1H, d, J=1.2Hz)
MS (ESI) m/z: 430 [M]⁺

### Example 21

### 3-(cis-3-(Carbamoyl(diphenyl)methyl)cyclopentyl)-2-methyl-1-(4-methyl-pentyl)-3H-imidazol-1-ium bromide

By using (cis-3-(2-methylimidazol-1-yl)cyclopentyl)diphenylacetamide (0.36 g, 1.00 mmol) obtained in Reference Example 5 and 1-bromo-4-methylpentane (1.65 g, 0.01 mol), 0.37 g (yield: 71%) of the title compound was obtained as a white crystalline compound in the same manner as that of Example 15.

¹H-NMR (DMSO-d₆, δ PPM): 0.84 (6H, d, J=6.7Hz),1.12-1.16 (2H, m), 1.29-1.38 (4H, m), 1.45-1.54 (2H, m), 1.64-1.68 (2H, m), 1.94-2.04 (1H, m), 2.57 (3H, s), 3.63-3.68 (1H, m), 4.00 (2H, t, J=7.5Hz),4.74-4.78 (1H, m), 6.75 (1H, brs), 6.93 (1H, d, J=1.9Hz),7.19 (1H, brs), 7.27-7.35 (10H, m), 7.59 (1H, d, J=1.9Hz)
MS (ESI) m/z: 444 [M]⁺

### Example 22

### 3-(cis-3-(Carbamoyl(diphenyl)methyl)cyclopenthyl)-2-methyl-1-(oxiranylmethyl-3H-imidazol-1-ium bromide

By using (cis-3-(2-methylimidazol-1-yl)cyclopentyl)diphenylacetamide (0.36 g, 1.00 mmol) obtained in Reference Example 5 and epibromohydrin (1.37 g, 0.01 mol), 0.19 g (yield: 38%) of the title compound was obtained as a white crystalline compound in the same manner as that of Example 15.

¹H-NMR (DMSO-d₆, δ PPM): 1.36-1.47 (3H, m), 1.95-2.05 (2H, m), 2.39-2.42 (1H, m), 2.59-2.62 (4H, m), 2.82-2.85 (1H, m), 3.32-3.34 (1H, m), 3.64-3.68 (1H, m), 4.06-4.13 (1H, m), 4.51-4.57 (1H, m), 4.76-4.80 (1H, m), 6.75 (1H, brs), 6.94-6.96 (1H, m), 7.19 (1H, brs), 7.27-7.31 (10H, m), 7.59 (1H, d, J=1.9Hz)
MS (ESI) m/z: 416 [M]⁺

### Example 23

### 3-(cis-3-(Carbamoyl(diphenyl)methyl)cyclopentyl)-1-(2-cyclohexyl-ethyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and 1-bromo-2-cyclohexylethane (0.20 ml), 0.19 g (yield: 42%) of the title compound was obtained as a white crystalline compound in the same manner as that of Example 6.

¹H-NMR (DMSO-d₆, δ PPM): 0.85-1.85 (16H, m), 1.90-2.08 (2H, m), 2.34-2.45 (1H, m), 2.57 (3H, s), 3.58-3.70 (1H, m), 4.06 (2H, t, J=7.8Hz),4.70-4.82 (1H, m), 6.75 (1H, brs), 6.92 (1H, brs), 7.19 (1H, brs), 7.23-7.39 (10H, m), 7.59 (1H, brs)
MS (ESI) m/z: 470 [M]⁺

### Example 24

### 1-Butyl-3-(cis-3-(carbamoyl(diphenyl)methyl)cyclopentyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and 1-bromobutane (0.20 ml), 0.06 g (yield: 16%) of the title compound was obtained as a white crystalline compound in the same manner as that of Example 6.

¹H-NMR (DMSO-d₆, δ PPM): 0.84-0.93 (3H, m), 1.18-1.50 (5H, m), 1.58-1.70 (2H, m), 1.90-2.10 (2H, m), 2.35-2.45 (1H, m), 2.57 (3H, s), 3.58-3.72 (1H, m), 4.03 (2H, t, J=7.4Hz),4.70-4.81 (1H, m), 6.75 (1H, brs), 6.92 (1H, d, J=1.4Hz),7.18 (1H, brs), 7.24-7.38 (10H, m), 7.58 (1H, brs)
MS (ESI) m/z: 416 [M]⁺

### Example 25

### 3-(cis-3-(Carbamoyl(diphenyl)methyl)cyclopentyl)-1-(2-ethoxy-ethyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and 2-bromoethyl ethyl ether (1.53 g, 0.01 mol), 0.28 g (yield: 55%) of the title compound was obtained as a white crystalline compound in the same manner as that of Example 15.

¹H-NMR (DMSO-d₆, δ PPM): 1.03 (3H, t, J=7.0Hz),1.28-1.49 (3H, m), 1.95-2.07 (2H, m), 2.39-2.49 (1H, m), 2.58 (3H, s), 3.37-3.43 (2H, m), 3.62-3.68 (3H, m), 4.22-4.24 (2H, m), 4.77-4.81 (1H, m), 6.75 (1H, brs), 6.94 (1H, d, J=2.1Hz),7.19 (1H, brs), 7.27-7.35 (10H, m), 7.53 (1H, d, J=2.1Hz)
MS (ESI) m/z: 432 [M]⁺

### Example 26

### 3-(cis-3-(Carbamoyl(diphenyl)methyl)cyclopentyl)-1-(2-methoxy-ethyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and 2-bromoethyl methyl ether (1.39 g, 0.01 mol), 0.25 g (yield: 51%) of the title compound was obtained as a white crystalline compound in the same manner as that of Example 15.

¹H-NMR (DMSO-d₆, δ PPM): 1.27-1.49 (3H, m), 1.94-2.07 (2H, m), 2.39-2.42 (1H, m), 2.57 (3H, s), 3.23 (3H, s), 3.45-3.67 (3H, m), 4.23-4.25 (2H, m), 4.76-4.80 (1H, m), 6.74 (1H, brs), 6.92-6.93 (1H, m), 7.18 (1H, brs), 7.27-7.35 (10H, m), 7.52-7.53 (1H, m)
MS (ESI) m/z: 418 [M]⁺

### Example 27

### 3-(cis-3-(Carbamoyl(diphenyl)methyl)cyclopentyl)-1-(4-methoxycarbonyl-benzyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and methyl 4-(bromomethyl)benzoate (0.20 ml), 0.47 g (yield: 95%) of the title compound was obtained as a pale yellow crystalline compound in the same manner as that of Example 6.

¹H-NMR (DMSO) δ: 1.26-1.54 (3H, m), 1.92-2.12 (2H, m), 2.37-2.47 (1H, m), 2.58 (3H, s), 3.59-3.72 (1H, m), 3.85 (3H, s), 4.73-4.85 (1H, m), 5.46 (2H, brs), 6.75 (1H, brs), 7.00 (1H, brs), 7.19 (1H, brs), 7.24-7.37 (10H, m), 7.41 (2H, d, J=8.1Hz),7.66 (1H, brs), 7.97 (2H, d, J=8,1Hz)
MS (ESI) m/z: 508 [M]⁺

### Example 28

### 3-(cis-3-(Carbamoyl(diphenyl)methyl)cyclopentyl)-1-but-2-enyl-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and crotyl bromide (1.35 g, 0.01 mol), 0.44 g (yield: 89%) of the title compound was obtained as a white crystalline compound in the same manner as that of Example 15.

¹H-NMR (DMSO-d₆, δ PPM): 1.32-1.49 (3H, m), 1.66-1.68 (2H, m), 1.72-1.74 (1H, m), 1.94-2.06 (2H, m), 2.39-2.42 (1H, m), 2.56 (3H, d, J=3.3Hz),3.64-3.68 (1H, m), 4.64 (1H, m), 4.74-4.79 (2H, m), 5.53-5.58 (1H, m), 5.72-5.77 (1H, m), 6.75 (1H, brs), 6.91-6.92 (1H, m), 7.19 (1H, brs), 7.25-7.34 (10H, m), 7.52 (1H, d, J=1.9Hz)
MS (ESI) m/z: 414 [M]⁺

### Example 29

### 3-(cis-3-(Carbamoyl(diphenyl)methyl)cyclopentyl)-1-(3-methoxycarbonyl-benzyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and methyl 3-(bromomethyl)benzoate (0.20 ml), 0.28 g (yield: 58%) of the title compound was obtained as a pale yellow crystalline compound in the same manner as that of Example 6.

¹H-NMR (DMSO) δ: 1.22-1.53 (3H, m), 1.88-2.12 (2H, m), 2.35-2.45 (1H, m), 2.60 (3H, s), 3.58-3.72 (1H, m), 3.86 (3H, s), 4.70-4.85 (1H, m), 5.43 (2H, brs), 6.75 (1H, brs), 6.98 (1H, brs), 7.18 (1H, brs), 7.22-7.41 (10H, m), 7.56 (2H, d, J=4.3Hz),7.65 (1H, brs), 7.95 (2H, brs)
MS (ESI) m/z: 508 [M]⁺

### Example 30

### 3-(cis-3-(Carbamoyl(diphenyl)methyl)cyclopentyl)-1-cyclopropylmethyl-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and (bromomethyl)cyclopropane (1.35 g, 0.01 mol), 0.41 g (yield: 82%) of the title compound was obtained as a white crystalline compound in the same manner as that of Example 15.

¹H-NMR (DMSO-d₆, δ PPM): 0.38-0.43 (2H, m), 0.53-0.58 (2H, m), 1.18-1.21 (1H, m), 1,30-1.48 (3H, m), 1.95-2.06 (2H, m), 2.40-2.43 (1H, m), 2.60 (3H, s), 3.64-3.68 (1H, m), 3.92 (2H, d, J=7.3Hz),4.76-4.80 (1H, m), 6.75 (1H, brs), 6.93 (1H, d, J=2.0Hz),7.19 (1H, brs), 7.27-7.35 (10H, m), 7.64 (1H, d, J=2.0Hz)
MS (ESI) m/z: 414 [M]⁺

### Example 31

### 3-(cis-3-(carbamoyl(diphenyl)methyl)cyclopentyl)-1-(2-fluoro-benzyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and 2-fluorobenzyl bromide (1.89 g, 0.01 mol), 0.47 g (yield: 86%) of the title compound was obtained as a white crystalline compound in the same manner as that of Example 15.

¹H-NMR (DMSO-d₆, δ PPM): 1.31-1.47 (3H, m), 1.94-2.07 (2H, m), 2.41-2.44 (1H, m), 2.61 (3H, s), 3.63-3.67 (1H, m), 4.76-4.80 (1H, m), 5.40 (2H, s), 6.75 (1H, brs), 6.96 (1H, d, J=2.1Hz),7.19-7.36 (14H, m), 7.42-7.47 (1H, m), 7.55 (1H, d, J=2.1Hz)
MS (ESI) m/z: 468 [M]⁺

### Example 32

### 3-(cis-3-(Carbamoyl(diphenyl)methyl)cyclopentyl)-1-(3-fluoro-benzyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and 3-fluorobenzyl bromide (1.89 g, 0.01 mol), 0.47 g (yield: 86%) of the title compound was obtained as a white crystalline compound in the same manner as that of Example 15.

¹H-NMR (DMSO-d₆, δ PPM): 1.32-1.48 (3H, m), 1.94-2.07 (2H, m), 2.41-2.44 (1H, m), 2.60 (3H, s), 3.63-3.68 (1H, m), 4.75-4.79 (1H, m), 5.35 (2H, s), 6.75 (1H, brs), 6.97 (1H, d, J=2.2Hz),7.13-7.34 (14H, m), 7.42-7.48 (1H, m), 7.63 (1H, d, J=2.2Hz)
MS (ESI) m/z: 468 [M]⁺

### Example 33

### 3-(cis-3-(Carbamoyl(diphenyl)methyl)cyclopentyl)-2-methyl-1-pentafluorophenylmethyl-3H-imidazol-1-ium bromide

By using (cis-3-(2-methylimidazol-1-yl)cyclopentyl)diphenylacetamide (0.36 g, 1.0 mmol) obtained in Reference Example 5 and 2,3,4,5,6-pentafluorobenzyl bromide (2.61 g, 0.01 mol), 0.39 g (yield: 62%) of the title compound was obtained as a white crystalline compound in the same manner as that of Example 15.

¹H-NMR (DMSO-d₆, δ PPM): 1.31-1.47 (3H, m), 1.96-2.04 (2H, m), 2.37-2.40 (1H, m), 2.61 (3H, s), 3.62-3.68 (1H, m), 4.77-4.81 (1H, m), 5.51 (2H, s), 6.73 (1H, brs), 6.98 (1H, d, J=2.0Hz),7.19 (1H, brs), 7.25-7.34 (10H, m), 7.56 (1H, d, J=2.0Hz)
MS (ESI) m/z: 540 [M]⁺

### Example 34

### 3-(cis-3-(Carbamoyl(diphenyl)methyl)cyclopentyl)-1-(5-ethoxycarbonyl-furan-2-ylmethyl)-2-methyl-3H-imidazol-1-ium bromide

By using (cis-3-(2-methylimidazol-1-yl)cyclopentyl)diphenylacetamide (0.30 g, 0.83 mmol) obtained in Reference Example 5 and 5-bromomethyl-furan-2-carboxylic acid ethyl ester (0.20 ml), 0.17 g (yield: 34%) of the title compound was obtained as a white amorphous compound in the same manner as that of Example 6.

¹H-NMR (DMSO) δ: 1.21-1.52 (6H, m), 1.89-2.10 (2H, m), 2.35-2.49 (1H, m), 2.65 (3H, s), 3.58-3.74 (1H, m), 4.20-4.33 (2H, m), 4.72-4.85 (1H, m), 5.53 (2H, brs), 6.78 (2H, brs), 6.96 (1H, brs), 7.19 (1H, brs), 7.22-7.44 (11H, m), 7.63 (1H, brs)
MS (ESI) m/z: 512 [M]⁺

### Example 35

### 3-(cis-3-(Carbamoyl(diphenyl)methyl)cyclopentyl)-2-methyl-1-(4,4,4-trifluoro-butyl)-3H-imidazol-1-ium iodide

By using (cis-3-(2-methylimidazol-1-yl)cyclopentyl)diphenylacetamide (0.36 g, 1.00 mmol) obtained in Reference Example 5 and 1-iodo-4,4,4-trifluorobutane (2.38 g, 0.01 mol), 0.28 g (yield: 47%) of the title compound was obtained as a yellow crystalline compound in the same manner as that of Example 15.

¹H-NMR (DMSO-d₆, δ PPM): 1.32-1.48 (3H, m), 1.87-2.06 (4H, m), 2.29-2.41 (3H, m), 2.57 (3H, s), 3.63-3.68 (1H, m), 4.09-4.12 (2H, m), 4.73-4.77 (1H, m), 6.74 (1H, brs), 6.94 (1H, d, J=2.2Hz),7.19 (1H, brs), 7.27-7.35 (10H, m), 7.60 (1H, d, J=2.2Hz)
MS (ESI) m/z: 470 [M]⁺

### Example 36

### 3-(cis-3-(Carbamoyl(diphenyl)methyl)cyclopentyl)-2-methyl-1-(2-(S)-oxiranyl-ethyl)-3H-imidazol-1-ium bromide

By using (cis-3-(2-methylimidazol-1-yl)cyclopentyl)diphenylacetamide (0.36 g, 1.00 mmol) obtained in Reference Example 5 and (S)-(-)-(2-bromoethyl)oxirane (1.51 g, 0.01 mol), 0.24 g (yield: 47%) of the title compound was obtained as a white crystalline compound in the same manner as that of Example 15.

¹H-NMR (DMSO-d₆, δ PPM): 1.43-1.48 (3H, m), 1.74-1.80 (2H, m), 1.94-2.06 (2H, m), 2.38-2.43 (1H, m), 2.59 (3H, s), 2.65-2.67 (1H, m), 2.93-2.96 (1H, m), 3.59-3.68 (2H, m), 4.18-4.22 (2H, m), 4.75-4.80 (1H, m), 6.75 (1H, brs), 6.94 (1H, d, J=2.0Hz),7.19 (1H, brs), 7.27-7.35 (10H, m), 7.59 (1H, d, J=2.Hz)
MS (ESI) m/z: 430 [M]⁺

### Example 37

### 3-(cis-3-(Carbamoyl(diphenyl)methyl)cyclopentyl)-1-difluoromethyl-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and ethyl bromodifluoroacetate (2.03 g, 0.01 mol), 0.07 g (yield: 13%) of the title compound was obtained as a white crystalline compound in the same manner as that of Example 15.

¹H-NMR (DMSO-d₆, δ PPM): 1.37-1.49 (3H, m), 1.96-2.08 (2H, m), 2.40-2.44 (1H, m), 2.75 (3H, s), 3.63-3.68 (1H, m), 4.83-4.87 (1H, m), 6.73 (1H, brs), 7.17-7.35 (12H, m), 8.03 (1H, t, J=57Hz),8.04-8.05 (1H, m)
MS (ESI) m/z: 410 [M]⁺

### Example 38

### 3-(cis-3-(Carbamoyl(diphenyl)methyl)cyclopentyl)-1-cyclohexylmethyl-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and bromomethylcyclohexane (1.77 g, 0.01 mol), 0.33 g (yield: 61%) of the title compound was obtained as a white crystalline compound in the same manner as that of Example 15.

¹H-NMR (DMSO-d₆, δ PPM): 0.92-0.98 (2H, m), 1.11-1.20 (3H, m), 1.30-1.35 (2H, m), 1.43-1.49 (3H, m), 1.62-1.68 (4H, m), 1.95-2.05 (2H, m), 2.40-2.43 (1H, m), 2.57 (3H, s), 3.63-3.68 (1H, m), 3.89-3.91 (2H, m), 4.73-4.77 (1H, m), 6.80 (1H, brs), 6.92-6.93 (1H, m), 7.21 (1H, brs), 7.27-7.35 (10H, m), 7.54-7.55 (1H, m)
MS (ESI) m/z: 456 [M]⁺

### Example 39

### 3-(cis-3-(Carbamoyl(diphenyl)methyl)cyclopentyl)-2-methyl-1-(3,3,3-trifluoro-pronyl)-3H-imidazol-1-ium iodide

By using (cis-3-(2-methylimidazol-1-yl)cyclopentyl)diphenylacetamide (0.36 g, 1.00 mmol) obtained in Reference Example 5 and 1,1,1-trifluoro-3-iodopropane (2.24 g, 0.01 mol), 0.31 g (yield: 53%) of the title compound was obtained as a white crystalline compound in the same manner as that of Example 15.

¹H-NMR (DMSO-d₆, δ PPM): 1.28-1.35 (2H, m), 1.44-1.47 (1H, m), 1.94-2.06 (2H, m), 2.37-2.50 (1H, m), 2.61 (3H, s), 2.84-2.93 (2H, m), 3.62-3.68 (1H, m), 4.33-4.37 (2H, m), 4.77-4.81 (1H, m), 6.79 (1H, brs), 6.98 (1H, d, J=2.1Hz),7.21 (1H, brs), 7.27-7.35 (10H, m), 7.62 (1H, d, J=2.1Hz)
MS (ESI) m/z: 456 [M]⁺

### Example 40

### 3-(cis-3-(Carbamoyl(diphenyl)methyl)cyclopentyl)-1-cyclobutylmethyl-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and (bromomethyl)cyclobutane (1.49 g, 0.01 mol), 0.10 g (yield: 19%) of the title compound was obtained as a white crystalline compound in the same manner as that of Example 15.

¹H-NMR (DMSO-d₆, δ PPM): 1.27-1.35 (2H, m), 1.43-1.46 (1H, m), 1.71-1.77 (2H, m), 1.80-1.87 (2H, m), 1.90-2.05 (4H, m), 2.38-2.41 (1H, m), 2.57 (3H, s), 2.64-2.68 (1H, m), 3.60-3.68 (1H, m), 4.08 (1H, d, J=7.6Hz),4.73-4.77 (1H, m), 6.80 (1H, brs), 6.92 (1H, d, J=2.0Hz),7.21 (1H, brs), 7.26-7.30 (2H, m), 7.33-7.35 (8H, m), 7.55 (1H, d, J=2.0Hz) MS (ESI) m/z: 428 [M]⁺

### Example 41

### 3-(cis-3-(Carbamoyl(diphenyl)methyl)cyclopentyl)-1-(3-ethoxycarbonyl-allyl)-2-methyl-3H-imidazol-1-ium bromide

By using (cis-3-(2-methylimidazol-1-yl)cyclopentyl)diphenylacetamide (0.72 g, 2.00 mmol) obtained in Reference Example 5 and ethyl 4-bromocrotonate (3.86 g, 0.02 mol), 0.81 g (yield: 73%) of the title compound was obtained as a white crystalline compound in the same manner as that of Example 15.

¹H-NMR (DMSO-d₆, δ PPM): 1.18-1.22 (3H, m), 1.32-1.45 (3H, m), 1.96-2.05 (2H, m), 2.40-2.44 (1H, m), 2.53 (3H, s), 3.64-3.68 (1H, m), 4.10-4.15 (2H, m), 4.74-4.78 (1H, m), 4.95-4.96 (2H, m), 5.82-5.85 (1H, m), 6.80 (1H, brs), 6.86-6.93 (1H, m), 6.95-6.96 (1H, m), 7.21 (1H, brs), 7.27-7.35 (10H, m), 7.55 (1H, brs)
MS (ESI) m/z: 472 [M]⁺

### Example 42

### 3-(cis-3-(Carbamoyl(diphenyl)methyl)cyclopentyl)-1-(3-methoxy-propyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and 1-bromo-3-methoxypropane (1.53 g, 0.01 mol), 0.43 g (yield: 84%) of the title compound was obtained as a white amorphous compound in the same manner as that of Example 15.

¹H-NMR (DMSO-d₆, δ PPM): 1.26-1.47 (3H, m), 1.89-2.05 (4H, m), 2.38-2.41 (1H, m), 2.56 (3H, s), 3.19 (3H, s), 3.21-3.30 (2H, m), 3.64-3.68 (1H, m), 4.06-4.10 (2H, m), 4.74-4.79 (1H, m), 6.80 (1H, brs), 6.92-6.93 (1H, m), 7.21 (1H, brs), 7.27-7.35 (10H, m), 7.56-7.57 (1H, m)
MS (ESI) m/z: 432 [M]⁺

### Example 43

### 3-(cis-3-(Carbamoyl(diphenyl)methyl)cyclopentyl)-2-methyl-1-(3-phenyl-allyl)-3H-imidazol-1-ium bromide

By using (cis-3-(2-methylimidazol-1-yl)cyclopentyl)diphenylacetamide (0.36 g, 1.00 mmol) obtained in Reference Example 5 and cinnamyl bromide (1.97 g, 0.01 mol), 0.50 g (yield: 89%) of the title compound was obtained as a white crystalline compound in the same manner as that of Example 15.

¹H-NMR (DMSO-d₆, δ PPM): 1.26-1.46 (3H, m), 1.96-2.05 (2H, m), 2.40-2.44 (1H, m), 2.62 (3H, s), 3.64-3.69 (1H, m), 4.77-4.81 (1H, m), 4.88-4.90 (2H, m), 6.34-6.41 (1H, m), 6.63-6.67 (1H, m), 6.81 (1H, brs), 6.94-6.95 (1H, m), 7.22 (1H, brs), 7.27-7.37 (13H, m), 7.44-7.46 (2H, m), 7.60 (1H, m)
MS (ESI) m/z: 476 [M]⁺

### Example 44

### 3-(cis-3-(Carbamoyl(diphenyl)methyl)cyclopentyl)-2-methyl-1-oct-2-ynyl-3H-imidazol-1-ium bromide

By using (cis-3-(2-methylimidazol-1-yl)cyclopentyl)diphenylacetamide (0.36 g, 1.00 mmol) obtained in Reference Example 5 and 1-bromo-2-octyne (1.89 g, 0.01 mol), 0.43 g (yield: 79%) of the title compound was obtained as a white crystalline compound in the same manner as that of Example 15.

¹H-NMR (DMSO-d₆, δ PPM): 0.81-0.86 (3H, m), 1.28-1.37 (6H, m), 1.39-1.48 (3H, m), 1.94-2.06 (2H, m), 2.21-2.25 (2H, m), 2.39-2.43 (1H, m), 2.60 (3H, s), 3.64-3.68 (1H, m), 4.77-4.81 (1H, m), 5.02 (2H, s), 6.80 (1H, brs), 6.95 (1H, d, J=2.0Hz),7.22 (1H, brs), 7.25-7.37 (10H, m), 7.60 (1H, d, J=2.0Hz)
MS (ESI) m/z: 468 [M]⁺

### Example 45

### 3-(cis-3-(Carbamoyl(diphenyl)methyl)cyclopentyl)-2-methyl-1-pent-2-enyl-3H-imidazol-1-ium bromide

By using (cis-3-(2-methylimidazol-1-yl)cyclopentyl)diphenylacetamide (0.36 g, 1.00 mmol) obtained in Reference Example 5 and 1-bromo-2-pentene (1.49 g, 0.01 mol), 0.44 g (yield: 87%) of the title compound was obtained as a white crystalline compound in the same manner as that of Example 15.

¹H-NMR (DMSO-d₆, δ PPM): 0.94 (3H, t, J=7.5Hz),1.27-1.47 (3H, m), 1.94-2.05 (4H, m), 2.39-2.42 (1H, m), 2.56 (3H, s), 3.64-3.68 (1H, m), 4.65 (2H, d, J=6.2Hz),4.74-4.79 (1H, m), 5.50-5.54 (1H, m), 5.75-5.81 (1H, m), 6.81 (1H, brs), 6.91 (1H, d, J=2.1Hz),7.21 (1H, brs), 7.27-7.50 (10H, m), 7.51 (1H, d, J=2.1Hz)
MS (ESI) m/z: 428 [M]⁺

### Example 46

### 3-(cis-3-(Carbamoyl(diphenyl)methyl)cyclopentyl)-2-methyl-1-(3-methyl-but-2-enyl)-3H-imidazol-1-ium bromide

By using (cis-3-(2-methylimidazol-1-yl)cyclopentyl)diphenylacetamide (0.36 g, 1.00 mmol) obtained in Reference Example 5 and 1-bromo-3-methyl-2-butene (1.49 g, 0.01 mol), 0.35 g (yield: 69%) of the title compound was obtained as a white crystalline compound in the same manner as that of Example 15.

¹H-NMR (DMSO-d₆, δ PPM): 1.27-1.46 (3H, m), 1.73 (6H, s), 1.95-2.03 (2H, m), 2.39-2.42 (1H, m), 2.56 (3H, s), 3.64-3.68 (1H, m), 4.66-4.67 (2H, m), 4.75-4.79 (1H, m), 5.23-5.26 (1H, m), 6.82 (1H, brs), 6.89 (1H, brs), 7.22 (1H, brs), 7.27-7.35 (10H, m), 7.48-7.49 (1H, m)
MS (ESI) m/z: 428 [M]⁺

### Example 47

### 3-(cis-3-(Carbamoyl(diphenyl)methyl)cyclopentyl)-2-methyl-1-thiophen-3-ylmethyl-3H-imidazol-1-ium bromide

By using (cis-3-(2-methylimidazol-1-yl)cyclopentyl)diphenylacetamide (0.36 g, 1.00 mmol) obtained in Reference Example 5 and 3-bromomethylthiophene (1.77 g, 0.01 mol), 0.36 g (yield: 67%) of the title compound was obtained as a white crystalline compound in the same manner as that of Example 15.

¹H-NMR (DMSO-d₆, δ PPM): 1.26-1.46 (3H, m), 1.93-2.06 (2H, m), 2.40-2.45 (1H, m), 2.62 (3H, s), 3.63-3.68 (1H, m), 4.74-4.79 (1H, m), 5.31 (2H, s), 6.81 (1H, brs), 6.94 (1H, d, J=2.1Hz),7.10-7.11 (1H, m), 7.21 (1H, brs), 7.26-7.36 (10H, m), 7.59 (1H, brs), 7.59-7.62 (2H, m)
MS (ESI) m/z: 456 [M]⁺

### Example 48

### 3-(cis-3-(Carbamoyl(diphenyl)methyl)cyclopentyl)-1-(3-cyclohexyl-allyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and (3-bromopropenyl)cyclohexane (2.03 g, 0.01 mol), 0.56 g (yield: 99%) of the title compound was obtained as a white crystalline compound in the same manner as that of Example 15.

¹H-NMR (DMSO-d₆, δ PPM): 1.19-1.47 (9H, m), 1.59-1.68 (5H, m), 1.94-2.05 (2H, m), 2.37-2.42 (1H, m), 2.54 (3H, s), 3.64-3.68 (1H, m), 4.64-4.65 (2H, m), 4.75-4.79 (1H, m), 5.46-5.51 (1H, m), 5.68-5.74 (1H, m), 6.80 (1H, brs), 6.91 (1H, brs), 7.21 (1H, brs), 7.27-7.35 (10H, m), 7.49-7.50 (1H, m)
MS (ESI) m/z: 482 [M]⁺

### Example 49

### 1-(5-Acetoxy-pentyl)-3-(cis-3-(carbamoyl(diphenyl)methyl)cyclopentyl)-2-methyl-3H-imidazol-1-ium bromide

A solution of (cis-3-(2-methylimidazol-1-yl)cyclopentyl)diphenylacetamide (0.18 g, 0.50 mmol) obtained in Reference Example 5 and 5-bromopentyl acetate (0.12 g, 0.55 mmol) in 1,4-dioxane (10 ml) was stirred for 18 hours under reflux by heating. The reaction mixture was cooled to room temperature, and then the precipitates were collected by filtration to obtain 0.05 g (yield: 16%) of the title compound as a white crystalline compound.

¹H-NMR (DMSO-d₆, δ PPM): 1.23-1.32 (3H, m), 1.35-1.38 (1H, m), 1.41-1.48 (1H, m), 1.54-1.61 (2H, m), 1.65-1.72 (2H, m), 1.94-2.06 (2H, m), 1.97 (3H, s), 2.37-2.43 (1H, m), 2.57 (3H, s), 3.63-3.67 (1H, m), 3.96-3.99 (2H, m), 4.01-4.05 (2H, m), 4.74-4.78 (1H, m), 6.74 (1H, brs), 6.93 (1H, d, J=1.9Hz),7.18 (1H, brs), 7.28-7.35 (10H, m), 7.57 (1H, d, J=2.0Hz)
MS (ESI) m/z: 488 [M]⁺

### Example 50

### 3-(cis-3-(Carbamoyl(diphenyl)methyl)cyclopentyl)-1-(3-methoxycarbonyl-allyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and methyl 4-bromocrotonate (1.79 g, 0.01 mol), 0.07 g (yield: 13%) of the title compound was obtained as a pale brown crystalline compound in the same manner as that of Example 15.

¹H-NMR (DMSO-d₆, δ PPM): 1.33-1.48 (3H, m), 1.94-2.07 (2H, m), 2.40-2.43 (1H, m), 2.60 (3H, s), 3.63-3.66 (4H, m), 4.74-4.78 (1H, m), 4.96 (2H, d, J=4.3Hz),5.86 (1H, d, J=15.7Hz),6.75 (1H, brs), 6.87-6.93 (1H, m), 6.96 (1H, d, J=2.0Hz),7.19 (1H, brs), 7.27-7.35 (10H, m), 7.55 (1H, d, J=2.0Hz)
MS (ESI) m/z: 458 [M]⁺

### Example 51

### 3-(cis-3-(Carbamoyl(diphenyl)methyl)cyclopentyl)-1-(2-acetoxy-ethyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and 2-bromoethyl acetate (1.67 g, 0.01 mol), 0.08 g (yield: 14%) of the title compound was obtained as a white crystalline compound in the same manner as that of Example 15.

¹H-NMR (DMSO-d₆, δ PPM): 1.28-1.49 (3H, m), 1.95-2.07 (5H, m), 2.38-2.42 (1H, m), 2.59 (3H, s), 3.63-3.68 (1H, m), 4.27 (2H, t, J=4.7Hz),4.35 (2H, t, J=4.7Hz),4.76-4.80 (1H, m), 6.74 (1H, brs), 6.94 (1H, d, J=2.0Hz),7.19 (1H, brs), 7.27-7.35 (10H, m), 7.58 (1H, d, J=2.0Hz)
MS (ESI) m/z: 446 [M]⁺

### Example 52

### 1-(3-Acetoxy-propyl)-3-(cis-3-(carbamoyl(diphenyl)methyl)cyclopentyl)-2-methyl-3H-imidazol-1-ium bromide

A solution of (cis-3-(2-methylimidazol-1-yl)cyclopentyl)diphenylacetamide (0.18 g, 0.50 mmol) obtained in Reference Example 5 and 3-bromopropyl acetate (0.27 g, 1.50 mmol) in 1,4-dioxane (10 ml) was stirred for 30 hours under reflux by heating. The reaction mixture was cooled to room temperature, and then the precipitates were collected by filtration to obtain 0.24 g (yield: 87%) of the title compound as a pale yellow powdery compound.

¹H-NMR (DMSO-d₆, δ PPM): 1.28-1.49 (3H, m), 1.95 (3H, s), 1.98-2.06 (4H, m), 2.36-2.43 (1H, m), 2.57 (3H, s), 3.63-3.67 (1H, m), 3.98-4.02 (2H, m), 4.10-4.14 (2H, m), 4.74-4.78 (1H, m), 6.74 (1H, brs), 6.94 (1H, d, J=1.9 Hz),7.19 (1H, brs), 7.27-7.35 (10H, m), 7.56 (1H, d, J=2.0Hz)
MS (ESI) m/z: 460 [M]⁺

### Example 53

### 3-(cis-3-(Carbamoyl(diphenyl)methyl)cyclopentyl)-1-(3-methoxy-benzyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5, (2.01 g, 0.01 mol), 0.25 g (yield: 44%) of the title compound was obtained as a white crystalline compound in the same manner as that of Example 15.

¹H-NMR (DMSO-d₆, δ PPM): 1.33-1.49 (3H, m), 1.94-2.07 (2H, m), 2.38-2.45 (1H, m), 2.60 (3H, s), 3.59-3.67 (1H, m), 3.74 (3H, s), 4.75-4.79 (1H, m), 5.29 (2H, s), 6.74 (1H, brs), 6.81-6.83 (1H, m), 6.89-6.96 (3H, m), 7.18 (1H, brs), 7.25-7.33 (11H, m), 7.63 (1H, brs)
MS (ESI) m/z: 480 [M]⁺

### Example 54

### 3-(cis-3-(Carbamoyl(diphenyl)methyl)cyclopentyl)-1-(3-hydroxy-propyl)-2-methyl-3H-imidazol-1-ium bromide

By using (cis-3-(2-methylimidazol-1-yl)cyclopentyl)diphenylacetamide (0.15 g, 0.42 mmol) obtained in Reference Example 13 and 3-bromopropanol (0.23 g, 1.67 mmol), 0.14 g (yield: 65%) of the title compound was obtained as a white crystalline compound in the same manner as that of Example 6.

¹H-NMR (DMSO-d₆, δ PPM): 1.28-1.48 (3H, m), 1.79-1.85 (2H, m), 1.94-2.08 (2H, m), 2.36-2.43 (1H, m), 2.57 (3H, s), 3.35-3.39 (2H, m), 3.63-3.70 (1H, m), 4.07-4.11 (2H, m), 4.65-4.67 (1H, m), 4.72-4.78 (1H, m), 6.74 (1H, brs), 6.92 (1H, d, J=1.9Hz),7.18 (1H, brs), 7.27-7.35 (10H, m), 7.55 (1H, d, J=2.0Hz)
MS (ESI) m/z: 418 [M]⁺

### Example 55

### 3-(cis-3-(Carbamoyl(diphenyl)methyl)cyclopentyl)-1-(2-hydroxy-ethyl)-2-methyl-3H-imidazol-1-ium bromide

By using (cis-3-(2-methylimidazol-1-yl)cyclopentyl)diphenylacetamide (0.36 g, 1.00 mmol) obtained in Reference Example 5 and 2-bromoethanol (1.25 g, 0.01 mol), 0.17 g (yield: 36%) of the title compound was obtained as in the same manner as that of Example 15.

¹H-NMR (DMSO-d₆, δ PPM): 1.28-1.50 (3H, m), 1.95-2.06 (2H, m), 2.38-2.43 (1H, m), 2.57 (3H, s), 3.65-3.67 (3H, m), 4.10-4.12 (2H, m), 4.76-4.80 (1H, m), 5.01-5.04 (1H, m), 6.74 (1H, brs), 6.91 (1H, brs), 7.19 (1H, brs), 7.28-7.35 (10H, m), 7.53 (1H, brs)
MS (ESI) m/z: 404 [M]⁺

### Example 56

### 3-(cis-3-(Carbamoyl(diphenyl)methyl)cyclopentyl)-1,2-dimethyl-4,5-dihydro-3H-imidazol-1-ium iodide

By using (cis-3-(2-methyl-4,5-dihydroimidazol-1-yl)cyclopentyl)diphenylacetamide (2.10 g, 5.82 mmol) obtained in Reference Example 13, 0.77 g (yield: 26%) of the title compound was obtained as a pale yellow amorphous compound in the same manner as that of Example 1.

¹H-NMR (CDCl₃, δ PPM): 1.61-1.87 (3H, m), 1.96-2.15 (3H, m), 2.37 (3H, s), 3.15 (3H, s), 3.40-3.57 (2H, m), 3.62-3.83 (2H, m), 3.89-4.04 (1H, m), 4.20-4.34 (1H, m), 5.54 (1H, brs), 5.61 (1H, brs), 7.20-7.38 (10H, m)
MS (ESI) m/z: 376 [M]⁺

### Example 57

### 3-((1R,3S)-3-(Carbamoyl(diphenyl)methyl)cyclopentyl)-1,2-dimethyl-4,5-dihydro-3H-imidazol-1-ium iodide

By using 2-((1S,3R)-3-(2-methyl-4,5-dihydroimidazol-1-yl)cyclopentyl)-2,2-diphenylacetamide (0.25 g, 0.69 mmol) obtained in Reference Example 26 and methyl iodide (0.20 g, 1.4 mmol), 0.15 g (yield: 43%) of the title compound was obtained as a pale yellow amorphous compound.

¹H-NMR (CDCl₃, δ PPM): 1.58-1.86 (2H, m), 1.95-2.15 (4H, m), 2.37 (3H, s), 3.15 (3H, s), 3.41-3.58 (2H, m), 3.62-3.84 (2H, m), 3.86-3.98 (1H, m), 4.24-4.34 (1H, m), 5.57 (1H, brs), 5.67 (1H, brs), 7.21-7.38 (10H, m)
MS (ESI) m/z: 376 [M]⁺

### Example 58

### 3-((1S,3R)-3-(Carbamoyl(diphenyl)methyl)cyclopentyl)-1,2-dimethyl-4,5-dihydro-3H-imidazol-1-ium iodide

By using 2-((1R,3S)-3-(2-methyl-4,5-dihydroimidazol-1-yl)cyclopentyl)-2,2-diphenylacetamide (0.12 g, 0.33 mmol) obtained in Reference Example 27 and methyl iodide (0.094 g, 0.66 mmol), 0.09 g (yield: 56%) of the title compound was obtained as a pale yellow amorphous compound.

¹H-NMR (CDCl₃, δ PPM): 1.58-1.86 (2H, m), 1.95-2.15 (4H, m), 2.37 (3H, s), 3.15 (3H, s), 3.41-3.58 (2H, m), 3.62-3.84 (2H, m), 3.86-3.98 (1H, m), 4.24-4.34 (1H, m), 5.57 (1H, brs), 5.67 (1H, brs), 7.21-7.38 (10H, m)
MS (ESI) m/z: 376 [M]⁺

### Example 59

### 3-(trans-3-(Carbamoyl(diphenyl)methyl)cyclopentyl)-1-methyl-3H-imidazol-1-ium iodide

By using 2-(trans-3-(imidazol-1-yl)cyclopentyl)-2,2-diphenylacetamide (0.3 g, 0.87 mmol) obtained in Reference Example 34, methyl iodide (0.8 ml) and acetone (4 ml), 0.20 g (yield: 46%) of the title compound was obtained as a white powdery compound in the same manner as that of Example 6.

1H-NMR (DMSO-d₆, δ PPM): 1.23-1.40 (1H, m), 1.64-2.20 (5H, m), 3.70-3.90 (4H, m), 4.06-4.20 (1H, m), 6.77 (1H, brs), 7.08-7.40 (11H, m), 7.72 (1H, brs), 7.83 (1H, brs), 9.15 (1H, s)
MS (ESI) m/z: 360 [M]⁺

### Example 60

### 3-((1R,3S)-3-(Carbamoyl(diphenyl)methyl)cyclopentyl)-1.2-dimethyl-4.5-dihydro-3H-imidazol-1-ium para-toluenesulfonate

By using 2-((1S,3R)-3-(2-methyl-4,5-dihydroimidazol-1-yl)cyclopentyl)-2,2-diphenylacetamide (0.36 g, 1.0 mmol) obtained in Reference Example 26 and methyl para-toluenesulfonate (0.2 g, 1.1 mmol), 0.14 g (yield: 26%) of the title compound was obtained as a white crystalline compound in the same manner as that of Example 6.

¹H-NMR (DMSO-d₆, δ PPM): 0.88-1.01 (1H, m), 1.23-1.38 (2H, m), 1.58-1.70 (1H, m), 1.78-1.93 (1H, m), 1.99-2.12 (1H, m), 2.20 (3H, s), 2.29 (3H, s), 2.97 (3H, s), 3.08-3.39 (2H, m), 3.50-3.68 (3H, m), 4.19-4.30 (1H, m), 6.75 (1H, brs), 7.05-7.16 (3H, m), 7.22-7.40 (10H, m), 7.47 (2H, d, J=7.9Hz)
MS (ESI) m/z: 376 [M]⁺

### Example 61

### 3-(cis-3-(Carbamoyl(diphenyl)methyl)cyclopentyl)-2-methyl-1-(2-oxopropyl)-3H-imidazol-1-ium bromide

By using 2-(cis-3-(2-methylimidazol-1-yl)cyclopentyl)-2,2-diphenylacetamide (1.0 g, 2.8 mmol) obtained in Reference Example 5, bromoacetone (1 ml) and tetrahydrofuran (15 ml), 0.36 g (yield: 26%) of the title compound was obtained as a white powdery compound in the same manner as that of Example 6.

¹H-NMR (DMSO-d₆, δ PPM): 1.08-1.56 (3H, m), 1.87-2.14 (2H, m), 2.23 (3H, s), 2.34-2.52 (4H, m), 3.57-3.74 (1H, m), 4.71-4.86 (1H, m), 5.27 (2H, s), 6.79 (1H, brs), 6.94 (1H, brs), 7.12-7.44 (12H, m)
MS (ESI) m/z: 416 [M]⁺

### Example 62

### 1-Benzyl-3-(trans-3-(carbamoyl(diphenyl)methyl)cyclopentyl)-2-methyl-3H-imidazol-1-ium bromide

By using 2-(trans-3-(2-methylimidazol-1-yl)cyclopentyl)-2,2-diphenylacetamide (1.0 g, 2.8 mmol) obtained in Reference Example 30, benzyl bromide (1 ml) and tetrahydrofuran (10 ml), 0.88 g (yield: 59%) of the title compound was obtained as a white powdery compound in the same manner as that of Example 6.

¹H-NMR (DMSO-d₆, δ PPM): 1.20-1.39 (1H, m), 1.70-1.90 (3H, m), 1.92-2.10 (2H, m), 2.44 (3H, s), 3.82-4.10 (2H, m), 5.38 (2H, s), 6.86 (1H, brs), 7.10-7.43 (16H, m), 7.82 (1H, brs), 8.00 (1H, brs)
MS (ESI) m/z: 450 [M]⁺

### Example 63

### 3-((1R,3S)-3-(Carbamoyl(diphenyl)methyl)cyclopentyl)-1-(2-methoxyethyl)-2-methyl-3H-imidazol-1-ium bromide

By using 2-((1S,3R)-3-(2-methylimidazol-1-yl)cyclopentyl)-2,2-diphenylacetamide (1 g, 2.78 mmol) obtained in Reference Example 22 and 2-bromoethyl methyl ether (3.86 g, 27.8 mmol), 1.39 g (yield: 99%) of the title compound was obtained as a white amorphous compound.

¹H-NMR (DMSO-d₆, δ PPM): 1.27-1.49 (3H, m), 1.94-2.07 (2H, m), 2.39-2.42 (1H, m), 2.57 (3H, s), 3.23 (3H, s), 3.45-3.67 (3H, m), 4.23-4.25 (2H, m), 4.76-4.80 (1H, m), 6.74 (1H, brs), 6.92-6.93 (1H, m), 7.18 (1H, brs), 7.27-7.35 (10H, m), 7.52-7.53 (1H, m)
MS (ESI) m/z: 418 [M]⁺

### Example 64

### 3-((1R,3S)-3-(Carbamoyl(diphenyl)methyl)cyclopentyl)-1-cyclopropylmethyl-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and (bromomethyl)cyclopropane (1.35 g, 0.01 mol), 0.87 g (yield: 75%) of the title compound was obtained as a white crystalline compound in the same manner as that of Example 6.

¹H-NMR (DMSO-d₆, δ PPM): 0.38-0.43 (2H, m), 0.53-0.58 (2H, m), 1.20-1.50 (4H, m), 1.95-2.07 (2H, m), 2.38-2.45 (1H, m), 2.59 (3H, s), 3.64-3.68 (1H, m), 3.91-3.93 (2H, m), 4.76-4.80 (1H, m), 6.75 (1H, brs), 6.93 (1H, brs), 7.18 (1H, brs), 7.27-7.35 (10H, m), 7.64 (1H, brs)
MS (ESI) m/z: 414 [M]⁺

### Example 65

### 3-((1S,3R)-3-(Carbamoyl(diphenyl)methyl)cyclopentyl)-1-cyclopropylmethyl-2-methyl-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and (bromomethyl)cyclopropane (1.35 g, 0.01 mol), 0.26 g (yield: 53%) of the title compound was obtained as white amorphous solid.

¹H-NMR (DMSO-d₆, δ PPM): 0.38-0.43 (2H, m), 0.53-0.58 (2H, m), 1.20-1.50 (4H, m), 1.95-2.07 (2H, m), 2.38-2.45 (1H, m), 2.59 (3H, s), 3.64-3.68 (1H, m), 3.91-3.93 (2H, m), 4.76-4.80 (1H, m), 6.75 (1H, brs), 6.93 (1H, brs), 7.18 (1H, brs), 7.27-7.35 (10H, m), 7.64 (1H, brs)
MS (ESI) m/z: 414 [M]⁺

### Example 66

### 3-(trans-3-(Carbamoyl(diphenyl)methyl)cyclopentyl)-1,2-dimethyl-3H-imidazol-1-ium iodide

By using 2-(trans-3-(2-methylimidazol-1-yl)cyclopentyl)-2,2-diphenylacetamide (0.69 g, 1.9 mmol) obtained in Reference Example 30, methyl iodide (1 ml) and acetone (7 ml), 0.90 g (yield: 94%) of the title compound was obtained as a yellow amorphous compound.

¹H-NMR (DMSO-d₆, δ PPM): 1.08-1.38 (1H, m), 1.64-1.88 (3H, m), 1.90-2.10 (2H, m), 2.37 (3H, s), 3.70 (3H, s), 3.76-4.08 (2H, m), 6.85 (1H, brs), 7.18 (1H, brs), 7.21-7.44 (10H, m), 7.68 (1H, brs), 7.88 (1H, brs)
MS (ESI) m/z: 374 [M]⁺

### Example 67

### 3-(trans-3-(Carbamoyl(diphenyl)methyl)cyclopentyl)-1-(2-methoxyethyl)-2-methyl-3H-imidazol-1-ium bromide

By using 2-(trans-3-(2-methylimidazol-1-yl)cyclopentyl)-2,2-diphenylacetamide (0.72 g, 2.0 mmol) obtained in Reference Example 30, 2-bromoethyl methyl ether (1 ml) and acetone (5 ml), 0.24 g (yield: 24%) of the title compound was obtained as a white powdery compound in the same manner as that of Example 6.

¹H-NMR (DMSO-d₆, δ PPM): 1.08-1.38 (1H, m), 1.69-1.90 (3H, m), 1.92-2.10 (2H, m), 2.40 (3H, s), 3.24 (3H, s), 3.63 (2H, t, J=4.9Hz),3.78-4.10 (2H, m), 4.27 (2H, t, J=4.9Hz),6.85 (1H, brs), 7.18 (1H, brs), 7.21-7.43 (10H, m), 7.70 (1H, brs), 7.93 (1H, brs)
MS (ESI) m/z: 418 [M]⁺

### Example 68

### 3-(trans-3-(Carbamoyl(diphenyl)methyl)cyclopentyl)-1-cyclopentylmethyl-2-methyl-3H-imidazol-1-ium bromide

By using 2-(trans-3-(2-methylimidazol-1-yl)cyclopentyl)-2,2-diphenylacetamide (0.70 g, 1.9 mmol) obtained in Reference Example 30, bromomethylcyclopropane (1 ml) and acetone (5 ml), 0.72 g (yield: 76%) of the title compound was obtained as a white powdery compound in the same manner as that of Example 6.

¹H-NMR (DMSO-d₆, δ PPM): 0.38-0.49 (2H, m), 0.52-0.65 (2H, m), 1.12-1.40 (2H, m), 1.70-1.90 (3H, m), 1.94-2.14 (2H, m), 2.42 (3H, s), 3.79-4.08 (4H, m), 6.86 (1H, brs), 7.19 (1H, brs), 7.24-7.46 (10H, m), 7.81 (1H, brs), 7.95 (1H, brs)
MS (ESI) m/z: 414 [M]⁺

### Example 69

### 4-(cis-3-(Carbamoyl(diphenyl)methyl)cyclopentyl)-1-methyl-4H-[1,2,4]triazol-1-ium iodide

2,2-Diphenyl-2-(cis-3-[1,2,4]triazol-4-yl-cyclopentyl)acetamide (200 mg, 0.58 mmol) obtained in Reference Example 18 was dissolved in acetone (2 ml), and the solution was added with methyl iodide (819 mg, 5.77 mmol), and stirred at room temperature for 3 days. The reaction mixture was left to cool, and then the produced white crystalline powder was collected by filtration to obtain 165 mg (yield: 59%) of the title compound.

¹H-NMR (DMSO-d₆) δ: 1.34-1.53 (3H, m), 2.01 (1H, m), 2.17 (1H, m), 2.56 (1H, m), 3.71 (1H, m), 3.95 (3H, s), 4.72 (1H, m), 6.79 (1H, brs), 7.20 (1H, brs), 7.21-7.32 (10H, m), 8.97 (1H, s), 9.88 (1H, s)
MS (ESI) m/z: 361 [M]⁺

### Example 70

### 1-Benzyl-4-(cis-3-(carbamoyl(diphenyl)methyl)cyclopentyl)-4H-[1,2,4]triazol-1-ium bromide

2,2-Diphenyl-2-(cis-3-[1,2,4]triazol-4-yl-cyclopentyl)acetamide (200 mg, 0.58 mmol) obtained in Reference Example 18 was dissolved in acetone (2 ml), and the solution was added with benzyl bromide (686 mg, 5.77 mmol), and stirred for 2 hours under reflux by heating. The solution was left to cool, and then the produced white crystalline powder was collected by filtration to obtain 214 mg (yield: 72%) of the title compound.

¹H-NMR (DMSO-d₆, δ PPM): 1.32-1.55 (3H, m), 2.00 (1H, m), 2.20 (1H, m), 2.59 (1H, m), 3.71 (1H, m), 4.73 (1H, m), 5.50 (2H, s), 6.80 (1H, brs), 7.19 (1H, brs), 7.21-7.33 (10H, m), 7.41 (5H, brs), 9.00 (1H, s), 10.13 (1H, s)
MS (ESI) m/z: 437 [M]⁺

### Example 71

### 4-Benzyl-1-(cis-3-(carbamoyl(diphenyl)methyl)cyclopentyl)-1H-[1,2,4]triazol-4-ium bromide

2,2-Diphenyl-2-(cis-3-[1,2,4]triazol-1-yl-cyclopentyl)acetamide (150 mg, 0.43 mmol) obtained in Reference Example 19 was suspended in tetrahydrofuran (4 ml), and added with benzyl bromide (515 mg, 3.03 mmol), and the mixture was stirred for 5 hours under reflux by heating. The reaction mixture was left to cool, and then the produced white crystalline powder was collected by filtration to obtain 149 mg (yield: 67%) of the title compound.

¹H-NMR (DMSO-d₆, δ PPM): 1.32 (1H, m), 1.44-1.58 (2H, m), 1.93 (1H, m), 2.13 (1H, m), 2.43 (1H, m), 3.66 (1H, m), 4.93 (1H, m), 5.40 (2H, s), 6.81 (1H, brs), 7.15 (1H, brs), 7.22-7.32 (10H, m), 7.43 (5H, brs), 9.16 (1H, s), 10.09 (1H, s)
MS (ESI) m/z: 437 [M]⁺

### Example 72

### 4-(cis-3-(Carbamoyl(diphenyl)methyl)cyclopentyl)-1-(2-methoxyethyl)-4H-[1,2,4]triazol-1-ium bromide

2,2-Diphenyl-2-(cis-3-[1,2,4]triazol-4-yl-cyclopentyl)acetamide (200 mg, 0.58 mmol) obtained in Reference Example 18 was dissolved in acetone (4 ml), and the solution was added with methoxyethyl bromide (802 mg, 5.77 mmol), and stirred for 10 hours under reflux by heating. The reaction mixture was left to cool, and then the produced white crystalline powder was collected by filtration, and recrystallized from methanol/tetrahydrofuran to obtain 36 mg (yield: 13%) of the title compound.

¹H-NMR (DMSO-d₆) δ: 1.30-1.52 (3H, m), 2.00 (1H, m), 2.20 (1H, m), 2.59 (1H, m), 3.24 (3H, s), 3.71 (1H, m), 3.73 (2H, t, J=4.9Hz),4.45 (2H, t, J=4.9Hz),4.75 (1H, m), 6.80 (1H, brs), 7.20 (1H, brs), 7.21-7.33 (10H, m), 9.03 (1H, s), 10.00 (1H, s)
MS (ESI) m/z: 405 [M]⁺

### Example 73

### 3-(trans-3-(Carbamoyl(diphenyl)methyl)cyclopentyl)-2-methyl-1-(3-methylbutyl)-3H-imidazol-1-ium bromide

By using 2-(trans-3-(2-methylimidazol-1-yl)cyclopentyl)-2,2-diphenylacetamide (0.70 g, 1.9 mmol) obtained in Reference Example 30, 1-bromo-3-methylbutane (1 ml) and acetone (5 ml), 0.51 g (yield: 53%) of the title compound was obtained as a white powdery compound in the same manner as that of Example 6.

¹H-NMR (DMSO-d₆, δ PPM): 0.91 (6H, d, J=6.1Hz),1.22-1.40 (1H, m), 1.48-2.14 (8H, m), 2.41 (3H, s), 3.78-4.14 (4H, m), 6.87 (1H, brs), 7.19 (1H, brs), 7.22-7.44 (10H, m), 7.78 (1H, brs), 7.95 (1H, brs)
MS (ESI) m/z: 430 [M]⁺

### Example 74

### 3-(trans-3-(Carbamoyl(diphenyl)methyl)cyclogentyl)-2-methyl-1-oxiranylmethyl-3H-imidazol-1-ium bromide

By using 2-(trans-3-(2-methylimidazol-1-yl)cyclopentyl)-2,2-diphenylacetamide (0.55 g, 1.5 mmol) obtained in Reference Example 30, epibromohydrin (1 ml) and acetone (7 ml), 0.17 g (yield: 23%) of the title compound was obtained as a white powdery compound in the same manner as that of Example 6.

¹H-NMR (DMSO-d₆, δ PPM): 1.08-1.38 (1H, m), 1.70-2.12 (5H, m), 2.30-2.54 (4H, m), 2.56-2.68 (1H, m), 2.78-2.90 (1H, m), 3.78-4.22 (3H, m), 4.48-4.60 (1H, m), 6.89 (1H, brs), 7.10-7.44 (11H, m), 7.73 (1H, brs), 7.98 (1H, brs)
MS (ESI) m/z: 416 [M]⁺

### Example 75

### 1-(3-Acetoxypropyl)-3-(trans-3-(carbamoyl(diphenyl)methyl)cyclopentyl)-2-methyl-3H-imidazol-1-ium bromide

By using 2-(trans-3-(2-methylimidazol-1-yl)cyclopentyl)-2,2-diphenylacetamide (0.55 g, 1.5 mmol) obtained in Reference Example 30, acetic acid 3-bromopropyl ester (0.8 ml) and acetone (7 ml), 0.08 g (yield: 10%) of the title compound was obtained as a white powdery compound in the same manner as that of Example 6.

¹H-NMR (DMSO-d₆, δ PPM): 1.20-1.40 (1H, m), 1.71-2.12 (10H, m), 2.41 (3H, s), 3.80-4.08 (4H, m), 4.10-4.22 (2H, m), 6.87 (1H, brs), 7.19 (1H, brs), 7.22-7.42 (10H, m), 7.78 (1H, brs), 7.96 (1H, brs)
MS (ESI) m/z: 460 [M]⁺

### Example 76

### 3-((1S,3R)-3-(Carbamoyl(diphenyl)methyl)cyclopentyl)-1-(2-methoxyethyl)-2-methyl-3H-imidazol-1-ium para-toluenesulfonate

By using 2-((1R,3S)-3-(2-methylimidazol-1-yl)cyclopentyl)-2,2-diphenylacetamide (0.36 g, 1.00 mmol) obtained in Reference Example 23 and para-toluenesulfonic acid 2-methoxyethyl ester (1.15 g, 0.01 mol), 0.11 g (yield: 18%) of the title compound was obtained as white amorphous solid.

¹H-NMR (DMSO-d₆, δ PPM): 1.32-1.49 (3H, m), 1.94-2.06 (2H, m), 2.28 (3H, s), 2.37-2.44 (1H, m), 2.57 (3H, s), 3.22 (3H, s), 3.59-3.67 (3H, m), 4.22-4.25 (2H, m), 4.76-4.80 (1H, m), 6.74 (1H, brs), 6.92 (1H, brs), 7.09-7.11 (2H, m), 7.18 (1H, brs), 7.25-7.35 (10H, m), 7.46-7.48 (2H, m), 7.51-7.52 (1H, m)
MS (ESI) m/z: 418 [M]⁺

### Example 77

### 3-((1R,3S)-3-(Carbamoyl(diphenyl)methyl)cyclopentyl)-1-(2-methoxyethyl)-2-methyl-3H-imidazol-1-ium para-toluenesulfonate

By using 2-((1S,3R)-3-(2-methylimidazol-1-yl)cyclopentyl)-2,2-diphenylacetamide (0.36 g, 1.00 mmol) obtained in Reference Example 22 and para-toluenesulfonic acid 2-methoxyethyl ester (1.15 g, 0.01 mol), 0.11 g (yield: 18%) of the title compound was obtained as white amorphous solid.

¹H-NMR (DMSO-d₆, δ PPM): 1.32-1.49 (3H, m), 1.94-2.06 (2H, m), 2.28 (3H, s), 2.37-2.44 (1H, m), 2.57 (3H, s), 3.22 (3H, s), 3.59-3.67 (3H, m), 4.22-4.25 (2H, m), 4.76-4.80 (1H, m), 6.74 (1H, brs), 6.92 (1H, brs), 7.09-7.11 (2H, m), 7.18 (1H, brs), 7.25-7.35 (10H, m), 7.46-7.48 (2H, m), 7.51-7.52 (1H, m)
MS (ESI) m/z: 418 [M]⁺

### Example 78

### 3-((1S,3R)-3-(Carbamoyl(diphenyl)methyl)cyclopentyl)-1-buten-2-yl-2-methyl-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and crotyl bromide (1.35 g, 0.01 mol), 0.41 g (yield: 83%) of the title compound was obtained as a white crystalline compound in the same manner as that of Example 6.

¹H-NMR (DMSO-d₆, δ PPM): 1.32-1.49 (3H, m), 1.66-1.68 (2H, m), 1.72-1.74 (1H, m), 1.94-2.06 (2H, m), 2.39-2.42 (1H, m), 2.56 (3H, d, J=3.3Hz),3.64-3.68 (1H, m), 4.64 (1H, m), 4.74-4.79 (2H, m), 5.53-5.58 (1H, m), 5.72-5.77 (1H, m), 6.75 (1H, brs), 6.91-6.92 (1H, m), 7.19 (1H, brs), 7.25-7.34 (10H, m), 7.52 (1H, d, J=1.9Hz)
MS (ESI) m/z: 414 [M]⁺

### Example 79

### 3-((1S,3R)-3-(Carbamoyl(diphenyl)methyl)cyclopentyl)-2-methyl-1-(3-methyl-butyl)-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and isoamyl bromide (1.51 g, 0.01 mol), 0.26 g (yield: 52%) of the title compound was obtained as a white crystalline compound in the same manner as that of Example 6.

¹H-NMR (DMSO-d₆, δ PPM): 0.90 (6H, d, J=5.7Hz),1.28-1.57 (6H, m), 1.94-2.06 (2H, m), 2.39-2.42 (1H, m), 2.57 (3H, s), 3.63-3.68 (1H, m), 4.01-4.05 (2H, m), 4.74-4.78 (1H, m), 6.75 (1H, brs), 6.93 (1H, d, J=1.2Hz),7.19 (1H, brs), 7.28-7.35 (10H, m), 7.59 (1H, d, J=1.2Hz)
MS (ESI) m/z: 430 [M]⁺

### Example 80

### 3-((1S,3R)-3-(Carbamoyl(diphenyl)methyl)cyclopentyl)-1,2-dimethyl-3H-imidazol-1-ium iodide

By using 2-((1R,3S)-3-(2-methylimidazol-1-yl)cyclopentyl)-2,2-diphenylacetamide (0.36 g, 1.00 mmol) obtained in Reference Example 23 and iodomethane (1.42 g, 0.01 mol), 0.43 g (yield: 84%) of the title compound was obtained as pale yellow amorphous solid.

¹H-NMR (DMSO-d₆, δ PPM): 1.26-1.49 (3H, m), 1.95-2.05 (2H, m), 2.37-2.40 (1H, m), 2.54 (3H, s), 3.63-3.74 (4H, m), 4.75-4.79 (1H, m), 6.74 (1H, brs), 6.88-6.89 (1H, m), 7.27 (1H, brs), 7.27-7.35 (10H, m), 7.51 (1H, brs)
MS (ESI) m/z: 374 [M]⁺

### Example 81

### 3-((1S,3R)-3-(Carbamoyl(diphenyl)methyl)cyclopentyl)-1-difluoromethyl-2-methyl-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and ethyl bromodifluoroacetate (2.03 g, 0.01 mol), 0.36 g (yield: 73%) of the title compound was obtained as a white crystalline compound in the same manner as that of Example 6.

¹H-NMR (DMSO-d₆, δ PPM): 1.37-1.49 (3H, m), 1.96-2.08 (2H, m), 2.40-2.44 (1H, m), 2.75 (3H, s), 3.63-3.68 (1H, m), 4.83-4.87 (1H, m), 6.73 (1H, brs), 7.17-7.35 (12H, m), 7.88-8.17 (2H, m)
MS (ESI) m/z: 410 [M]⁺

### Example 82

### 3-((1S,3R)-3-(Carbamoyl(diphenyl)methyl)cyclopentyl)-1-butyn-2-yl-2-methyl-3H imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and 1-bromo-2-butyne (1.33 g, 0.01 mol), 0.42 g (yield: 85%) of the title compound was obtained as a pale yellow crystalline compound in the same manner as that of Example 6.

¹H-NMR (DMSO-d₆, δ PPM): 1.29-1.50 (3H, m), 1.84-1.86 (3H, m), 1.94-2.07 (2H, m), 2.39-2.42 (1H, m), 2.60 (3H, s), 3.63-3.68 (1H, m), 4.76-4.80 (1H, m), 4.98-4.99 (2H, m), 6.75 (1H, brs), 6.94 (1H, d, J=2.2Hz),7.19 (1H, brs), 7.26-7.35 (10H, m), 7.61 (1H, d, J=2.2Hz)
MS (ESI) m/z: 412 [M]⁺

### Example 83

### 3-((1S,3R)-3-(Carbamoyl(diphenyl)methyl)cyclolpentyl)-1-benzyl-2-methyl-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and benzyl bromide (1.71 g, 0.01 mol), 0.46 g (yield: 87%) of the title compound was obtained as a white crystalline compound in the same manner as that of Example 6.

¹H-NMR (DMSO-d₆, δ PPM): 1.29-1.48 (3H, m), 1.94-2.07 (2H, m), 2.39-2.45 (1H, m), 2.59 (3H, s), 3.63-3.67 (1H, m), 4.75-4.79 (1H, m), 5.33 (2H, s), 6.74 (1H, brs), 6.96-6.97 (1H, m), 7.18-7.42 (16H, m), 7.61 (1H, s)
MS (ESI) m/z: 430 [M]⁺

### Example 84

### 3-((1S,3R)-3-(Carbamoyl(diphenyl)methyl)cyclopentyl)-1-hydroxyethyl-2-methyl-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and 2-bromoethanol (1.25 g, 0.01 mol), 0.34 g (yield: 70%) of the title compound was obtained as a white crystalline compound in the same manner as that of Example 6.

¹H-NMR (DMSO-d₆, δ PPM): 1.28-1.50 (3H, m), 1.95-2.06 (2H, m), 2.38-2.43 (1H, m), 2.57 (3H, s), 3.65-3.67 (3H, m), 4.10-4.12 (2H, m), 4.76-4.80 (1H, m), 5.01-5.04 (1H, m), 6.74 (1H, brs), 6.91 (1H, brs), 7.19 (1H, brs), 7.28-7.35 (10H, m), 7.53 (1H, brs)
MS (ESI) m/z: 404 [M]⁺

### Example 85

### 3-((1S,3R)-3-(Carbamoyl(diphenyl)methyl)cyclopentyl)-1-(4-fluorobenzyl)-2-methyl-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and 4-fluorobenzyl bromide (1.89 g, 0.01 mol), 0.33 g (yield: 60%) of the title compound was obtained as a white crystalline compound in the same manner as that of Example 6.

¹H-NMR (DMSO-d₆, δ PPM): 1.29-1.48 (3H, m), 1.94-2.07 (2H, m), 2.40-2.45 (1H, m), 2.60 (3H, s), 3.63-3.67 (1H, m), 4.75-4.79 (1H, m), 5.32 (2H, s), 6.74 (1H, brs), 6.96 (1H, brs), 7.18-7.40 (15H, m), 7.60 (1H, m)
MS (ESI) m/z: 468 [M]⁺

### Example 86

### 3-(trans-3-(Carbamoyl(diphenyl)methyl)cyclopentyl)-2-methyl-1-(2-oxopropyl)-3H-imidazol-1-ium bromide

By using 2-(trans-3-(2-methylimidazol-1-yl)cyclopentyl)-2,2-diphenylacetamide (0.65 g, 1.8 mmol) obtained in Reference Example 30, bromoacetone (1 ml) and tetrahydrofuran (10 ml), 0.18 g (yield: 20%) of the title compound was obtained as a white powdery compound in the same manner as that of Example 6.

¹H-NMR (DMSO-d₆, δ PPM): 1.20-1.39 (1H, m), 1.70-1.92 (3H, m), 1.94-2.13 (2H, m), 2.25 (3H, s), 2.30 (3H, s), 3.80-3.97 (1H, m), 3.99-4.15 (1H, m), 5.33 (2H, s), 6.88 (1H, brs), 7.12-7.44 (11H, m), 7.58 (1H, brs), 7.98 (1H, brs)
MS (ESI) m/z: 416 [M]⁺

### Example 87

### 1-Benzyl-3-(trans-3-(cyano(diphenyl)methyl)cyclopentyl)-2-methyl-3H-imidazol-1-ium bromide

By using (trans-3-(2-methylimidazol-1-yl)cyclopentyl)diphenylacetonitrile (0.68 g, 2.0 mmol) obtained in Reference Example 29, benzyl bromide (1 ml) and acetone (7 ml), 0.38 g (yield: 37%) of the title compound was obtained as a white powdery compound in the same manner as that of Example 6.

¹H-NMR (DMSO-d₆, δ PPM): 1.46-1.64 (1H, m), 1.73-2.09 (4H, m), 2.24-2.40 (1H, m), 2.64 (3H, s), 3.83-4.00 (1H, m), 4.84-5.04 (1H, m), 5.39 (2H, s), 7.20-7.60 (15H, m), 7.82 (1H, brs), 8.03 (1H, brs)
MS (ESI) m/z: 432 [M]⁺

### Example 88

### 3-(trans-3-(Cyano(diphenyl)methyl)cyclopentyl)-2-methyl-1-(2-oxopropyl)-3H-imidazol-1-ium bromide

By using (trans-3-(2-methylimidazol-1-yl)cyclopentyl)diphenylacetonitrile (0.75 g, 2.2 mmol) obtained in Reference Example 29, bromoacetone (1.2 ml) and acetone (10 ml), 0.36 g (yield: 34%) of the title compound was obtained as a white powdery compound in the same manner as that of Example 6.

¹H-NMR (DMSO-d₆, δ PPM): 1.46-1.64 (1H, m), 1.77-2.09 (4H, m), 2.25 (3H, s), 2.28-2.40 (1H, m), 2.50 (3H, s), 3.83-4.01 (1H, m), 4.88-5.02 (1H, m), 5.32 (2H, s), 7.24-7.65 (11H, m), 8.00 (1H, brs)
MS (ESI) m/z: 398 [M]⁺

### Example 89

### 3-((1S,3R)-3-(Carbamoyl(diphenyl)methyl)cyclopentyl)-2-methyl-1-(2-oxopropyl)-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and bromoacetone (1.37 g, 0.01 mol), 0.41 g (yield: 83%) of the title compound was obtained as a white crystalline compound in the same manner as that of Example 6.

¹H-NMR (DMSO-d₆, δ PPM): 1.26-1.51 (3H, m), 1.95-2.09 (2H, m), 2.22 (3H, s), 2.39-2.51 (4H, m), 3.64-3.68 (1H, m), 4.79-4.83 (1H, m), 5.25 (2H, s), 6.75 (1H, brs), 6.94-6.95 (1H, m), 7.19 (1H, brs), 7.27-7.39 (11H, m)
MS (ESI) m/z: 416 [M]⁺

### Example 90

### 3-((1R,3S)-3-(Carbamoyl(diphenyl)methyl)cyclopentyl)-2-methyl-1-(2-oxopropyl)-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and bromoacetone (1.37 g, 0.01 mol), 0.45 g (yield: 91%) of the title compound was obtained as a white crystalline compound in the same manner as that of Example 6.

¹H-NMR (DMSO-d₆, δ PPM): 1.26-1.51 (3H, m), 1.95-2.09 (2H, m), 2.22 (3H, s), 2.39-2.51 (4H, m), 3.64-3.68 (1H, m), 4.79-4.83 (1H, m), 5.25 (2H, s), 6.75 (1H, brs), 6.94-6.95 (1H, m), 7.19 (1H, brs), 7.27-7.39 (11H, m)
MS (ESI) m/z: 416 [M]⁺

### Example 91

### 3-((1R,3S)-3-(Carbamoyl(diphenyl)methyl)cyclopentyl)-1-benzyl-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and benzyl bromide (1.71 g, 0.01 mol), 0.54 g (yield: 99%) of the title compound was obtained as a white crystalline compound in the same manner as that of Example 6.

¹H-NMR (DMSO-d₆, δ PPM): 1.29-1.48 (3H, m), 1.94-2.07 (2H, m), 2.39-2.45 (1H, m), 2.59 (3H, s), 3.63-3.67 (1H, m), 4.75-4.79 (1H, m), 5.33 (2H, s), 6.74 (1H, brs), 6.96-6.97 (1H, m), 7.18-7.42 (16H, m), 7.61 (1H, s)
MS (ESI) m/z: 450 [M]⁺

### Example 92

### 3-((1R,3S)-3-(Carbamoyl(diphenyl)methyl)cyclopentyl)-1-difluoromethyl-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and ethyl bromodifluoroacetate (2.03 g, 0.01 mol), 0.33 g (yield: 68%) of the title compound was obtained as a white crystalline compound in the same manner as that of Example 6.

¹H-NMR (DMSO-d₆, δ PPM): 1.37-1.49 (3H, m), 1.96-2.08 (2H, m), 2.40-2.44 (1H, m), 2.75 (3H, s), 3.63-3.68 (1H, m), 4.83-4.87 (1H, m), 6.73 (1H, brs), 7.17-7.35 (12H, m), 7.88-8.17 (2H, m)
MS (ESI) m/z: 410 [M]⁺

### Example 93

### 3-((1R,3S)-3-(Carbamoyl(diphenyl)methyl)cyclopentyl)-1,2-dimethyl-3H-imidazol-1-ium para-toluenesulfonate

By using 2-((1S,3R)-3-(2-methylimidazol-1-yl)cyclopentyl)-2,2-diphenylacetamide (0.36 g, 1.00 mmol) obtained in Reference Example 22 and methyl para-toluenesulfonate (1.86 g, 0.01 mol), 0.30 g (yield: 40%) of the title compound was obtained as a white crystalline compound in the same manner as that of Example 6.

¹H-NMR (DMSO-d₆, δ PPM): 1.25-1.49 (3H, m), 1.94-2.05 (2H, m), 2.28 (3H, s), 2.37-2.40 (1H, m), 2.52 (3H, s), 3.63-3.66 (4H, m), 4.74-4.78 (1H, m), 6.74 (1H, brs), 6.88 (1H, brs), 7.09-7.11 (2H, m), 7.18 (1H, brs), 7.26-7.35 (10H, m), 7.46-7.50 (3H, m)
MS (ESI) m/z: 374 [M]⁺

### Example 94

### 3-((1S,3R)-3-(Carbamoyl(diphenyl)methyl)cyclopentyl)-1,2-dimethyl-3H-imidazol-1-ium para-toluenesulfonate

By using 2-((1R,3S)-3-(2-methylimidazol-1-yl)cyclopentyl)-2,2-diphenylacetamide (0.36 g, 1.00 mmol) obtained in Reference Example 23 and methyl para-toluenesulfonate (1.86 g, 0.01 mol), 0.31 g (yield: 40%) of the title compound was obtained as a white crystalline compound in the same manner as that of Example 6.

¹H-NMR (DMSO-d₆, δ PPM): 1.25-1.49 (3H, m), 1.94-2.05 (2H, m), 2.28 (3H, s), 2.37-2.40 (1H, m), 2.52 (3H, s), 3.63-3.66 (4H, m), 4.74-4.78 (1H, m), 6.74 (1H, brs), 6.88 (1H, brs), 7.09-7.11 (2H, m), 7.18 (1H, brs), 7.26-7.35 (10H, m), 7.46-7.50 (3H, m)
MS (ESI) m/z: 374 [M]⁺

### Example 95

### 3-(trans-3-(Carbamoyl(diphenyl)methyl)cyclopentyl)-1,2-dimethyl-3H-imidazol-1-ium iodide

By using 2-(trans-3-(2-methylimidazol-1-yl)cyclopentyl)-2,2-diphenylacetamide (0.54 g, 1.5 mmol) obtained in Reference Example 38, methyl iodide (1 ml), acetone (5 ml) and tetrahydrofuran (8 ml), 0.64 g (yield: 85%) of the title compound was obtained as a yellow amorphous compound.

¹H-NMR (DMSO-d₆, δ PPM): 1.08-1.38 (1H, m), 1.64-1.88 (3H, m), 1.90-2.10 (2H, m), 2.37 (3H, s), 3.70 (3H, s), 3.76-4.08 (2H, m), 6.85 (1H, brs), 7.18 (1H, brs), 7.21-7.44 (10H, m), 7.68 (1H, brs), 7.88 (1H, brs)
MS (ESI) m/z: 374 [M]⁺

### Example 96

### 3-(trans-3-(Carbamoyl(diphenyl)methyl)cyclopentyl)-1,2-dimetbyl-3H-imidazol-1-ium iodide

By using 2-(trans-3-(2-methylimidazol-1-yl)cyclopentyl)-2,2-diphenylacetamide (0.54 g, 1.5 mmol) obtained in Reference Example 39, methyl iodide (1 ml), acetone (5 ml) and tetrahydrofuran (8 ml), 0.53 g (yield: 71%) of the title compound was obtained as a yellow amorphous compound.

¹H-NMR (DMSO-d₆, δ PPM): 1.08-1.38 (1H, m), 1.64-1.88 (3H, m), 1.90-2.10 (2H, m), 2.37 (3H, s), 3.70 (3H, s), 3.76-4.08 (2H, m), 6.85 (1H, brs), 7.18 (1H, brs), 7.21-7.44 (10H, m), 7.68 (1H, brs), 7.88 (1H, brs)
MS (ESI) m/z: 374 [M]⁺

### Example 97

### 1-Benzyl-3-(trans-3-(carbamoyl(diphenyl)methyl)cyclopentyl)-2-methyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 38, benzyl bromide (1 ml) and tetrahydrofuran (8 ml), 0.56 g (yield: 70%) of the title compound was obtained as a yellow amorphous compound.

¹H-NMR (DMSO-d₆, δ PPM): 1.20-1.39 (1H, m), 1.70-1.90 (3H, m), 1.92-2.10 (2H, m), 2.44 (3H, s), 3.82-4.10 (2H, m), 5.38 (2H, s), 6.86 (1H, brs), 7.10-7.43 (16H, m), 7.82 (1H, brs), 8.00 (1H, brs)
MS (ESI) m/z: 450 [M]⁺

### Example 98

### 1-Benzyl-3-(trans-3-(carbamoyl(diphenyl)methyl)cyclopentyl)-2-methyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 39, benzyl bromide (1 ml) and tetrahydrofuran (8 ml), 0.51 g (yield: 64%) of the title compound was obtained as a yellow amorphous compound.

¹H-NMR (DMSO-d₆, δ PPM): 1.20-1.39 (1H, m), 1.70-1.90 (3H, m), 1.92-2.10 (2H, m), 2.44 (3H, s), 3.82-4.10 (2H, m), 5.38 (2H, s), 6.86 (1H, brs), 7.10-7.43 (16H, m), 7.82 (1H, brs), 8.00 (1H, brs)
MS (ESI) m/z: 450 [M]⁺

### Example 99

### 3-(trans-3-(Carbamoyl(diphenyl)methyl)cyclonentyl)-2-methyl-1-(2-oxopropyl)-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 39, bromoacetone (1 ml) and tetrahydrofuran (8 ml), 0.58 g (yield: 77%) of the title compound was obtained as a white powdery compound.

¹H-NMR (DMSO-d₆, δ PPM): 1.20-1.39 (1H, m), 1.70-1.92 (3H, m), 1.94-2.13 (2H, m), 2.25 (3H, s), 2.30 (3H, s), 3.80-3.97 (1H, m), 3.99-4.15 (1H, m), 5.33 (2H, s), 6.88 (1H, brs), 7.12-7.44 (11H, m), 7.58 (1H, brs), 7.98 (1H, brs)
MS (ESI) m/z: 416 [M]⁺

### Example 100

### 3-(trans-3-(Carbamoyl(diphenyl)methyl)cyclopentyl)-1-(2-methoxyethyl)-2-methyl-3H-imidazol-1-ium para-toluenesulfonate

By using 2-(trans-3-(2-methylimidazol-1-yl)cyclopentyl)-2,2-diphenylacetamide (0.54 g, 1.5 mmol) obtained in Reference Example 38, para-toluenesulfonic acid 2-methoxyethyl ester (1 ml) and tetrahydrofuran (8 ml), 0.27 g (yield: 25%) of the title compound was obtained as a colorless amorphous compound.

¹H-NMR (DMSO-d₆, δ PPM): 1.18-1.40 (1H, m), 1.65-2.12 (5H, m), 2.28 (3H, s), 2.40 (3H, s), 3.24 (3H, s), 3.50-3.70 (2H, m), 3.77-4.12 (2H, m), 4.16-4.36 (2H, m), 6.84 (1H, brs), 6.96-7.52 (15H, m), 7.69 (1H, brs), 7.92 (1H, brs)
MS (ESI) m/z: 418 [M]⁺

### Example 101

### 1-(cis-3-(Carbamoyl(diphenyl)methyl)cyclopentyl)-4-(2-oxopropyl)-1H-[1,2,4]triazol-4-ium bromide

By using 2,2-diphenyl-2-(cis-3-[1,2,4]triazol-1-yl-cyclopentyl)acetamide (0.2 g, 0.58 mmol) obtained in Reference Example 19, bromoacetone (0.54 ml) and acetone (4 ml), 0.23 g (yield: 82%) of the title compound was obtained as a white crystalline compound in the same manner as that of Example 6.

¹H-NMR (DMSO-d₆, δ PPM): 1.21-1.59 (3H, m), 1.83-2.00 (1H, m), 2.04-2.10 (1H, m), 2.25 (3H, s), 2.38-2.50 (1H, m), 3.57-3.73 (1H, m), 4.99-5.12 (1H, m), 5.32 (2H, s), 6.82 (1H, brs), 7.16 (1H, brs), 7.18-7.40 (10H, m), 8.90 (1H, s), 9.89 (1H, s)
MS (ESI) m/z: 403 [M]⁺

### Example 102

### 4-(cis-3-(Carbamoyl(diphenyl)methyl)cyclopentyl)-1-(2-oxopronyl)-4H-[1,2,4]triazol-1-ium bromide

By using 2,2-diphenyl-2-(cis-3-[1,2,4]triazol-4-yl-cyclopentyl)acetamide (0.2 g, 0.58 mmol) obtained in Reference Example 18, bromoacetone (0.54 ml) and acetone (4 ml), 0.26 g (yield: 92%) of the title compound was obtained as a white crystalline compound in the same manner as that of Example 6.

¹H-NMR (DMSO-d₆, δ PPM): 1.28-1.56 (3H, m), 1.84-2.09 (1H, m), 2.10-2.29 (4H, m), 2.50-2.66 (1H, m), 3.60-3.76 (1H, m), 4.72-4.90 (1H, m), 5.50 (2H, s), 6.78 (1H, brs), 7.13-7.40 (11H, m), 9.07 (1H, s), 9.93 (1H, s)
MS (ESI) m/z: 403 [M]⁺

### Example 103

### 3-(cis-3-(Carbamoyl(diphenyl)methyl)cyclopentyl)-2-ethyl-1-(2-oxopropyl)-3H-imidazol-1-ium bromide

By using 2-(cis-3-(2-ethylimidazol-1-yl)cyclopentyl)-2,2-diphenylacetamide (0.2 g, 0.54 mmol) obtained in Reference Example 15, bromoacetone (0.5 ml) and acetone (4 ml), 0.26 g (yield: 94%) of the title compound was obtained as a white crystalline compound in the same manner as that of Example 6.

¹H-NMR (DMSO-d₆, δ PPM): 1.05 (3H, t, J=7.5Hz),1.11-1.54 (3H, m), 1.90-2.14 (2H, m), 2.24 (3H, s), 2.31-2.46 (1H, m), 2.95 (2H, dd, J=15.0, 7.5Hz), 3.56-3.74 (1H, m), 4.72-4.90 (1H, m), 5.29 (2H, s), 6.78 (1H, brs), 6.89 (1H, brs), 7.16-7.44 (12H, m)
MS (ESI) m/z: 430 [M]⁺

### Example 104

### 1-Benzyl-3-(cis-3-(carbamoyl(diphenyl)methyl)cyclopentyl)-2-ethyl-3H-imidazol-1-ium bromide

By using 2-(cis-3-(2-ethylimidazol-1-yl)cyclopentyl)-2,2-diphenylacetamide (0.2 g, 0.54 mmol) obtained in Reference Example 15, benzyl bromide (0.65 g, 3.78 mmol) and acetone (4 ml), 0.23 g (yield: 70%) of the title compound was obtained as a white crystalline compound in the same manner as that of Example 6.

¹H-NMR (DMSO-d₆, δ PPM): 0.88-0.98 (3H, m), 1.22-1.54 (3H, m), 1.84-2.10 (2H, m), 2.31-2.50 (1H, m), 3.01-3.19 (2H, m), 3.58-3.78 (1H, m), 4.70-4.90 (1H, m), 5.39 (2H, s), 6.78 (1H, brs), 6.92 (1H, brs), 7.15-7.43 (16H, m), 7.67 (1H, brs)
MS (ESI) m/z: 464 [M]⁺

### Example 105

### 3-(cis-3-(Carbamoyl(diphenyl)methyl)cyclopentyl)-2-ethyl-1-methyl-3H-imidazol-1-ium iodide

By using 2-(cis-3-(2-ethylimidazol-1-yl)cyclopentyl)-2,2-diphenylacetamide (0.2 g, 0.54 mmol) obtained in Reference Example 15, methyl iodide (0.34 ml) and tetrahydrofuran (4 ml), 0.27 g (yield: 97%) of the title compound was obtained as a white crystalline compound in the same manner as that of Example 6.

¹H-NMR (DMSO-d₆, δ PPM): 1.08-1.18 (3H, m), 1.19-1.53 (3H, m), 1.88-2.11 (2H, m), 2.30-2.44 (1H, m), 2.91-3.08 (2H, m), 3.52-3.78 (4H, m), 4.72-4.88 (1H, m), 6.77 (1H, brs), 6.85 (1H, brs), 7.14-7.40 (11H, m), 7.54 (1H, brs)
MS (ESI) m/z: 388 [M]⁺

### Example 106

### 3-(trans-3-(Carbamoyl(dinhenyl)methyl)cyclopentyl)-1-(2-methoxyethyl)-2-methyl-3H-imidazol-1-ium para-toluenesulfonate

By using 2-(trans-3-(2-methylimidazol-1-yl)cyclopentyl)-2,2-diphenylacetamide (0.54 g, 1.5 mmol) obtained in Reference Example 39, para-toluenesulfonic acid 2-methoxyethyl ester (1 ml) and tetrahydrofuran (8 ml), 0.22 g (yield: 25%) of the title compound was obtained as a colorless amorphous compound.

¹H-NMR (DMSO-d₆, δ PPM): 1.18-1.40 (1H, m), 1.65-2.12 (5H, m), 2.28 (3H, s), 2.40 (3H, s), 3.24 (3H, s), 3.50-3.70 (2H, m), 3.77-4.12 (2H, m), 4.16-4.36 (2H, m), 6.84 (1H, brs), 6.96-7.52 (15H, m), 7.69 (1H, brs), 7.92 (1H, brs)
MS (ESI) m/z: 418 [M]⁺

### Example 107

### 3-((1R,3S)-3-(Carbamoyl(diphenyl)methyl)cyclopentyl)-1-hydroxyethyl-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and 2-bromoethanol (1.25 g, 0.01 mol), 0.25 g (yield: 51%) of the title compound was obtained as a white crystalline compound in the same manner as that of Example 6.

¹H-NMR (DMSO-d₆, δ PPM): 1.28-1.50 (3H, m), 1.95-2.06 (2H, m), 2.38-2.43 (1H, m), 2.57 (3H, s), 3.65-3.67 (3H, m), 4.10-4.12 (2H, m), 4.76-4.80 (1H, m), 5.01-5.04 (1H, m), 6.74 (1H, brs), 6.91 (1H, brs), 7.19 (1H, brs), 7.28-7.35 (10H, m), 7.53 (1H, brs)
MS (ESI) m/z: 404 [M]⁺

### Example 108

### 3-(trans-3-(Carbamoyl(dinhenyl)methyl)cyclopentyl)-1-cyclopentylmethyl-2-methyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 38, bromomethylcyclopropane (1 ml) and acetone (8 ml), 0.57 g (yield: 77%) of the title compound was obtained as a colorless amorphous compound.

¹H-NMR (DMSO-d₆, δ PPM): 0.38-0.49 (2H, m), 0.52-0.65 (2H, m), 1.12-1.40 (2H, m), 1.70-1.90 (3H, m), 1.94-2.14 (2H, m), 2.42 (3H, s), 3.79-4.08 (4H, m), 6.86 (1H, brs), 7.19 (1H, brs), 7.24-7.46 (10H, m), 7.81 (1H, brs), 7.95 (1H, brs)
MS (ESI) m/z: 414 [M]⁺

### Example 109

### 3-(trans-3-(Carbamoyl(diphenyl)methyl)cyclopentyl)-1-cyclopentylmethyl-2-methyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 39, bromomethylcyclopropane (1 ml) and acetone (8 ml), 0.60 g (yield: 80%) of the title compound was obtained as a colorless amorphous compound.

¹H-NMR (DMSO-d₆, δ PPM): 0.38-0.49 (2H, m), 0.52-0.65 (2H, m), 1.12-1.40 (2H, m), 1.70-1.90 (3H, m), 1.94-2.14 (2H, m), 2.42 (3H, s), 3.79-4.08 (4H, m), 6.86 (1H, brs), 7.19 (1H, brs), 7.24-7.46 (10H, m), 7.81 (1H, brs), 7.95 (1H, brs)
MS (ESI) m/z: 414 [M]⁺

### Example 110

### 3-(cis-3-(Carbamoyl(diphenyl)methyl)cyclopentyl)-2-ethyl-1-(2-methoxyethyl)-3H-imidazol-1-ium para-toluenesulfonate

By using 2-(cis-3-(2-ethylimidazol-1-yl)cyclopentyl)-2,2-diphenylacetamide (0.2 g, 0.54 mmol) obtained in Reference Example 15, para-toluenesulfonic acid 2-methoxyethyl ester (0.87 g, 3.78 mmol) and 1,4-dioxane (4 ml), 0.22 g (yield: 68%) of the title compound was obtained as a white amorphous compound.

¹H-NMR (DMSO-d₆, δ PPM): 1.14 (3H, t, J=7.5Hz),1.22-1.55 (3H, m), 1.88-2.14 (2H, m), 2.29 (3H, s), 2.31-2.43 (1H, m), 2.96-3.10 (2H, m), 3.34 (3H, s), 3.53-3.78 (3H, m), 4.16-4.30 (2H, m), 4.72-4.90 (1H, m), 6.77 (1H, brs), 6.87 (1H, brs), 7.11 (2H, d, J=8.1Hz),7.11-7.38 (11H, m), 7.47 (2H, d, J=8.1Hz),7.58 (1H, brs)
MS (ESI) m/z: 432 [M]⁺

### Example 111

### 1-(cis-3-(Carbamoyl(diphenyl)methyl)cvclopentyl)-4-(2-methoxyethyl)-1H-[1,2,4]triazol-4-ium para-toluenesulfonate

By using 2,2-diphenyl-2-(cis-3-[1,2,4]triazol-1-yl-cyclopentyl)acetamide (0.2 g, 0.58 mmol) obtained in Reference Example 19, para-toluenesulfonic acid 2-methoxyethyl ester (0.94 g, 4.1 mmol) and 1,4-dioxane (4 ml), 0.18 g (yield: 54%) of the title compound was obtained as a white crystalline compound in the same manner as that of Example 6.

¹H-NMR (DMSO-d₆, δ PPM): 1.24-1.60 (3H, m), 1.86-2.02 (1H, m), 2.04-2.12 (1H, m), 2.29 (3H, s), 2.38-2.54 (1H, m), 3.26 (3H, s), 3.59-3.72 (3H, m), 4.14-4.40 (2H, m), 4.88-5.08 (1H, m), 6.79 (1H, brs), 7.08-7.21 (3H, m), 7.23-7.42 (10H, m), 7.47 (2H, d, J=8.0Hz),9.01 (1H, s), 9.95 (1H, s)
MS (ESI) m/z: 405 [M]⁺

### Example 112

### 3-(trans-3-(Carbamoyl(diphenyl)methyl)cyclopentyl)-2-methyl-1-(3-methylbutyl)-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 38, bromo-3-methylbutane (1 ml) and tetrahydrofuran (8 ml), 0.51 g (yield: 66%) of the title compound was obtained as a colorless amorphous compound.

¹H-NMR (DMSO-d₆, δ PPM): 0.91 (6H, d, J=6.1Hz),1.22-1.40 (1H, m), 1.48-2.14 (8H, m), 2.41 (3H, s), 3.78-4.14 (4H, m), 6.87 (1H, brs), 7.19 (1H, brs), 7.22-7.44 (10H, m), 7.78 (1H, brs), 7.95 (1H, brs)
MS (ESI) m/z: 430 [M]⁺

### Example 113

### 3-(trans-3-(Carbamoyl(diphenyl)methyl)cyclopentyl)-2-methyl-1-(3-methylbutyl)-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 39, bromo-3-methylbutane (1 ml) and tetrahydrofuran (8 ml), 0.45 g (yield: 59%) of the title compound was obtained as a colorless amorphous compound.

¹H-NMR (DMSO-d₆, δ PPM): 0.91 (6H, d, J=6.1Hz), 1.22-1.40 (1H, m), 1.48-2.14 (8H, m), 2.41 (3H, s), 3.78-4.14 (4H, m), 6.87 (1H, brs), 7.19 (1H, brs), 7.22-7.44 (10H, m), 7.78 (1H, brs), 7.95 (1H, brs)
MS (ESI) m/z: 430 [M]⁺

### Example 114

### 1-(3-Acetoxypropyl)-3-(trans-3-(carbamoyl(diphenyl)methyl)cyclopentyl)-2-methyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 38, acetic acid 3-bromopropyl ester (0.7 ml) and acetone (8 ml), 0.66 g (yield: 82%) of the title compound was obtained as a colorless amorphous compound.

¹H-NMR (DMSO-d₆, δ PPM): 1.20-1.40 (1H, m), 1.71-2.12 (10H, m), 2.41 (3H, s), 3.80-4.08 (4H, m), 4.10-4.22 (2H, m), 6.87 (1H, brs), 7.19 (1H, brs), 7.22-7.42 (10H, m), 7.78 (1H, brs), 7.96 (1H, brs)
MS (ESI) m/z: 460 [M]⁺

### Example 115

### 1-(3-Acetoxypropyl)-3-(trans-3-(carbamoyl(diphenyl)methyl)cyclopentyl)-2-methyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 39, acetic acid 3-bromopropyl ester (0.7 ml) and acetone (8 ml), 0.67 g (yield: 83%) of the title compound was obtained as a colorless amorphous compound.

¹H-NMR (DMSO-d₆, δ PPM): 1.20-1.40 (1H, m), 1.71-2.12 (10H, m), 2.41 (3H, s), 3.80-4.08 (4H, m), 4.10-4.22 (2H, m), 6.87 (1H, brs), 7.19 (1H, brs), 7.22-7.42 (10H, m), 7.78 (1H, brs), 7.96 (1H, brs)
MS (ESI) m/z: 460 [M]⁺

### Example 116

### 3-((1R,3S)-3-(Carbamoyl(diphenyl)methyclopentyl)-2-methyl-1-(3-methyl-butyl)-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and isoamyl bromide (1.51 g, 0.01 mol), 0.10 g (yield: 19%) of the title compound was obtained as a white crystalline compound in the same manner as that of Example 6.

¹H-NMR (DMSO-d₆, δ PPM): 0.90 (6H, d, J=5.7Hz),1.28-1.57 (6H, m), 1.94-2.06 (2H, m), 2.39-2.42 (1H, m), 2.57 (3H, s), 3.63-3.68 (1H, m), 4.01-4.05 (2H, m), 4.74-4.78 (1H, m), 6.75 (1H, brs), 6.93 (1H, d, J=1.2Hz),7.19 (1H, brs), 7.28-7.35 (10H, m), 7.59 (1H, d, J=1.2Hz)
MS (ESI) m/z: 430 [M]⁺

### Example 117

### 3-(trans-3-(Carbamoyl(diphenyl)methyl)cyclopentyl)-2-methyl-1-phenethyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 30, phenethyl bromide (1 ml) and acetone (8 ml), 0.64 g (yield: 79%) of the title compound was obtained as a pale yellow amorphous compound.

¹H-NMR (DMSO-d₆, δ PPM): 1.20-1.41 (1H, m), 1.63-1.85 (3H, m), 1.90-2.14 (5H, m), 3.02 (2H, t, J=7.0Hz),3.76-3.96 (2H, m), 4.33 (2H, t, J=7.0Hz),6.85 (1H, brs), 7.02-7.40 (16H, m), 7.73 (1H, brs), 7.92 (1H, brs)
MS (ESI) m/z: 464 [M]⁺

### Example 118

### 3-(cis-3-(Carbamoyl(diphenyl)methyl)cyclonentyl)-2-methyl-1-(2-oxo-2-phenylethyl)-3H-imidazol-1-ium bromide

By using 2-(cis-3-(2-methylimidazol-1-yl)cyclopentyl)-2,2-diphenylacetamide (0.54 g, 1.5 mmol) obtained in Reference Example 5, phenacyl bromide (2 g, 10 mmol) and acetone (8 ml), 0.82 g (yield: 98%) of the title compound was obtained as a white powdery compound in the same manner as that of Example 6.

¹H-NMR (DMSO-d₆, δ PPM): 1.24-1.59 (3H, m), 1.92-2.10 (2H, m), 2.41-2.60 (4H, m), 3.58-3.74 (1H, m), 4.78-4.94 (1H, m), 6.01 (2H, s), 6.81 (1H, brs), 7.01 (1H, brs), 7.12-7.40 (11H, m), 7.52 (1H, brs), 7.54-7.64 (2H, m), 7.69 (1H, m), 7.94-8.08 (2H, m)
MS (ESI) m/z: 478 [M]⁺

### Example 119

### 3-(trans-3-(Carbamoyl(diphenyl)methyl)cyclopentyl)-2-methyl-1-(2-oxo-2-phenylethyl)-3H-imidazol-1-ium bromide

By using 2-(trans-3-(2-methylimidazol-1-yl)cyclopentyl)-2,2-diphenylacetamide obtained in Reference Example 30, phenacyl bromide (2 g, 10 mmol) and acetone (8 ml), 0.81 g (yield: 96%) of the title compound was obtained as a white powdery compound in the same manner as that of Example 6.

¹H-NMR (DMSO-d₆, δ PPM): 1.23-1.42 (1H, m), 1.75-2.19 (5H, m), 2.37 (3H, s), 3.84-3.99 (1H, m), 4.04-4.19 (1H, m), 6.04 (2H, s), 6.87 (1H, brs), 7.12-7.48 (11H, m), 7.54-7.70 (3H, m), 7.72-7.80 (1H, m), 7.92-8.10 (3H, m)
MS (ESI) m/z: 478 [M]⁺

### Example 120

### 3-((1R,3S)-3-(Carbamoyl(diphenyl)methyl)cyclopentyl)-1-buten-2-yl-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and crotyl bromide (1.35 g, 0.01 mol), 0.44 g (yield: 89%) of the title compound was obtained as a white crystalline compound in the same manner as that of Example 6.

¹H-NMR (DMSO-d₆, δ PPM): 1.32-1.49 (3H, m), 1.66-1.68 (2H, m), 1.72-1.74 (1H, m), 1.94-2.06 (2H, m), 2.39-2.42 (1H, m), 2.56 (3H, d, J=3.3Hz),3.64-3.68 (1H, m), 4.64 (1H, m), 4.74-4.79 (2H, m), 5.53-5.58 (1H, m), 5.72-5.77 (1H, m), 6.75 (1H, brs), 6.91-6.92 (1H, m), 7.19 (1H, brs), 7.25-7.34 (10H, m), 7.52 (1H, d, J=1.9Hz)
MS (ESI) m/z: 414 [M]⁺

### Example 121

### 3-((1R,3S)-3-(Carbamoyl(diphenyl)methyl)cyclopentyl)-1-butyn-2-yl-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and 1-bromo-2-butyne (1.33 g, 0.01 mol), 0.48 g (yield: 98%) of the title compound was obtained as a brown amorphous compound.

¹H-NMR (DMSO-d₆, δ PPM): 1.29-1.50 (3H, m), 1.84-1.86 (3H, m), 1.94-2.07 (2H, m), 2.39-2.42 (1H, m), 2.60 (3H, s), 3.63-3.68 (1H, m), 4.76-4.80 (1H, m), 4.98-4.99 (2H, m), 6.75 (1H, brs), 6.94 (1H, d, J=2.2Hz),7.19 (1H, brs), 7.26-7.35 (10H, m), 7.61 (1H, d, J=2.2Hz)
MS (ESI) m/z: 412 [M]⁺

### Example 122

### 3-((1R,3S)-3-(Carbamoyl(diphenyl)methyl)cyclopentyl)-1-(3-acetoxypropyl)-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and 3-bromopropyl acetate (1.67 g, 0.01 mol), 0.40 g (yield: 75%) of the title compound was obtained as a pale yellow amorphous compound.

¹H-NMR (DMSO-d₆, δ PPM): 1.27-1.47 (3H, m), 1.91-2.06 (7H, m), 2.37-2.43 (1H, m), 2.58 (3H, s), 3.64-3.69 (1H, m), 3.98-4.01 (2H, m), 4.10-4.15 (2H, m), 4.76-4.80 (1H, m), 6.79 (1H, brs), 6.93-6.94 (1H, m), 7.21 (1H, brs), 7.27-7.35 (10H, m), 7.60-7.61 (1H, m) MS (ESI) m/z: 460 [M]⁺

### Example 123

### 3-((1S,3R)-3-(Carbamoyl(diphenyl)methyl)cyclopentyl)-1-(3-acetoxypropyl)-2-methyl-3H-imidazol-1-ium bromide

By using 2-((1R,3S)-3-(2-methylimidazol-1-yl)cyclopentyl)-2,2-diphenylacetamide (0.36 g, 1.00 mmol) obtained in Reference Example 23 and 3-bromopropyl acetate (1.67 g, 0.01 mol), 0.37 g (yield: 69%) of the title compound was obtained as a white amorphous compound.

¹H-NMR (DMSO-d₆, δ PPM): 1.27-1.47 (3H, m), 1.91-2.06 (7H, m), 2.37-2.43 (1H, m), 2.58 (3H, s), 3.64-3.69 (1H, m), 3.98-4.01 (2H, m), 4.10-4.15 (2H, m), 4.76-4.80 (1H, m), 6.79 (1H, brs), 6.93-6.94 (1H, m), 7.21 (1H, brs), 7.27-7.35 (10H, m), 7.60-7.61 (1H, m) MS (ESI) m/z: 460 [M]⁺

### Example 124

### 3-(trans-3-(Carbamoyl(diphenyl)methyl)cyclopentyl)-2-methyl-1-(3-phenylpropyl)-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 30, phenylpropyl bromide (1.5 ml) and acetone (7 ml), 0.20 g (yield: 24%) of the title compound was obtained as a white powdery compound in the same manner as that of Example 6.

¹H-NMR (DMSO-d₆, δ PPM): 1.18-1.40 (1H, m), 1.63-2.14 (7H, m), 2.37 (3H, s), 2.50-2.68 (2H, m), 3.71-4.16 (4H, m), 6.87 (1H, brs), 6.96-7.44 (16H, m), 7.80 (1H, brs), 7.93 (1H, brs)
MS (ESI) m/z: 478 [M]⁺

### Example 125

### 3-((1R,3S)-3-(Carbamoyl(diphenyl)methyl)cyclopentyl)-1-(4-fluorobenzyl)-2-methyl-3H-imidazol-1-ium bromide

By using 2-((1S,3R)-3-(2-methylimidazol-1-yl)cyclopentyl)-2,2-diphenylacetamide (0.36 g, 1.00 mmol) obtained in Reference Example 22 and 4-fluorobenzyl bromide (1.89 g, 0.01 mol), 0.25 g (yield: 51%) of the title compound was obtained as a white crystalline compound in the same manner as that of Example 6.

¹H-NMR (DMSO-d₆, δ PPM): 1.29-1.48 (3H, m), 1.94-2.07 (2H, m), 2.40-2.45 (1H, m), 2.60 (3H, s), 3.63-3.67 (1H, m), 4.75-4.79 (1H, m), 5.32 (2H, s), 6.74 (1H, brs), 6.96 (1H, brs), 7.18-7.40 (15H, m), 7.60 (1H, m)
MS (ESI) m/z: 468 [M]⁺

### Example 126

### 3-(trans-3-(Carbamoyl(diphenyl)methyl)cyclopentyl)-1-difluoromethyl-2-methyl-3H-imidazol-1-ium bromide

By using 2-(trans-3-(2-methylimidazol-1-yl)cyclopentyl)-2,2-diphenylacetamide (0.54 g, 1.5 mmol) obtained in Reference Example 30, ethyl bromodifluoroacetate (1.5 ml) and tetrahydrofuran (10 ml), 0.35 g (yield: 47%) of the title compound was obtained as pale yellow powdery compound in the same manner as that of Example 6.

¹H-NMR (DMSO-d₆, δ PPM): 1.18-1.40 (1H, m), 1.70-2.08 (5H, m), 2.59 (3H, s), 3.83-4.00 (1H, m), 4.03-4.10 (1H, m), 6.88 (1H, brs), 7.06-7.43 (11H, m), 8.11 (1H, t, J=57.3Hz),8.21 (1H, s), 8.25 (1H, s)
MS (ESI) m/z: 410 [M]⁺

### Example 127

### 3-(trans-3-(Carbamoyl(diphenyl)methyl)cyclopentyl)-1-(2-oxonropyl)-3H-imidazol-1-ium bromide

By using 2-(trans-3-(imidazol-1-yl)cyclopentyl)-2,2-diphenylacetamide (0.52 g, 1.5 mmol) obtained in Reference Example 34, bromoacetone (1 ml) and tetrahydrofuran (6 ml), 0.66 g (yield: 91%) of the title compound was obtained as a white powdery compound in the same manner as that of Example 6.

¹H-NMR (DMSO-d₆, δ PPM): 1.22-1.40 (1H.m),1.64-2.16 (5H, m), 2.24 (3H, s), 3.74-3.92 (1H, m), 4.12-4.32 (1H, m), 5.31 (2H, s), 6.80 (1H, brs), 7.12-7.42 (11H, m), 7.63 (1H, s), 7.91 (1H, s), 9.11 (1H, s)
MS (ESI) m/z: 402 [M]⁺

### Example 128

### 1-Benzyl-3-(trans-3-(carbamoyl(diphenyl)methyl)cyclopentyl)-5-methyl-3H-imidazol-1-ium bromide

By using 2-(trans-3-(4-methylimidazol-1-yl)cyclopentyl)-2,2-diphenylacetamide (0.54 g, 1.5 mmol) obtained in Reference Example 32, benzyl bromide (1 ml) and tetrahydrofuran (7 ml), 0.66 g (yield: 83%) of the title compound was obtained as a white powdery compound in the same manner as that of Example 6.

¹H-NMR (DMSO-d₆, δ PPM): 1.21-1.40 (1H, m), 1.64-2.22 (8H, m), 3.71-3.90 (1H, m), 4.06-4.20 (1H, m), 5.43 (2H, s), 6.80 (1H, brs), 7.12-7.43 (16H, m), 7.72 (1H, s), 9.36 (1H, s)
MS (ESI) m/z: 450 [M]⁺

### Example 129

### 3-(trans-3-(Carbamoyl(diphenyl)methyl)cyclopentyl)-5-methyl-1-(2-oxopropyl)-3H-imidazol-1-ium bromide

By using 2-(trans-3-(4-methylimidazol-1-yl)cyclopentyl)-2,2-diphenylacetamide (0.54 g, 1.5 mmol) obtained in Reference Example 32, bromoacetone (1 ml) and tetrahydrofuran (7 ml), 0.66 g (yield: 89%) of the title compound was obtained as a white powdery compound in the same manner as that of Example 6.

¹H-NMR (DMSO-d₆, δ PPM): 1.21-1.39 (1H, m), 1.56-2.17 (8H, m), 2.29 (3H, s), 3.75-3.92 (1H, m), 4.09-4.26 (1H, m), 5.35 (2H, s), 6.82 (1H, brs), 7.08-7.40 (11H, m), 7.72 (1H, s), 9.06 (1H, s)
MS (ESI) m/z: 416 [M]⁺

### Example 130

### 1-Benzyl-3-(trans-3-(carbamoyl(diphenyl)methyl)cyclopentyl)-3H-imidazol-1-ium bromide

By using 2-(trans-3-(imidazol-1-yl)cyclopentyl)-2,2-diphenylacetamide (0.52 g, 1.5 mmol) obtained in Reference Example 34, benzyl bromide (1 ml) and tetrahydrofuran (6 ml), 0.52 g (yield: 68%) of the title compound was obtained as a pale yellow amorphous compound.

¹H-NMR (DMSO-d₆, δ PPM): 1.22-1.42 (1H, m), 1.68-2.28 (5H, m), 3.74-3.90 (1H, m), 4.10-4.28 (1H, m), 5.41 (2H, s), 6.78 (1H, brs), 7.12-7.52 (16H, m), 7.84 (1H, s), 7.91 (1H, s), 9.43 (1H, s)
MS (ESI) m/z: 436 [M]⁺

### Example 131

### 1-(3-Acetoxypropyl)-3-(trans-3-(carbamoyl(diphenyl)methyl)cyclopentyl)-5-methyl-3H-imidazol-1-ium bromide

By using 2-(trans-3-(4-methylimidazol-1-yl)cyclopentyl)-2,2-diphenylacetamide (0.54 g, 1.5 mmol) obtained in Reference Example 32, acetic acid 3-bromopropyl ester (1.2 ml) and tetrahydrofuran (7 ml), 0.28 g (yield: 34%) of the title compound was obtained as a white powdery compound in the same manner as that of Example 6.

¹H-NMR (DMSO-d₆, δ PPM): 1.20-1.42 (1H, m), 1.60-2.16 (10H, m), 2.29 (3H, s), 3.72-3.96 (1H, m), 3.96-4.28 (5H, m), 6.77 (1H, brs), 7.08-7.42 (11H, m), 7.65 (1H, s), 9.16(1H,s)
MS (ESI) m/z: 460 [M]⁺

### Example 132

### 3-(trans-3-(Carbamoyl(diphenyl)methyl)cyclopentyl)-1-(2-methoxyethyl)-3H-imidazol-1-ium para-toluenesulfonate

By using 2-(trans-3-(imidazol-1-yl)cyclopentyl)-2,2-diphenylacetamide (0.52 g, 1.5 mmol) obtained in Reference Example 34, para-toluenesulfonic acid 2-methoxyethyl ester (1 ml) and tetrahydrofuran (7 ml), 0.04 g (yield: 4%) of the title compound was obtained as a colorless amorphous compound.

¹H-NMR (DMSO-d₆, δ PPM): 1.18-1.40 (1H, m), 1.64-2.14 (5H, m), 2.28 (3H, s), 3.26 (3H, s), 3.69 (2H, t, J=4.7Hz),3.71-3.88 (1H, m), 4.08-4.22 (1H, m), 4.32 (2H, t, J=4.7Hz),6.76 (1H, brs), 7.10 (2H, d, J=7.8Hz),7.19 (1H, brs), 7.20-7.37 (10H, m), 7.47 (2H, t, J=7.8Hz),7.77 (1H, s), 7.87 (1H, s), 9.18 (1H, s)
MS (ESI) m/z: 404 [M]⁺

### Example 133

### 3-((1R,3S)-3-(Carbamoyl(diphenyl)methyl)cyclopentyl)-1-(2-methoxyethyl)-2-methyl-3H-imidazol-1-ium mesylate

By using 2-((1S,3R)-3-(2-methylimidazol-1-yl)cyclopentyl)-2,2-diphenylacetamide (0.36 g, 1.00 mmol) obtained in Reference Example 22, ethyl methanesulfonate methyl ether (1.54 g, 0.01 mol), 0.42 g (yield: 83%) of the title compound was obtained as a white crystalline compound in the same manner as that of Example 6.

¹H-NMR (DMSO-d₆, δ PPM): 1.26-1.49 (3H, s), 1.95-2.07 (2H, m), 2.32 (3H, s), 2.41-2.44 (1H, m), 2.59 (3H, s), 3.23 (3H, s), 3.60-3.62 (2H, m), 3.66-3.71 (1H, m), 4.24-4.27 (2H, m), 4.79-4.83 (1H, m), 6.82 (1H, brs), 6.92 (1H, m), 7.21 (1H, brs), 7.28-7.35 (10H, m), 7.55 (1H, m)
MS (ESI) m/z: 418 [M]⁺

### Example 134

### 1-(3-Acetoxypropyl)-3-(trans-3-(carbamoyl(diphenyl)methyl)cyclopentyl)-3H-imidazol-1-ium bromide

By using 2-(trans-3-(imidazol-1-yl)cyclopentyl)-2,2-diphenylacetamide (0.52 g, 1.5 mmol) obtained in Reference Example 34, acetic acid 3-bromopropyl ester (0.8 ml) and tetrahydrofuran (7 ml), 0.36 g (yield: 45%) of the title compound was obtained as a colorless amorphous compound.

¹H-NMR (DMSO-d₆, δ PPM): 1.22-1.44 (1H, m), 1.63-2.22 (10H, m), 3.71-3.90 (1H, m), 3.92-4.30 (5H, m), 6.78 (1H, brs), 7.10-7.42 (11H, m), 7.85 (1H, s), 7.89 (1H, s), 9.29 (1H, s)
MS (ESI) m/z: 446 [M]⁺

### Example 135

### 3-((1S,3R)-3-(Carbamoyl(diphenyl)methyl)cyclopentyl)-1-(2-methoxyethyl)-2-methyl-3H-imidazol-1-ium methanesulfonate

By using 2-((1R,3S)-3-(2-methylimidazol-1-yl)cyclopentyl)-2,2-diphenylacetamide (6.67 g, 18.5 mmol) obtained in Reference Example 23, methanesulfonic acid 2-methoxyethyl ester (2.85 g, 18.5 mmol) and 1,4-dioxane (7 ml), 4.66 g (yield: 49%) of the title compound was obtained as a white powdery compound in the same manner as that of Example 6.

¹H-NMR (DMSO-d₆, δ PPM): 1.26-1.49 (3H, s), 1.95-2.07 (2H, m), 2.32 (3H, s), 2.41-2.44 (1H, m), 2.59 (3H, s), 3.23 (3H, s), 3.60-3.62 (2H, m), 3.66-3.71 (1H, m), 4.24-4.27 (2H, m), 4.79-4.83 (1H, m), 6.82 (1H, brs), 6.92 (1H, m), 7.21 (1H, brs), 7.28-7.35 (10H, m), 7.55 (1H, m)
MS (ESI) m/z: 418 [M]⁺

### Example 136

### 3-(cis-3-(Carbamoyl(diphenyl)methyl)cyclopentyl)-1-(2-methoxyethyl)-2-methyl-3H-imidazol-1-ium methanesulfonate

By using 2-(cis-3-(2-methylimidazol-1-yl)cyclopentyl)-2,2-diphenylacetamide (2.0 g, 5.6 mmol) obtained in Reference Example 5, methanesulfonic acid 2-methoxyethyl ester (0.86 g, 5.6 mmol) and 1,4-dioxane (4 ml), 1.5 g (yield: 52%) of the title compound was obtained as a white powdery compound in the same manner as that of Example 6.

¹H-NMR (DMSO-d₆, δ PPM): 1.26-1.49 (3H, s), 1.95-2.07 (2H, m), 2.32 (3H, s), 2.41-2.44 (1H, m), 2.59 (3H, s), 3.23 (3H, s), 3.60-3.62 (2H, m), 3.66-3.71 (1H, m), 4.24-4.27 (2H, m), 4.79-4.83 (1H, m), 6.82 (1H, brs), 6.92 (1H, m), 7.21 (1H, brs), 7.28-7.35 (10H, m), 7.55 (1H, m)
MS (ESI) m/z: 418 [M]⁺

### Example 137

### 1-Benzyl-3-(cis-3-(carbamoyl(diphenyl)methyl)cyclopentyl)-2-methyl-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and benzyl bromide (0.2 g, 1.2 mmol), 0.06 g (yield: 14%) of the title compound was obtained as a colorless amorphous compound.

¹H-NMR (DMSO-d₆, δ PPM): 0.90-1.04 (1H, m), 1.24-1.39 (2H, m), 1.62-1.73 (1H, m), 1.80-1.94 (1H, m), 2.04-2.15 (1H, m), 2.13 (3H, s), 3.14-3.39 (2H, m), 3.46-3.64 (3H, m), 4.26-4.37 (1H, m), 4.63 (2H, s), 6.75 (1H, brs), 7.13 (1H, brs), 7.19-7.43 (15H, m)
MS (ESI) m/z: 452 [M]⁺

### Example 138

### 3-(cis-3-(Carbamoyl(diphenyl)methyl)cyclopentyl)-1-(4-fluorobenzyl)-2-methyl-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and 4-fluorobenzyl bromide (0.2 g, 1.1 mmol), 0.11 g (yield: 24%) of the title compound was obtained as a colorless amorphous compound.

¹H-NMR (DMSO-d₆, δ PPM): 0.88-1.05 (1H, m), 1.22-1.40 (2H, m), 1.62-1.74 (1H, m), 1.79-1.94 (1H, m), 2.04-2.16 (1H, m), 2.38 (3H, s), 3.14-3.38 (2H, m), 3.47-3.68 (3H, m), 4.25-4.38 (1H, m), 4.62 (2H, s), 6.75 (1H, brs), 7.13 (1H, brs), 7.17-7.42 (14H, m)
MS (ESI) m/z: 470 [M]⁺

### Example 139

### 3-(cis-3-(Carbamoyl(diphenyl)methyl)cyclopentyl)-2-methyl-1-(oxopropyl)-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and bromoacetone (0.2 g, 1.4 mmol), 0.36 g (yield: 88%) of the title compound was obtained as a white crystalline compound in the same manner as that of Example 6.

¹H-NMR (DMSO-d₆, δ PPM): 0.90-1.05 (1H, m), 1.22-1.40 (2H, m), 1.63-1.75 (1H, m), 1.80-1.93 (1H, m), 2.03-2.20 (7H, m), 3.18-3.42 (2H, m), 3.51-3.64 (3H, m), 4.25-4.38 (1H, m), 4.54 (2H, s), 6.76 (1H, brs), 7.13 (1H, brs), 7.22-7.39 (10H, m)
MS (ESI) m/z: 418 [M]⁺

### Example 140

### 3-((1R,3S)-3-(Carbamoyl(diphenyl)methyl)cyclopentyl)-2-methyl-1-(2-oxobutyl)-3H-imidazol-1-ium bromide

By using 2-((1S,3R)-3-(2-methylimidazol-1-yl)cyclopentyl)-2,2-diphenylacetamide (1.5 g, 4.2 mmol) obtained in Reference Example 22 and 1-bromo-2-butanone (1.0 g, 6.6 mmol), 1.29 g (yield: 61%) of the title compound was obtained as a colorless amorphous compound.

¹H-NMR (DMSO-d₆, δ PPM): 0.98 (3H, m), 1.22-1.55 (3H, m), 1.91-2.22 (2H, m), 2.38-2.65 (6H, m), 3.61-3.72 (1H, m), 4.74-4.86 (1H, m), 5.27 (2H, s), 6.76 (1H, brs), 6.95 (1H, brs), 7.20 (1H, brs), 7.24-7.38 (10H, m), 7.41 (1H, brs)
MS (ESI) m/z: 430 [M]⁺

### Example 141

### 3-(cis-3-(Carbamoyl(diphenyl)methyl)cyclopentyl)-2-methyl-1-pentyl-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and 1-bromopentane (0.2 g, 1.3 mmol), 0.16 g (yield: 31%) of the title compound was obtained as a pale yellow amorphous compound.

¹H-NMR (DMSO-d₆, δ PPM): 0.86 (3H, m), 0.89-1.00 (1H, m), 1.14-1.38 (6H, m), 1.42-1.55 (2H, m), 1.57-1.70 (1H, m), 1.78-1.92 (1H, m), 2.01-2.10 (1H, m), 2.24 (3H, s), 3.10-3.38 (4H, m), 3.51-3.58 (1H, m), 3.65 (2H, m), 4.18-4.30 (1H, m), 6.74 (1H, brs), 7.12 (1H, brs), 7.18-7.38 (10H, m)
MS (ESI) m/z: 432 [M]⁺

### Example 142

### 3-(cis-3-(Carbamoyl(diphenyl)methyl)cyclopentyl)-2-methyl-1-octyl-4,5-dihydro-3H-imidazol-1-ium iodide

By using 2-(cis-3-(2-methyl-4,5-dihydroimidazol-1-yl)cyclopentyl)-2,2-diphenylacetamide (0.3 g, 0.83 mmol) obtained in Reference Example 13 and 1-iodooctane (0.2 g, 0.83 mmol), 0.16 g (yield: 31%) of the title compound was obtained as a colorless amorphous compound.

¹H-NMR (DMSO-d₆, δ PPM): 0.85 (3H, m), 0.88-1.00 (1H, m), 1.16-1.38 (12H, m), 1.40-1.54 (2H, m), 1.57-1.70 (1H, m), 1.78-1.90 (1H, m), 1.99-2.10 (1H, m), 2.23 (3H, s), 3.12-3.38 (4H, m), 3.50-3.68 (3H, m), 4.18-4.30 (1H, m), 6.73 (1H, brs), 7.11 (1H, brs), 7.21-7.38 (10H, m)
MS (ESI) m/z: 474 [M]⁺

### Example 143

### 3-(cis-3-(Carbamoyl(dipheyl)methyl)cyclopentyl)-2-methyl-1-(2-oxobutyl)-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and 1-bromo-2-butanone (0.2 g, 1.3 mmol), 0.13 g (yield: 30%) of the title compound was obtained as a pale yellow amorphous compound.

¹H-NMR (DMSO-d₆, δ PPM): 0.88-1.04 (4H, m), 1.23-1.39 (2H, m), 1.62-1.75 (1H, m), 1.79-1.95 (1H, m), 2.03-2.12 (1H, m), 2.14 (3H, s), 2.40-2.52 (2H, m), 3.18-3.42 (2H, m), 3.51-3.62 (3H, m), 4.24-4.35 (1H, m), 4.52 (2H, s), 6.75 (1H, brs), 7.13 (1H, brs), 7.19-7.40 (10H, m)
MS (ESI) m/z: 432 [M]⁺

### Example 144

### 3-(cis-3-(Carbamoyl(diphenyl)methyl)cyclopentyl)-1-(2-methoxyethyl)-2-methyl-4,5-dihydro-3H-imidazol-1-ium para-toluenesulfonate

By using 2-(cis-3-(2-methyl-4,5-dihydroimidazol-1-yl)cyclopentyl)-2,2-diphenylacetamide (0.3 g, 0.83 mmol) obtained in Reference Example 13 and para-toluenesulfonic acid 2-methoxyethyl ester (0.2 g), 0.17 g (yield: 42%) of the title compound was obtained as a colorless amorphous compound.

¹H-NMR (DMSO-d₆, δ PPM): 0.88-1.02 (1H, m), 1.22-1.40 (2H, m), 1.58-1.70 (1H, m), 1.74-1.96 (1H, m), 2.00-2.11 (1H, m), 2.23 (3H, s), 2.29 (3H, s), 3.13-3.39 (6H, m), 3.42-3.59 (4H, m), 3.69 (2H, m), 4.19-4.31 (1H, m), 6.72 (1H, brs), 7.05-7.15 (2H, m), 7.18-7.38 (11H, m), 7.42-7.50 (2H, m)
MS (ESI) m/z: 420 [M]⁺

### Example 145

### 3-(cis-3-(Carbamoyl(diphenyl)methyl)cyclopentyl)-2-methyl-1-(2-methylbenzyl)-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and 2-methylbenzyl bromide (0.2 g, 1.1 mmol), 0.25 g (yield: 54%) of the title compound was obtained as a colorless amorphous compound.

¹H-NMR (DMSO-d₆, δ PPM): 0.95-1.10 (1H, m), 1.28-1.48 (2H, m), 1.68-1.80 (1H, m), 1.85-1.99 (1H, m), 2.10-2.24 (1H, m), 2.31 (3H, s), 2.40 (3H, s), 3.23-3.72 (5H, m), 4.32-4.43 (1H, m), 4.69 (2H, s), 6.82 (1H, brs), 7.13-7.46 (15H, m)
MS (ESI) m/z: 466 [M]⁺

### Example 146

### 3-(cis-3-(Carbamoyl(diphenyl)methyl)cyclopentyl)-2-methyl-1-naphthalen-1-ylmethyl-4,5-dihydro-3H-imidazol-1-ium chloride

By using 2-(cis-3-(2-methyl-4,5-dihydroimidazol-1-yl)cyclopentyl)-2,2-diphenylacetamide (0.3 g, 0.83 mmol) obtained in Reference Example 13 and 1-chloromethylnaphthalene (0.2 g, 1.1 mmol), 0.43 g (yield: 97%) of the title compound was obtained as a colorless amorphous compound.

¹H-NMR (DMSO-d₆, δ PPM): 0.95-1.14 (1H, m), 1.27-1.50 (2H, m), 1.65-2.00 (2H, m), 2.08-2.26 (1H, m), 2.49 (3H, s), 3.21-3.72 (5H, m), 4.35-4.46 (1H, m), 5.21 (2H, s), 6.83 (1H, brs), 7.20 (1H, brs), 7.25-7.48 (13H, m), 7.53-7.70 (2H, m), 7.95-8.08 (2H, m)
MS (ESI) m/z: 502 [M]⁺

### Example 147

### 3-(cis-3-(Carbamoyl(diphenyl)methyl)cyclopentyl)-2-methyl-1-(oxiranylmethyl)-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and epibromohydrin (0.2 g, 1.5 mmol), 0.11 g (yield: 27%) of the title compound was obtained as a colorless amorphous compound.

¹H-NMR (DMSO-d₆, δ PPM): 0.88-1.03 (1H, m), 1.23-1.40 (2H, m), 1.60-1.72 (1H, m), 1.78-1.92 (1H, m), 1.19-2.10 (1H, m), 2.26 (3H, s), 2.62-2.67 (1H, m), 2.74-2.82 (1H, m), 3.14-3.40 (4H, m), 3.50-3.62 (1H, m), 3.72 (2H, m), 3.80-3.90 (1H, m), 4.22-4.35 (1H, m), 6.75 (1H, brs), 7.13 (1H, brs), 7.21-7.40 (10H, m)
MS (ESI) m/z: 418 [M]⁺

### Example 148

### 3-((1R,3S)-3-(Carbamoyl(diphenyl)methyl)cyclopentyl)-1-(3-phenylpropyl)-2-methyl-3H-imidazol-1-ium bromide

By using 2-((1S,3R)-3-(2-methylimidazol-1-yl)cyclopentyl)-2,2-diphenylacetamide (1.00 g, 2.78 mmol) obtained in Reference Example 22 and 1-bromo-3-phenylpropane (0.665 g, 3.34 mmol), 0.964 g (yield: 62%) of the title compound was obtained as white powder in the same manner as that of Example 6.

¹H-NMR (DMSO-d₆, δ PPM): 1.27-1.46 (3H, m), 1.94-2.06 (4H, m), 2.33-2.41 (1H, m), 2.53 (3H, s), 2.57-2.61 (2H, m), 3.62-3.67 (1H, m), 4.05-4.08 (2H, m), 4.71-4.75 (1H, m), 6.74 (1H, brs), 6.90 (1H, m), 7.14-7.35 (16H, m), 7.59 (1H, m)
MS (ESI) m/z: 478 [M]⁺

### Example 149

### 3-((1S,3R)-3-(Carbamoyl(diphenyl)methyl)cyclopentyl)-1-(3-phenylpropyl)-2-methyl-3H-imidazol-1-ium bromide

By using 2-((1R,3S)-3-(2-methylimidazol-1-yl)cyclopentyl)-2,2-diphenylacetamide (0.50 g, 1.39 mmol) obtained in Reference Example 23 and 1-bromo-3-phenylpropane (0.33 g, 1.66 mmol), 0.615 g (yield: 79%) of the title compound was obtained as white powder in the same manner as that of Example 6.

¹H-NMR (DMSO-d₆, δ PPM): 1.26-1.48 (3H, m), 1.91-2.06 (4H, m), 2.32-2.41 (1H, m), 2.53 (3H, s), 2.58-2.61 (2H, m), 3.61-3.69 (1H, m), 4.05-4.09 (2H, m), 4.69-4.77 (1H, m), 6.74 (1H, brs), 6.90 (1H, m), 7.14-7.35 (16H, m), 7.59 (1H, m)
MS (ESI) m/z: 478 [M]⁺

### Example 150

### 3-((1R,3S)-3-(Carbamoyl(diphenyl)methyl)cyclopentyl)-1-(2-(2-methoxyethoxy)ethyl)-2-methyl-3H-imidazol-1-ium bromide

2-((1S,3R)-3-(2-Methylimidazol-1-yl)cyclopentyl)-2,2-diphenylacetamide (0.30 g, 0.83 mmol) obtained in Reference Example 22 and 1-bromo-2-(2-methoxyethoxy)ethane (0.183 g, 1.00 mmol) were reacted, and then the product was purified by silica gel column chromatography to obtain 0.033 g (yield: 7%) of the title compound as white powder.

¹H-NMR (DMSO-d₆, δ PPM): 1.23-1.48 (3H, m), 1.94-2.08 (2H, m), 2.36-2.43 (1H, m), 2.57 (3H, s), 3.15 (3H, s), 3.31-3.36 (2H, m), 3.47-3.49 (2H, m), 3.64-3.69 (3H, m), 4.22-4.24 (2H, m), 4.74-4.80 (1H, m), 6.78 (1H, brs), 6.93-6.94 (1H, m), 7.20 (1H, brs), 7.27-7.35 (10H, m), 7.52-7.53 (1H, m)
MS (ESI) m/z: 462 [M]⁺

### Example 151

### 3-((1S,3R)-3-(Carbamoyl(diphenyl)methyl)cyclopentyl)-1,2-dimethyl-4,5-dihydro-3H-imidazol-1-ium para-toluenesulfonate

By using 2-((1R,3S)-3-(2-methyl-4,5-dihydroimidazol-1-yl)cyclopentyl)-2,2-diphenylacetamide (0.36 g, 1.0 mmol) obtained in Reference Example 27, methyl para-toluenesulfonate (1.5 ml) and acetone (4 ml), 0.12 g (yield: 22%) of the title compound was obtained as a white crystalline compound in the same manner as that of Example 6.

¹H-NMR (DMSO-d₆, δ PPM): 0.88-1.01 (1H, m), 1.23-1.38 (2H, m), 1.58-1.70 (1H, m), 1.78-1.93 (1H, m), 1.99-2.12 (1H, m), 2.20 (3H, s), 2.29 (3H, s), 2.97 (3H, s), 3.08-3.39 (2H, m), 3.50-3.68 (3H, m), 4.19-4.30 (1H, m), 6.75 (1H, brs), 7.05-7.16 (3H, m), 7.22-7.40 (10H, m), 7.47 (2H, d, J=7.9Hz)
MS (ESI) m/z: 376 [M]⁺

### Example 152

### 3-(trans-3-(Carbamoyl(diphenyl)methyl)cyclopentyl)-1,2-dimethyl-4,5-dihydro-3H-imidazol-1-ium para-toluenesulfonate

By using 2-(trans-3-(2-methyl-4,5-dihydroimidazol-1-yl)cyclopentyl)-2,2-diphenylacetamide (1.0 g, 2.8 mmol) obtained in Reference Example 42, methyl para-toluenesulfonate (4.2 ml) and acetone (11 ml), 0.45 g (yield: 30%) of the title compound was obtained as a white powdery compound in the same manner as that of Example 6.

¹H-NMR (DMSO-d₆, δ PPM): 1.14-1.27 (1H, m), 1.45-1.68 (3H, m), 1.86-2.04 (5H, m), 2.29 (3H, s), 2.99 (3H, s), 3.27-3.45 (1H, m), 3.58-3.93 (5H, m), 6.81 (1H, brs), 7.05-7.19 (3H, m), 7.22-7.40 (10H, m), 7.46 (2H, d, J=8.0Hz)
MS (ESI) m/z: 376 [M]⁺

### Example 153

### 3-(trans-3-(Carbamoyl(diphenyl)methyl)cyclopentyl)-1,2-dimethyl-4,5-dihydro-3H-imidazol-1-ium para-toluenesulfonate

By using 2-(trans-3-(2-methyl-4,5-dihydroimidazol-1-yl)cyclopentyl)-2,2-diphenylacetamide (1.4 g, 3.8 mmol) obtained in Reference Example 43, methyl para-toluenesulfonate (5.7 ml) and acetone (6 ml), 0.29 g (yield: 14%) of the title compound was obtained as a white powdery compound in the same manner as that of Example 6.

¹H-NMR (DMSO-d₆, δ PPM): 1.14-1.27 (1H, m), 1.45-1.68 (3H, m), 1.86-2.04 (5H, m), 2.29 (3H, s), 2.99 (3H, s), 3.27-3.45 (1H, m), 3.58-3.93 (5H, m), 6.81 (1H, brs), 7.05-7.19 (3H, m), 7.22-7.40 (10H, m), 7.46 (2H, d, J=8.0Hz)
MS (ESI) m/z: 376 [M]⁺

### Example 154

### 3-((1R,3S)-3-(Cyano(diphenyl)methyl)cyclopentyl)-1,2-dimethyl-1-4,5-dihydro-3H-imidazol-1-ium para-toluenesulfonate

By using ((1S,3R)-3-(2-methyl-4,5-dihydroimidazol-1-yl)cyclopentyl)diphenylacetonitrile (0.5 g, 1.5 mmol) obtained in Reference Example 24 and methyl para-toluenesulfonate (0.3 g, 1.6 mmol), 0.64 g (yield: 83%) of the title compound was obtained as a white crystalline compound in the same manner as that of Example 6.

¹H-NMR (DMSO-d₆, δ PPM): 1.46-1.64 (2H, m), 1.71-1.98 (4H, m), 2.22 (3H, s), 2.28 (3H, s), 3.02 (3H, s), 3.49-3.60 (1H, m), 3.68-3.90 (4H, m), 4.32-4.45 (1H, m), 7.11 (2H, d, J=7.9Hz),7.25-7.58 (12H, m)
MS (ESI) m/z: 358 [M]⁺

### Example 155

### 3-(cis-3-(Carbamoyl(diphenyl)methyl)cyclopentyl)-2-methyl-1-(3-phenoxypropyl)-4,5-dihydro-3H-imidazol-1-ium bromide

By using 2-(cis-3-(2-methyl-4,5-dihydroimidazol-1-yl)cyclopentyl)-2,2-diphenylacetamide (0.3 g, 0.83 mmol) obtained in Reference Example 13 and phenoxypropyl bromide (0.2 g, 0.93 mmol), 0.27 g (yield: 57%) of the title compound was obtained as a white crystalline compound in the same manner as that of Example 6.

¹H-NMR (DMSO-d₆, δ PPM): 1.86-1.98 (1H, m), 1.20-1.39 (2H, m), 1.55-1.68 (1H, m), 1.78-1.90 (1H, m), 1.91-2.10 (3H, m), 2.20 (3H, s), 3.12-3.36 (2H, m), 3.46-3.60 (3H, m), 3.68 (2H, m), 3.99 (2H, m), 4.16-4.28 (1H, m), 6.78 (1H, brs), 6.86-6.95 (3H, m), 7.15 (1H, brs), 7.23-7.39 (12H, m)
MS (ESI) m/z: 496 [M]⁺

### Example 156

### 3-((1R,3S)-3-(Carbamoyl(diphenyl)methyl)cyclopentyl)-1-(3-phenyloxypropyl)-2-methyl-3H-imidazol-1-ium bromide

By using 2-((1S,3R)-3-(2-methylimidazol-1-yl)cyclopentyl)-2,2-diphenylacetamide (0.20 g, 0.56 mmol) obtained in Reference Example 22 and 3-phenoxypropyl bromide (0.144 g, 0.67 mmol), 0.203 g (yield: 63%) of the title compound was obtained as white powder.

¹H-NMR (DMSO-d₆, δ PPM): 1.31-1.47 (3H, m), 1.90-2.06 (2H, m), 2.13-2.19 (2H, m), 2.35-2.42 (1H, m), 2.55 (3H, s), 3.61-3.70 (1H, m), 3.98 (2H, t, J=5.8Hz),4.23 (2H, t, J=6.8Hz),4.71-4.79 (1H, m), 6.79 (1H, brs), 6.83-6.85 (2H, m), 6.91-6.94 (2H, m), 7.13-7.35 (13H, m), 7.61 (1H, m)
MS (ESI) m/z: 494 [M]⁺

### Example 157

### 3-((1R,3S)-3-(Carbamoyl(diphenyl)methyl)cyclopentyl)-1-(2-phenyloxyethyl)-2-methyl-3H-imidazol-1-ium bromide

By using 2-((1S,3R)-3-(2-methylimidazol-1-yl)cyclopentyl)-2,2-diphenylacetamide (0.20 g, 0.56 mmol) obtained in Reference Example 22 and 2-phenoxyethyl bromide (0.135 g, 0.67 mmol), 0.188 g (yield: 60%) of the title compound was obtained as white powder in the same manner as that of Example 6.

¹H-NMR (DMSO-d₆, δ PPM): 1.24-1.47 (3H, m), 1.90-2.07 (2H, m), 2.35-2.42 (1H, m), 2.64 (3H, s), 3.62-3.70 (1H, m), 4.27 (2H, t, J=4.9Hz),4.48-4.50 (2H, m), 4.74-4.82 (1H, m), 6.78 (1H, brs), 6.87-6.89 (2H, m), 6.92-6.95 (2H, m), 7.13-7.35 (13H, m), 7.62 (1H, m)
MS (ESI) m/z: 480 [M]⁺

### Example 158

### 3-((1S,3R)-3-(Carbamoyl(diphenyl)methyl)cyclopentyl)-1-(3-phenyloxypropyl)-2-methyl-3H-imidazol-1-ium bromide

By using 2-((1R,3S)-3-(2-methylimidazol-1-yl)cyclopentyl)-2,2-diphenylacetamide (0.20 g, 0.56 mmol) obtained in Reference Example 23 and 3-phenoxypropyl bromide (0.144 g, 0.67 mmol), 0.217 g (yield: 67%) of the title compound was obtained as white powder in the same manner as that of Example 6.

¹H-NMR (DMSO-d₆, δ PPM): 1.25-1.47 (3H, m), 1.90-2.06 (2H, m), 2.13-2.19 (2H, m), 2.35-2.42 (1H, m), 2.55 (3H, s), 3.61-3.70 (1H, m), 3.98 (2H, t, J=5.8Hz),4.23 (2H, t, J=6.8Hz),4.72-4.79 (1H, m), 6.79 (1H, brs), 6.83-6.85 (2H, m), 6.91-6.94 (2H, m), 7.13-7.35 (13H, m), 7.61 (1H, m)
MS (ESI) m/z: 494 [M]⁺

### Example 159

### 3-((1S.3R)-3-(Carbamoyl(diphenyl)methyl)cyclopentyl)-1-(2-phenyloxyethyl)-2-methyl-3H-imidazol-1-ium bromide

By using 2-((1R,3S)-3-(2-methylimidazol-1-yl)cyclopentyl)-2,2-diphenylacetamide (0.20 g, 0.56 mmol) obtained in Reference Example 23 and 2-phenoxyethyl bromide (0.135 g, 0.67 mmol), 0.241 g (yield: 77%) of the title compound was obtained as white powder in the same manner as that of Example 6.

¹H-NMR (DMSO-d₆, δ PPM): 1.24-1.47 (3H, m), 1.90-2.05 (2H, m), 2.35-2.42 (1H, m), 2.64 (3H, s), 3.62-3.68 (1H, m), 4.27 (2H, t, J=4.8Hz),4.48-4.50 (2H, m), 4.74-4.80 (1H, m), 6.78 (1H, brs), 6.87-6.89 (2H, m), 6.92-6.95 (2H, m), 7.13-7.35 (13H, m), 7.62 (1H, m)
MS (ESI) m/z: 480 [M]⁺

The structures of the compounds obtained in the reference examples and examples are shown below.

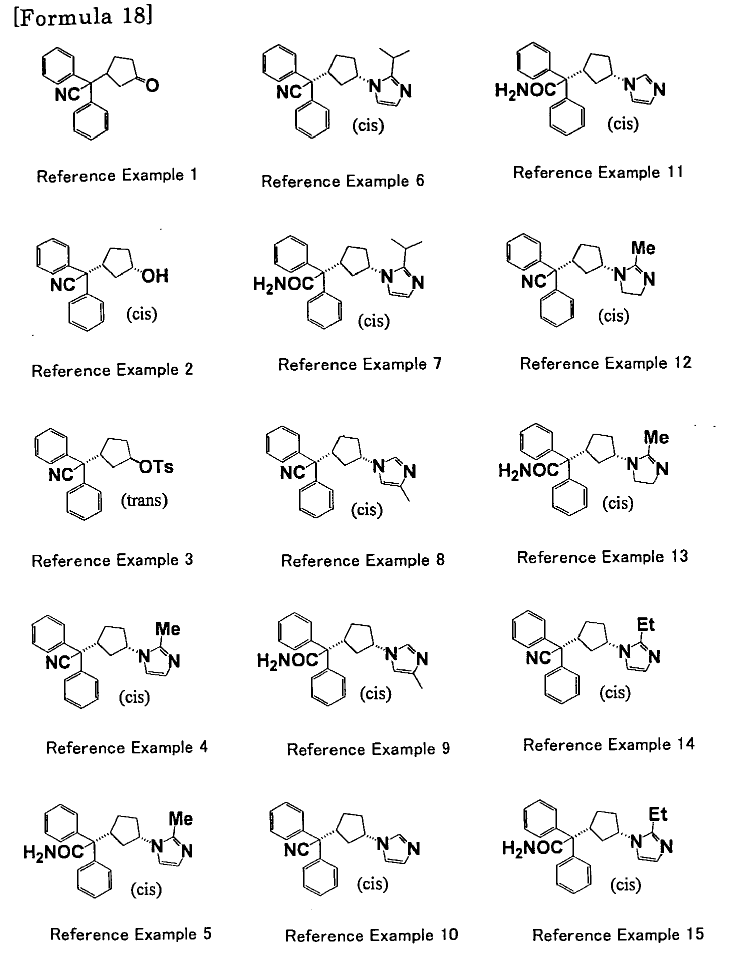

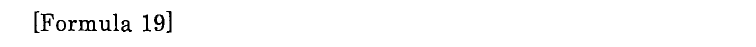

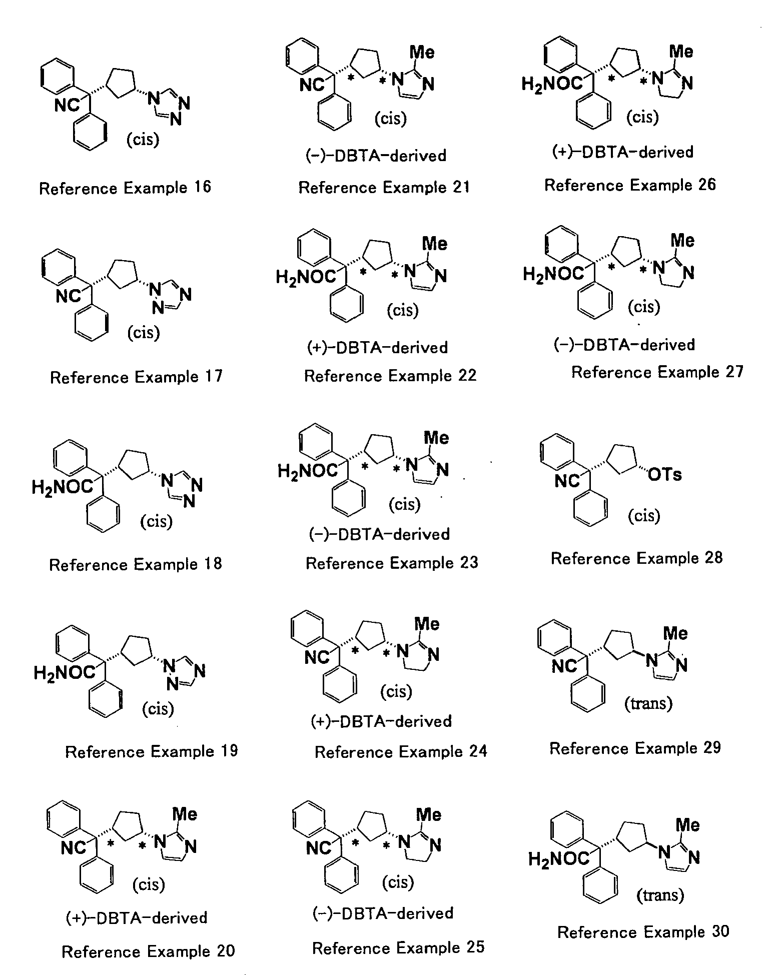

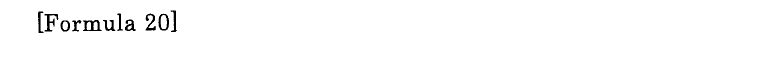

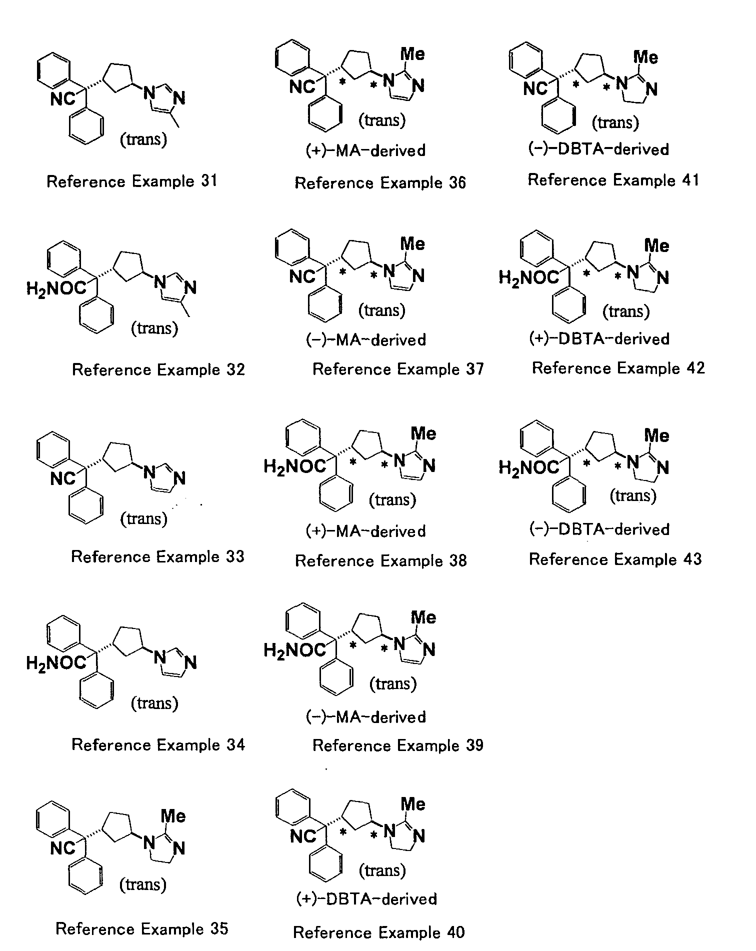

The abbreviations used in the tables of the compounds mentioned below have the following meanings.
Ac: acetyl, Et: ethyl, iPr: isopropyl, Me: methyl, Ph: phenyl, Cl-: chloride ion, Br-: bromide ion, I-: iodide ion, MsO-: mesylate ion, TsO-: tosylate ion, cis: steric configuration of the cycloalkane moiety is cis-configuration, trans: steric configuration of the cycloalkane moiety is trans-configuration, (+)-DBTA: (+)-dibenzoyl-D-tartaric acid, (-)-DBTA: (-)-dibenzoyl-L-tartaric acid, (+)-MA: L-(+)-mandelic acid, (-)-MA: D-(-)-mandelic acid.

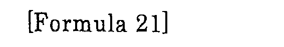

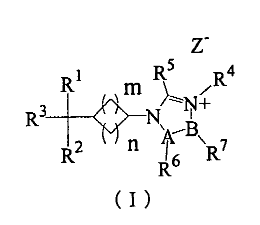

[Formula 22]

| Example | R1 | R2 | R3 | m | n | R4 | R5 | R6 | R7 | A-B | Z- | |
|---|---|---|---|---|---|---|---|---|---|---|---|---|
| 1 | Ph | Ph | CONH2 | 1 | 2 | Me | Me | H | H | C=C | I- | cis |
| 2 | Ph | Ph | CN | 1 | 2 | Me | iPr | H | H | C=C | I- | cis |
| 3 | Ph | Ph | CONH2 | 1 | 2 | Me | iPr | H | H | C=C | I- | cis |
| 4 | Ph | Ph | CONH2 | 1 | 2 | Me | H | Me | H | C=C | I- | cis |
| 5 | Ph | Ph | CONH2 | 1 | 2 | Me | H | H | H | C=C | I- | cis |
| 6 | Ph | Ph | CONH2 | 1 | 2 | 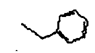 | Me | H | H | C=C | Br- | cis |
| 7 | Ph | Ph | CONH2 | 1 | 2 | 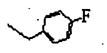 | Me | H | H | C=C | Br- | cis |
| 8 | Ph | Ph | CONH2 | 1 | 2 | 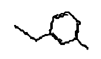 | Me | H | H | C=C | Br- | cis |
| 9 | Ph | Ph | CONH2 | 1 | 2 | 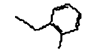 | Me | H | H | C=C | Br- | cis |
| 10 | Ph | Ph | CONH2 | 1 | 2 | 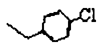 | Me | H | H | C=C | Br- | cis |
| 11 | Ph | Ph | CONH2 | 1 | 2 | 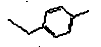 | Me | H | H | C=C | Br- | cis |
| 12 | Ph | Ph | CONH2 | 1 | 2 | 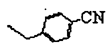 | Me | H | H | C=C | Br- | cis |
| 13 | Ph | Ph | CONH2 | 1 | 2 | 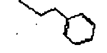 | Me | H | H | C=C | Br- | cis |
| 14 | Ph | Ph | CONH2 | 1 | 2 | 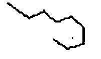 | Me | H | H | C=C | Br- | cis |
| 15 | Ph | Ph | CONH2 | 1 | 2 | 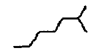 | Me | H | H | C=C | Br- | cis |
| 16 | Ph | Ph | CONH2 | 1 | 2 | 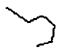 | Me | H | H | C=C | Br- | cis |
| 17 | Ph | Ph | CONH2 | 1 | 2 | 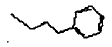 | Me | H | H | C=C | Br- | cis |
| 18 | Ph | Ph | CONH2 | 1 | 2 | 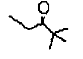 | Me | H | H | C=C | Br- | cis |
| 19 | Ph | Ph | CONH2 | 1 | 2 | 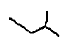 | Me | H | H | C=C | Br- | cis |
| 20 | Ph | Ph | CONH2 | 1 | 2 | 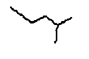 | Me | H | H | C=C | Br- | cis |

[Formula 23]

| Example | R1 | R2 | R3 | m | n | R4 | R5 | R6 | R7 | A-B | Z- | |
|---|---|---|---|---|---|---|---|---|---|---|---|---|
| 21 | Ph | Ph | CONH2 | 1 | 2 | 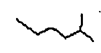 | Me | H | H | C=C | Br- | cis |
| 22 | Ph | Ph | CONH2 | 1 | 2 | 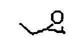 | Me | H | H | C=C | Br- | cis |
| 23 | Ph | Ph | CONH2 | 1 | 2 | 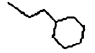 | Me | H | H | C=C | Br- | cis |
| 24 | Ph | Ph | CONH2 | 1 | 2 | 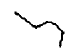 | Me | H | H | C=C | Br- | cis |
| 25 | Ph | Ph | CONH2 | 1 | 2 | 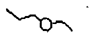 | Me | H | H | C=C | Br- | cis |
| 26 | Ph | Ph | CONH2 | 1 | 2 | 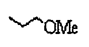 | Me | H | H | C=C | Br- | cis |
| 27 | Ph | Ph | CONH2 | 1 | 2 | 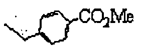 | Me | H | H | C=C | Br- | cis |
| 28 | Ph | Ph | CONH2 | 1 | 2 | 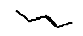 | Me | H | H | C=C | Br- | cis |
| 29 | Ph | Ph | CONH2 | 1 | 2 | 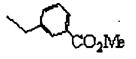 | Me | H | H | C=C | Br- | cis |
| 30 | Ph | Ph | CONH2 | 1 | 2 | 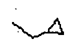 | Me | H | H | C=C | Br- | cis |
| 31 | Ph | Ph | CONH2 | 1 | 2 | 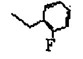 | Me | H | H | C=C | Br- | cis |
| 32 | Ph | Ph | CONH2 | 1 | 2 | 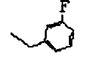 | Me | H | H | C=C | Br- | cis |
| 33 | Ph | Ph | CONH2 | 1 | 2 | 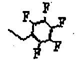 | Me | H | H | C=C | Br- | cis |
| 34. | Ph | Ph | CONH2 | 1 | 2 | 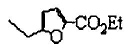 | Me | H | H | C=C | Br- | cis |
| 35 | Ph | Ph | CONH2 | 1 | 2 | 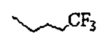 | Me | H | H | C=C | I- | cis |
| 36 | Ph | Ph | CONH2 | 1 | 2 | 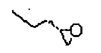 | Me | H | H | C=C | Br- | cis |
| 37 | Ph | Ph | CONH2 | 1 | 2 | CHF2 | Me | H | H | C=C | Br- | cis |
| 38 | Ph | Ph | CONH2 | 1 | 2 | 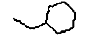 | Me | H | H | C=C | Br- | cis |
| 39 | Ph | Ph | CONH2 | 1 | 2 | 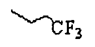 | Me | H | H | C=C | I- | cis |
| 40 | Ph | Ph | CONH2 | 1 | 2 | 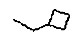 | Me | H | H | C=C | Br- | cis |

[Formula 24]

| Example | R1 | R2 | R3 | m | n | R4 | R5 | R6 | R7 | A-B | Z- | |
|---|---|---|---|---|---|---|---|---|---|---|---|---|
| 41 | Ph | Ph | CONH2 | 1 | 2 | 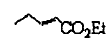 | Me | H | H | C=C | Br- | cis |
| 42 | Ph | Ph | CONH2 | 1 | 2 | 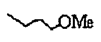 | Me | H | H | C=C | Br- | cis |
| 43 | Ph | Ph | CONH2 | 1 | 2 | 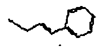 | Me | H | H | C=C | Br- | cis |
| 44 | Ph | Ph | CONH2 | 1 | 2 | 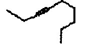 | Me | H | H | C=C | Br- | cis |
| 45 | Ph | Ph | CONH2 | 1 | 2 | 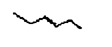 | Me | H | H | C=C | Br- | cis |
| 46 | Ph | Ph | CONH2 | 1 | 2 | 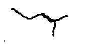 | Me | H | H | C=C | Br- | cis |
| 47 | Ph | Ph | CONH2 | 1 | 2 | 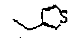 | Me | H | H | C=C | Br- | cis |
| 48 | Ph | Ph | CONH2 | 1 | 2 | 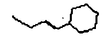 | Me | H | H | C=C | Br- | cis |
| 49 | Ph | Ph | CONH2 | 1 | 2 | 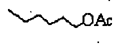 | Me | H | H | C=C | Br- | cis |
| 50 | Ph | Ph | CONH2 | 1 | 2 | 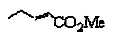 | Me | H | H | C=C | Br- | cis |
| 51 | Ph | Ph | CONH2 | 1 | 2 | 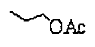 | Me | H | H | C=C | Br- | cis |
| 52 | Ph | Ph | CONH2 | 1 | 2 | 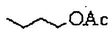 | Me | H | H | C=C | Br- | cis |
| 53 | Ph | Ph | CONH2 | 1 | 2 | 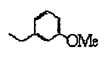 | Me | H | H | C=C | Br- | cis |
| 54 | Ph | Ph | CONH2 | 1 | 2 | 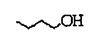 | Me | H | H | C=C | Br- | cis |
| 55 | Ph | Ph | CONH2 | 1 | 2 | 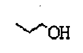 | Me | H | H | C=C | Br- | cis |
| 56 | Ph | Ph | CONH2 | 1 | 2 | Me | Me | H | H | CH-CH | I- | cis |

[Formula 25]

| Example | R1 | R2 | R3 | m | n | R4 | R5 | R6 | R7 | A-B | Z- | | Resolving agent |
|---|---|---|---|---|---|---|---|---|---|---|---|---|---|
| 57 | Ph | Ph | CONH2 | 1 | 2 | Me | Me | H | H | CH-CH | I- | cis | (+)-DBTA |
| 58 | Ph | Ph | CONH2 | 1 | 2 | Me | Me | H | H | CH-CH | I- | cis | (-)-DBTA |
| 59 | Ph | Ph | CONH2 | 1 | 2 | Me | H | H | H | C=C | I- | trans | |
| 60 | Ph | Ph | CONH2 | 1 | 2 | Me | Me | H | H | CH-CH | TsO- | cis | (+)-DBTA |
| 61 | Ph | Ph | CONH2 | 1 | 2 | 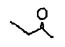 | Me | H | H | C=C | Br- | cis | |
| 62 | Ph | Ph | CONH2 | 1 | 2 | 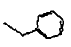 | Me | H | H | C=C | Br- | trans | |
| 63 | Ph | Ph | CONH2 | 1 | 2 | 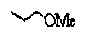 | Me | H | H | C=C | Br- | cis | (+)-DBTA |
| 64 | Ph | Ph | CONH2 | 1 | 2 | 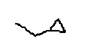 | Me | H | H | C=C | Br- | cis | (+)-DBTA |
| 65 | Ph | Ph | CONH2 | 1 | 2 | 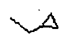 | Me | H | H | C=C | Br- | cis | (-)-DBTA |
| 66 | Ph | Ph | CONH2 | 1 | 2 | Me | Me | H | H | C=C | I- | trans | |
| 67 | Ph | Ph | CONH2 | 1 | 2 | 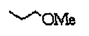 | Me | H | H | C=C | Br- | trans | |
| 68 | Ph | Ph | CONH2 | 1 | 2 | 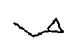 | Me | H | H | C=C | Br- | trans | |
| 69 | Ph | Ph | CONH2 | 1 | 2 | Me | H | H | | C=N | I- | cis | |
| 70 | Ph | Ph | CONH2 | 1 | 2 | 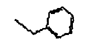 | H | H | | C=N | Br- | cis | |
| 71 | Ph | Ph | CONH2 | 1 | 2 | 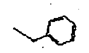 | H | | H | N=C | Br- | cis | |
| 72 | Ph | Ph | CONH2 | 1 | 2 | 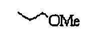 | H | H | | C=N | Br- | cis | |
| 73 | Ph | Ph | CONH2 | 1 | 2 | 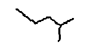 | Me | H | H | C=C | Br- | trans | |
| 74 | Ph | Ph | CONH2 | 1 | 2 | 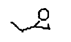 | Me | H | H | C=C | Br- | trans | |
| 75 | Ph | Ph | CONH2 | 1 | 2 | 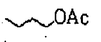 | Me | H | H | C=C | Br- | trans | |
| 76 | Ph | Ph | CONH2 | 1 | 2 | 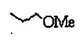 | Me | H | H | C=C | TsO- | cis | (-)-DBTA |
| 77 | Ph | Ph | CONH2 | 1 | 2 | 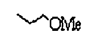 | Me | H | H | C=C | TsO- | cis | (+)-OBTA |

[Formula 26]

| Example | R1 | R2 | R3 | m | n | R4 | R5 | R6 | R7 | A-B | Z- | | Resolving agent |
|---|---|---|---|---|---|---|---|---|---|---|---|---|---|
| 78 | Ph | Ph | CONH2 | 1 | 2 | 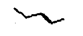 | Me | H | H | C=C | Br- | cis | (-)-DBTA |
| 79 | Ph | Ph | CONH2 | 1 | 2 | 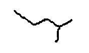 | Me | H | H | C=C | Br- | cis | (-)-DBTA |
| 80 | Ph | Ph | CONH2 | 1 | 2 | Me | Me | H | H | C=C | I- | cis | (-)-DBTA |
| 81 | Ph | Ph | CONH2 | 1 | 2 | CHF2 | Me | H | H | C=C | Br- | cis | (-)-DBTA |
| 82 | Ph | Ph | CONH2 | 1 | 2 | 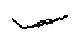 | Me | H | H | C=C | Br- | cis | (-)-DBTA |
| 83 | Ph | Ph | CONH2 | 1 | 2 | 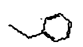 | Me | H | H | C=C | Br- | cis | (-)-DBTA |
| 84 | Ph | Ph | CONH2 | 1 | 2 | 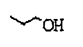 | Me | H | H | C=C | Br- | cis | (-)-DBTA |
| 85 | Ph | Ph | CONH2 | 1 | 2 | 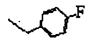 | Me | H | H | C=C | Br- | cis | (-)-DBTA |
| 86 | Ph | Ph | CONH2 | 1 | 2 | 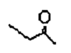 | Me | H | H | C=C | Br- | trans | |
| 87 | Ph | Ph | CN | 1 | 2 | 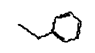 | Me | H | H | C=C | Br- | trans | |
| 88 | Ph | Ph | CN | 1 | 2 | 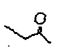 | Me | H | H | C=C | Br- | trans | |
| 89 | Ph | Ph | CONH2 | 1 | 2 | 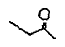 | Me | H | H | C=C | Br- | cis | (-)-DBTA |
| 90 | Ph | Ph | CONH2 | 1 | 2 | 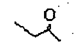 | Me | H | H | C=C | Br- | cis | (+)-DBTA |
| 91 | Ph | Ph | CONH2 | 1 | 2 | 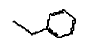 | Me | H | H | C=C | Br- | cis | (+)-DBTA |
| 92 | Ph | Ph | CONH2 | 1 | 2 | CHF2 | Me | H | H | C=C | Br- | cis | (+)-DBTA |
| 93 | Ph | Ph | CONH2 | 1 | 2 | Me | Me | H | H | C=C | TsO- | cis | (+)-DBTA |
| 94 | Ph | Ph | CONH2 | 1 | 2 | Me | Me | H | H | C=C | TsO- | cis | (-)-DBTA |
| 95 | Ph | Ph | CONH2 | 1 | 2 | Me | Me | H | H | C=C | I- | trans | (+)-MA |
| 96 | Ph | Ph | CONH2 | 1 | 2 | Me | Me | H | H | C=C | I- | trans | (-)-MA |
| 97 | Ph | Ph | CONH2 | 1 | 2 | 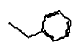 | Me | H | H | C=C | Br- | trans | (+)-MA |
| 98 | Ph | Ph | CONH2 | 1 | 2 | 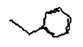 | Me | H | H | C=C | Br- | trans | (-)-MA |

[Formula 27]

| Example | R1 | R2 | R3 | m | n | R4 | R5 | R6 | R7 | A-B | Z- | | Resolving agent |
|---|---|---|---|---|---|---|---|---|---|---|---|---|---|
| 99 | Ph | Ph | CONH2 | 1 | 2 | 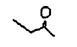 | Me | H | H | C=C | Br- | trans | (-)-MA |
| 100 | Ph | Ph | CONH2 | 1 | 2 | 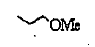 | Me | H | H | C=C | TsO- | trans | (+)-MA |
| 101 | Ph | Ph | CONH2 | 1 | 2 | 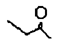 | H | | H | N=C | Br- | cis | |
| 102 | Ph | Ph | CONH2 | 1 | 2 | 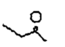 | H | H | | C=N | Br- | cis | |
| 103 | Ph | Ph | CONH2 | 1 | 2 | 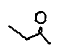 | Et | H | H | C=C | Br- | cis | |
| 104 | Ph | Ph | CONH2 | 1 | 2 | 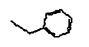 | Et | H | H | C=C | Br- | cis | |
| 105 | Ph | Ph | CONH2 | 1 | 2 | Me | Et | H | H | C=C | I- | cis | |
| 106 | Ph | Ph | CONH2 | 1 | 2 | 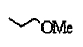 | Me | H | H | C=C | TsO- | trans | (-)-MA |
| 107 | Ph | Ph | CONH2 | 1 | 2 | 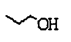 | Me | H | H | C=C | Br- | cis | (+)-DBTA |
| 108 | Ph | Ph | CONH2 | 1 | 2 | 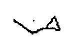 | Me | H | H | C=C | Br- | trans | (+)-MA |
| 109 | Ph | Ph | CONH2 | 1 | 2 | 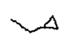 | Me | H | H | C=C | Br- | trans | (-)-MA |
| 110 | Ph | Ph | CONH2 | 1 | 2 | 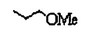 | Et | H | H | C=C | TsO- | cis | |
| 111 | Ph | Ph | CONH2 | 1 | 2 | 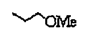 | H | | H | N=C | TsO- | cis | |
| 112 | Ph | Ph | CONH2 | 1 | 2 | 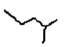 | Me | H | H | C=C | Br | trans | (+)-MA |
| 113 | Ph | Ph | CONH2 | 1 | 2 | 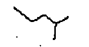 | Me | H | H | C=C | Br- | trans | (-)-MA |
| 114 | Ph | Ph | CONH2 | 1 | 2 | 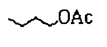 | Me | H | H | C=C | Br- | trans | (+)-MA |
| 115 | Ph | Ph | CONH2 | 1 | 2 | 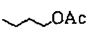 | Me | H | H | C=C | Br- | trans | (-)-MA |
| 116 | Ph | Ph | CONH2 | 1 | 2 | 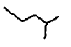 | Me | H | H | C=C | Br- | cis | (+)-DBTA |
| 117 | Ph | Ph | CONH2 | 1 | 2 | 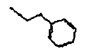 | Me | H | H | C=C | Br- | trans | |
| 118 | Ph | Ph | CONH2 | 1 | 2 | 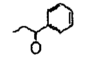 | Me | H | H | C=C | Br- | cis | |
| 119 | Ph | Ph | CONH2 | 1 | 2 | 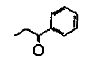 | Me | H | H | C=C | Br- | trans | |

[Formula 28]

| Example | R1 | R2 | R3 | m | n | R4 | R5 | R6 | R7 | A-B | Z- | | Resolving agent |
|---|---|---|---|---|---|---|---|---|---|---|---|---|---|
| 120 | Ph | Ph | CONH2 | 1 | 2 | 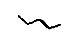 | Me | H | H | C=C | Br- | cis | (+)-DBTA |
| 121 | Ph | Ph | CONH2 | 1 | 2 | 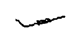 | Me | H | H | C=C | Br- | cis | (+)-DBTA |
| 122 | Ph | Ph | CONH2 | 1 | 2 | 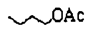 | Me | H | H | C=C | Br- | cis | (+)-DBTA |
| 123 | Ph | Ph | CONH2 | 1 | 2 | 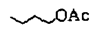 | Me | H | H | C=C | Br- | cis | (-)-DBTA |
| 124 | Ph | Ph | CONH2 | 1 | 2 | 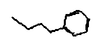 | Me | H | H | C=C | Br- | trans | |
| 125 | Ph | Ph | CONH2 | 1 | 2 | 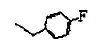 | Me | H | H | C=C | Br- | cis | (+)-DBTA |
| 126 | Ph | Ph | CONH2 | 1 | 2 | CHF2 | Me | H | H | C=C | Br- | trans | |
| 127 | Ph | Ph | CONH2 | 1 | 2 | 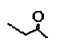 | H | H | H | C=C | Br- | trans | |
| 128 | Ph | Ph | CONH2 | 1 | 2 | 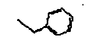 | H | H | Me | C=C | Br- | trans | |
| 129 | Ph | Ph | CONH2 | 1 | 2 | 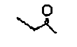 | H | H | Me | C=C | Br- | trans | |
| 130 | Ph | Ph | CONH2 | 1 | 2 | 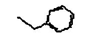 | H | H | H | C=C | Br- | trans | |
| 131 | Ph | Ph | CONH2 | 1 | 2 | 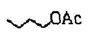 | H | H | Me | C=C | Br- | trans | |
| 132 | Ph | Ph | CONH2 | 1 | 2 | 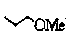 | H | H | H | C=C | TsO- | trans | |
| 133 | Ph | Ph | CONH2 | 1 | 2 | 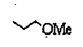 | Me | H | H | C=C | MsO- | cis | (+)-DBTA |
| 134 | Ph | Ph | CONH2 | 1 | 2 | 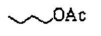 | H | H | H | C=C | Br- | trans | |
| 135 | Ph | Ph | CONH2 | 1 | 2 | 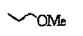 | Me | H | H | C=C | MsO- | cis | (-)-DBTA |
| 136 | Ph | Ph | CONH2 | 1 | 2 | 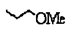 | Me | H | H | C=C | MsO- | cis | |
| 137 | Ph | Ph | CONH2 | 1 | 2 | 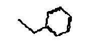 | Me | H | H | CH-CH | Br- | cis | |
| 138 | Ph | Ph | CONH2 | 1 | 2 | 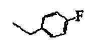 | Me | H | H | CH-CH | Br- | cis | |
| 139 | Ph | Ph | CONH2 | 1 | 2 | 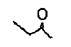 | Me | H | H | CH-CH | Br- | cis | |
| 140 | Ph | Ph | CONH2 | 1 | 2 | 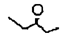 | Me | H | H | C=C | Br- | cis | (+)-DBTA |

[Formula 29]

| Example | R1 | R2 | R3 | m | n | R4 | R5 | R6 | R7 | A-B | Z- | | Resolving agent |
|---|---|---|---|---|---|---|---|---|---|---|---|---|---|
| 141 | Ph | Ph | CONH2 | 1 | 2 | 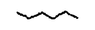 | Me | H | H | CH-CH | Br- | cis | |
| 142 | Ph | Ph | CONH2 | 1 | 2 | 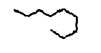 | Me | H | H | CH-CH | I- | cis | |
| 143 | Ph | Ph | CONH2 | 1 | 2 | 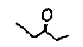 | Me | H | H | CH-CH | Br | cis | |
| 144 | Ph | Ph | CONH2 | 1 | 2 | 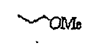 | Me | H | H | CH-CH | Br- | cis | |
| 145 | Ph | Ph | CONH2 | 1 | 2 | 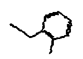 | Me | H | H | CH-CH | Br- | cis | |
| 146 | Ph | Ph | CONH2 | 1 | 2 | 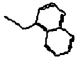 | Me | H | H | CH-CH | Cl- | cis | |
| 147 | Ph | Ph | CONH2 | 1 | 2 | 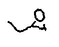 | Me | H | H | CH-CH | Br- | cis | |
| 148 | Ph | Ph | CONH2 | 1 | 2 | 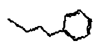 | Me | H | H | C=C | Br- | cis | (+)-DBTA |
| 149 | Ph | Ph | CONH2 | 1 | 2 | 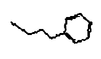 | Me | H | H | C=C | Br- | cis | (-)-DBTA |
| 150 | Ph | Ph | CONH2 | 1 | 2 | 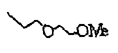 | Me | H | H | C=C | Br- | cis | (+)-DBTA |
| 151 | Ph | Ph | CONH2 | 1 | 2 | Me | Me | H | H | CH-CH | TsO- | cis | (-)-DBTA |
| 152 | Ph | Ph | CONH2 | 1 | 2 | Me | Me | H | H | CH-CH | TsO- | trans | (+)-DBTA |
| 153 | Ph | Ph | CONH2 | 1 | 2 | Me | Me | H | H | CH-CH | TsO- | trans | (-)-DBTA |
| 154 | Ph | Ph | CN | 1 | 2 | Me | Me | H | H | CH-CH | Br- | cis | (+)-DBTA |
| 155 | Ph | Ph | CONH2 | 1 | 2 | 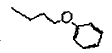 | Me | H | H | CH-CH | Br- | cis | |
| 156 | Ph | Ph | CONH2 | 1 | 2 | 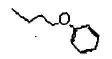 | Me | H | H | C=C | Br | cis | (+)-DBTA |
| 157 | Ph | Ph | CONH2 | 1 | 2 | 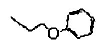 | Me | H | H | C=C | Br- | cis | (+)-DBTA |
| 158 | Ph | Ph | CONH2 | 1 | 2 | 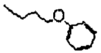 | Me | H | H | C=C | Br- | cis | (-)-DBTA |
| 159 | Ph | Ph | CONH2 | | 2 | 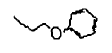 | Me | H | H | C=C | Br- | cis | (-)-DBTA |

### Test Example 1: Muscarinic M₃ antagonistic action in isolated guinea pig trachea

Male Hartley guinea pigs (350 to 600 g) were sacrificed by bleeding, and trachea was excised, and cut at every two tracheal cartilages to prepare tissue specimens. Each tissue specimen was kept warm at 37°C, suspended with a resting tension of 1 g in a 5-mL Magnus bath filled with a Krebs carbonate buffer containing indomethacin (1 µM) bubbled with 95% O₂/5% CO₂, and left for 60 minutes as a stabilization period. The contraction reaction induced with carbachol was recorded by the cumulative method, and measurement was performed isometrically from 10⁻⁸ M in an equal ratio of 3 with an interval of 7 minutes for one concentration. After the measurement, the tissue specimen was immediately washed, and left for 60 minutes as a stabilization period before the next contraction reaction, and the tissue specimen of which EC₅₀ for the carbachol-induced contraction reaction was stabilized was considered as a control. Each test compound was applied 15 minutes before the addition of carbachol. The affinity (pA₂) of the test compound was obtained by the Schild method (Arunlakshana, O. and Schild, H.O., Brit. J. Phrmacol., 1959, 14, 48-58). The results are shown in Table 1. Ipratropium and atropine mentioned in the table are reagents produced by SIGMA, and the test was performed by using purchased reagents.

### Test Example 2: Muscarinic M₂ antagonistic action in isolated guinea pig left atrium

Male Hartley guinea pigs (350 to 600 g) were sacrificed by bleeding, and left atria were excised to prepare tissue specimens. Each tissue specimen was kept warm at 37°C, suspended with a resting tension of 0.5 g in a 5-mL Magnus bath filled with a Krebs carbonate buffer bubbled with 95% O₂/5% CO₂. Then, contraction evoked by field electrical stimulation (4 Hz, 2 msec, 1.5 x threshold potential) was measured. After a stabilization period of 60 minutes, carbachol-induced relaxation was isometrically measured and recorded by the cumulative method from 10⁻⁸ M with an interval of 90 seconds for one concentration in an equal ratio of 3. After the measurement, the tissue specimen was immediately washed, and left for 45 minutes as a stabilization period before the next observation. The tissue specimen of which EC₅₀ for the carbachol-induced relaxation was stabilized was considered as a control. Each test compound was applied 30 minutes before the addition of carbachol. The affinity (pA₂) of the test compound was obtained in the same manner as in the case of the trachea. The results are shown in Table 1. Ipratropium and atropine mentioned in the table are reagents produced by SIGMA, and the test was performed by using purchased reagents.

[Table 1]

**Table 1: Muscarinic receptor antagonistic action (in vitro)**

| Example No. and Comparative Compound | pA₂ | |
|---|---|---|
| | Trachea M₃ | Left atrium M₂ |
| Ipratropium | 9 | 8.4 |
| Atropine | 8.9 | 8.6 |
| Reference Example 5 | 9.1 | 7.3 |
| Reference Example 13 | 8.3 | 7.1 |
| Example 1 | 8.8 | 7.5 |
| Example 4 | 8.7 | 7.1 |
| Example 5 | 8.6 | 7.5 |
| Example 7 | 9.6 | 8.5 |
| Example 8 | 9.5 | 8 |
| Example 22 | 9 | 7.7 |
| Example 25 | 8.8 | 7.6 |
| Example 26 | 9.7 | 7.9 |
| Example 30 | 9.7 | 8.3 |
| Example 36 | 9 | 7.7 |
| Example 37 | 9.1 | 7.8 |
| Example 40 | 9.4 | 8.1 |
| Example 51 | 9 | 7.5 |
| Example 54 | 9 | 7.5 |
| Example 56 | 8.6 | 7.5 |
| Example 57 | 8.9 | 7.6 |
| Example 58 | 8.5 | 7.1 |
| Example 59 | 9.1 | 7.7 |
| Example 60 | 9.2 | 7.8 |
| Example 61 | 9 | 6.6 |
| Example 63 | 9.5 | 8.1 |
| Example 65 | 8.9 | 7.3 |
| Example 66 | 8.9 | 7.4 |
| Example 67 | 8.9 | 7.1 |
| Example 68 | 9.4 | 7.4 |

**[Table 2]**

| Table 1: Muscarinic receptor antagonistic action (in vitro) (Cont.) | | |
|---|---|---|
| Example No. and Comparative Compound | pA₂ | |
| | Trachea M₃ | Left atrium M₂ |
| Example 69 | 9.1 | 7.6 |
| Example 71 | 9.1 | 7.4 |
| Example 72 | 9.1 | 7.5 |
| Example 73 | 9.2 | 8.2 |
| Example 75 | 9.3 | 6.8 |
| Example 76 | 8.9 | 6.7 |
| Example 77 | 9.6 | 8.2 |
| Example 78 | 9.2 | 7.9 |
| Example 79 | 9.2 | 7.2 |
| Example 80 | 9.4 | 6.6 |
| Example 81 | 9 | 6.8 |
| Example 82 | 9.1 | 7.2 |
| Example 83 | 10.1 | 8.3 |
| Example 84 | 8.2 | 6.3 |
| Example 85 | 9.1 | 7.5 |
| Example 86 | 8.9 | 5.9 |
| Example 87 | 9 | 7.3 |
| Example 88 | 9 | 6.6 |
| Example 89 | 7.7 | 5.5 |
| Example 90 | 9.1 | 6.5 |
| Example 91 | 10 | 8.4 |
| Example 92 | 9.4 | 7.9 |
| Example 94 | 9.1 | 7 |
| Example 97 | 10 | 8.4 |
| Example 98 | 9.4 | 7.8 |

As shown in Table 1, it was revealed that the differences of the pA₂ values for the muscarinic M₂ and M₃ receptors of the compounds represented by the general formula (I) were larger than the difference of the pA₂ values of atropine already clinically used. It was also revealed that, among the compounds represented by the general formula (I), many compounds had differences of pA₂ of 1 or larger. From these results, it was concluded that the compounds of the present invention had superior selectivity for the muscarinic M₃ receptor.

### Test Example 3: Effect on bronchoconstriction in guinea pigs (via inhalation administration)

The test was performed according to the method of Konzett et al. (Arch. Exp. Path Pharmak., 1940, 195, 71-74). Male Hartley guinea pigs (350 to 600 g) were anesthetized with urethane (1.8 g/kg, i.p.), and then a cannula for drug administration was inserted into each left jugular vein. After the immobilization with decamethonium (2 mg/kg, i.v.), a tracheal cannula was inserted, and controlled respiration was attained by a respirator (Harvard). Bronchoconstriction was measured as changes of airway pressure with a pressure transducer (Nihon Kohden Corp.). Each test compound was administered (10 µg/head) from dissected trachea by using a device for intratracheal administration of liquid for guinea pigs (Penn Century), and after suture, the animals were returned to usual breeding. After 24 hours, acetylcholine (4 µg/kg, i.v.) was administered to induce bronchoconstriction, and inhibitory activity on bronchoconstriction was calculated as percent inhibition comparing to the change of airway pressure in control group was obtained. The results are shown in Table 2.

**[Table 3]**

| Table 2: Inhibitory activity on bronchoconstriction | |
|---|---|
| Example No. and Comparative Compound | Percent of inhibition of bronchoconstriction (%) |
| Reference Example 5 | 0 |
| Reference Example 13 | 20 |
| Example 7 | 90 |
| Example 8 | 90 |
| Example 22 | 80 |
| Example 26 | 70 |
| Example 30 | 80 |
| Example 36 | 90 |
| Example 37 | 80 |
| Example 40 | 100 |
| Example 56 | 100 |
| Example 59 | 90 |
| Example 60 | 90 |
| Example 63 | 100 |
| Example 83 | 100 |
| Example 90 | 100 |
| Example 91 | 100 |

From the results shown in Table 2, it was revealed that the compounds represented by the general formula (I) had superior prolonged inhibitory activity on bronchoconstriction compared with the compounds of the reference examples.

From the results of the test examples mentioned above, it is considered that the compounds represented by the general formula (I) are muscarinic M₃ receptor antagonists more unlikely to cause cardiac side effects and thus safe compounds, and are muscarinic M₃ receptor antagonists with prolonged action.

### Industrial Applicability

The compounds of the present invention are useful as prophylactic and/or therapeutic agents for a disease in which the muscarinic M₃ receptor is involved.

This application was filed based on Patent Application No. 2005-215146 filed in Japan, of which entire disclosure is herein incorporated.

## Claims

1. A nitrogen-containing heterocyclic derivative represented by the general formula (I): 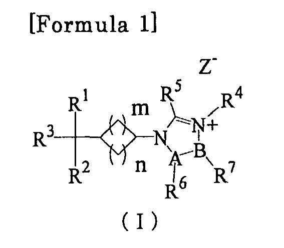 [wherein, in the formula, R¹ represents aryl of which ring may be substituted, R² represents aryl of which ring may be substituted, lower alkyl which may be substituted, or cycloalkyl which may be substituted, R³ represents hydrogen atom, cyano, a CONHR⁸ group (R⁸ represents hydrogen atom or lower alkyl), a CO₂R⁹ group (R⁹ represents hydrogen atom, aryl, lower alkyl, lower alkenyl, lower alkynyl, or aralkyl), hydroxyl group, a OCOR¹⁰ group (R¹⁰ represents aryl, lower alkyl, or aralkyl), or SONH₂ group, R⁴ represents alkyl which may be substituted or a CO₂R¹¹ group (R¹¹ represents aryl, lower alkyl, or aralkyl), R⁵ represents hydrogen atom, aryl, lower alkyl, cycloalkyl, or aralkyl, R⁶ and R⁷ independently do not exist, or represent hydrogen atom or lower alkyl, -A-B- represents -CH-CH-, -C=C-, -C=N-, or -N=C-, the sum of m and n is an integer of 1 to 6, and Z represents an anion], or a hydrate or solvate thereof.

2. The nitrogen-containing heterocyclic derivative, or a hydrate or solvate thereof according to claim 1, wherein R¹ and R² are phenyls which may be independently substituted.

3. The nitrogen-containing heterocyclic derivative, or a hydrate or solvate thereof according to claim 1 or 2, wherein R³ is cyano, a CONHR⁸ group (R⁸ represents hydrogen atom, methyl, or ethyl), carboxy, methoxycarbonyl, ethoxycarbonyl, hydroxyl group, methylcarbonyloxy, ethylcarbonyloxy, or SONH₂ group.

4. The nitrogen-containing heterocyclic derivative, or a hydrate or solvate thereof according to any one of claims 1 to 3, wherein R⁴ is alkyl having 1 to 8 carbon atoms which may be substituted.

5. The nitrogen-containing heterocyclic derivative, or a hydrate or solvate thereof according to any one of claims 1 to 4, wherein R¹ and R² are phenyls, R³ is cyano or CONH₂ group, and R⁴ is alkyl having 1 to 8 carbon atoms which may be substituted.

6. The nitrogen-containing heterocyclic derivative, or a hydrate or solvate thereof according to any one of claims 1 to 5, wherein R⁴ is methyl, methoxyethyl, benzyl, acetylmethyl, phenylpropyl, or hydroxyethyl.

7. The nitrogen-containing heterocyclic derivative, or a hydrate or solvate thereof according to any one of claims 1 to 6, wherein R⁵ is hydrogen atom or lower alkyl.

8. The nitrogen-containing heterocyclic derivative, or a hydrate or solvate thereof according to any one of claims 1 to 7, wherein R⁶ and R⁷ independently do not exist, or represent hydrogen atom, methyl, or ethyl.

9. The nitrogen-containing heterocyclic derivative, or a hydrate or solvate thereof according to any one of claims 1 to 8, wherein R⁵ is hydrogen atom or alkyl having 1 to 3 carbon atoms, and R⁶ and R⁷ independently do not exist, or represent hydrogen atom or methyl.

10. The nitrogen-containing heterocyclic derivative, or a hydrate or solvate thereof according to any one of claims 1 to 9, wherein -A-B- is -CH-CH- or -C=C-.

11. The nitrogen-containing heterocyclic derivative, or a hydrate or solvate thereof according to any one of claims 1 to 10, wherein the sum of m and n is 3 or 4.

12. The nitrogen-containing heterocyclic derivative, or a hydrate or solvate thereof according to any one of claims 1 to 11, wherein the sum of m and n is 3.

13. The nitrogen-containing heterocyclic derivative, or a hydrate or solvate thereof according to any one of claims 1 to 12, wherein Z⁻ is an anion formed from a halogen atom or an organic sulfonic acid.

14. The nitrogen-containing heterocyclic derivative, or a hydrate or solvate thereof according to any one of claims 1 to 13, wherein Z- is chloride ion, bromide ion, iodide ion, tosylate ion, or mesylate ion.

15. 3-((1R,3S)-3-(Carbamoyl(diphenyl)methyl)cyclopentyl)-1,2-dimethyl-4,5-dihydro-3H-imidazol-1-ium para-toluenesulfonate.

16. 3-((1R,3S)-3-(Carbamoyldiphenylmethyl)cyclopentan-1-yl)-1-(2-methoxyethyl)-2-methyl-3H-imidazol-1-ium mesylate.

17. 3-((1S,3R)-3-(Carbamoyldiphenylmethyl)cyclopentan-1-yl)-1-benzyl-2-methyl-3H-imidazol-1-ium bromide.

18. 3-((1R,3S)-3-(Carbamoyldiphenylmethyl)cyclopentan-1-yl)-2-methyl-1-(2-oxopropyl)-3H-imidazol-1-ium bromide.

19. 3-((1R,3S)-3-(Carbamoyl(diphenyl)methyl)cyclopentyl)-1-(3-phenylpropyl)-2-methyl-3H-imidazol-1-ium bromide.

20. 3-((1R,3S)-3-(Carbamoyldiphenylmethyl)cyclopentan-1-yl)-1-hydroxyethyl-2-methyl-3H-imidazol-1-ium bromide.

21. A pharmaceutical composition comprising a nitrogen-containing heterocyclic derivative represented by the general formula (I): 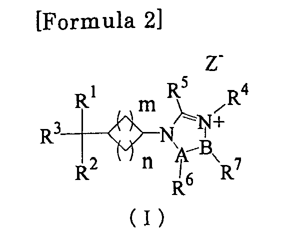 [wherein, in the formula, R¹ represents aryl of which ring may be substituted, R² represents aryl of which ring may be substituted, lower alkyl which may be substituted, or cycloalkyl which may be substituted, R³ represents hydrogen atom, cyano, a CONHR⁸ group (R⁸ represents hydrogen atom or lower alkyl), a CO₂R⁹ group (R⁹ represents hydrogen atom, aryl, lower alkyl, lower alkenyl, lower alkynyl, or aralkyl), hydroxyl group, a OCOR¹⁰ group (R¹⁰ represents aryl, lower alkyl, or aralkyl), or SONH₂ group, R⁴ represents alkyl which may be substituted or a CO₂R¹¹ group (R¹¹ represents aryl, lower alkyl, or aralkyl), R⁵ represents hydrogen atom, aryl, lower alkyl, cycloalkyl, or aralkyl, R⁶ and R⁷ independently do not exist, or represent hydrogen atom or lower alkyl, -A-B- represents -CH-CH-, -C=C-, -C=N-, or -N=C-, the sum of m and n is an integer of 1 to 6, and Z⁻ represents an anion], or a hydrate or solvate thereof, and a pharmaceutically acceptable carrier.

22. The pharmaceutical composition according to claim 21, which is a prophylactic and/or therapeutic agent for a disease in which the muscarinic M₃ receptor is involved.

23. The pharmaceutical composition according to claim 21 or 22, which is a prophylactic and/or therapeutic agent for a respiratory disease, a urologic disease, a digestive system disease, or a central nervous system disease.

24. The pharmaceutical composition according to claim 23, wherein the respiratory disease is chronic obstructive pulmonary disease, chronic bronchitis, asthma, chronic airway obstruction, pulmonary fibrosis, emphysema, diffuse panbronchiolitis, bronchiectasis, idiopathic interstitial pneumonia, or rhinitis.

25. The pharmaceutical composition according to claim 23, wherein the urologic disease is neurogenic pollakiuria, neurogenic bladder dysfunction, enuresis nocturna, unstable bladder, bladder spasms, chronic cystitis, or urinary incontinence and/or pollakiuria in interstitial cystitis.

26. The pharmaceutical composition according to claim 23, wherein the digestive system disease is irritable bowel syndrome, spasticity colitis, diverticulitis, functional diarrhea, esophageal achalasia, cardiac achalasia, or biliary spasm.

27. The pharmaceutical composition according to claim 23, wherein the central nervous system disease is nausea or vomition caused by drug administration, kinesia, or Meniere's disease.

28. A prophylactic and/or therapeutic agent for chronic obstructive pulmonary disease, which comprises 3-((1R,3S)-3-(carbamoyl(diphenyl)methyl)cyclopentyl)-1,2-dimethyl-4,5-dihydro-3H-imidazol-1-ium para-toluenesulfonate and a pharmaceutically acceptable carrier.

29. A prophylactic and/or therapeutic agent for chronic obstructive pulmonary disease, which comprises 3-((1R,3S)-3-(carbamoyldiphenylmethyl)cyclopentan-1-yl)-1-(2-methoxyethyl)-2-methyl-3H-imidazol-1-ium mesylate and a pharmaceutically acceptable carrier.

30. A prophylactic and/or therapeutic agent for chronic obstructive pulmonary disease, which comprises 3-((1S,3R)-3-(carbamoyldiphenylmethyl)cyclopentan-1-yl)-1-benzyl-2-methyl-3H-imidazol-1-ium bromide and a pharmaceutically acceptable carrier.

31. A prophylactic and/or therapeutic agent for chronic obstructive pulmonary disease, which comprises 3-((1R,3S)-3-(carbamoyldiphenylmethyl)cyclopentan-1-yl)-2-methyl-1-(2-oxopropyl)-3H-imidazol-1-ium bromide and a pharmaceutically acceptable carrier.

32. A prophylactic and/or therapeutic agent for chronic obstructive pulmonary disease, which comprises 3-((1R,3S)-3-(carbamoyl(diphenyl)methyl)cyclopentyl)-1-(3-phenylpropyl)-2-methyl-3H-imidazol-1-ium bromide and a pharmaceutically acceptable carrier.

33. A prophylactic and/or therapeutic agent for chronic obstructive pulmonary disease, which comprises 3-((1R,3S)-3-(carbamoyldiphenylmethyl)cyclopentan-1-yl)-1-hydroxyethyl-2-methyl-3H-imidazol-1-ium bromide and a pharmaceutically acceptable carrier.

34. A muscarinic M₃ receptor antagonist containing a nitrogen-containing heterocyclic derivative represented by the general formula (I): 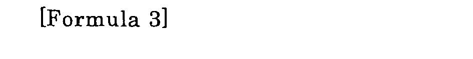 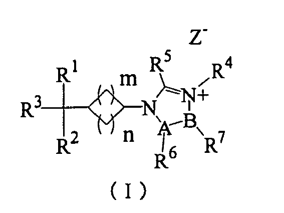 [wherein, in the formula, R¹ represents aryl of which ring may be substituted, R² represents aryl of which ring may be substituted, lower alkyl which may be substituted, or cycloalkyl which may be substituted, R³ represents hydrogen atom, cyano, a CONHR⁸ group (R⁸ represents hydrogen atom or lower alkyl), a CO₂R⁹ group (R⁹ represents hydrogen atom, aryl, lower alkyl, lower alkenyl, lower alkynyl, or aralkyl), hydroxyl group, a OCOR¹⁰ group (R¹⁰ represents aryl, lower alkyl, or aralkyl), or SONH₂ group, R⁴ represents alkyl which may be substituted or a CO₂R¹¹ group (R¹¹ represents aryl, lower alkyl, or aralkyl), R⁵ represents hydrogen atom, aryl, lower alkyl, cycloalkyl, or aralkyl, R⁶ and R⁷ independently do not exist, or represent hydrogen atom or lower alkyl, -A-B- represents -CH-CH-, -C=C-, -C=N-, or -N=C-, the sum of m and n is an integer of 1 to 6, and Z- represents an anion], or a hydrate or solvate thereof, or a pharmaceutically acceptable salt thereof as an active ingredient.
